(19) Europäisches Patentamt
European Patent Office
Office européen des brevets

(11) EP 3 954 363 A1

(12) EUROPEAN PATENT APPLICATION

(43) Date of publication:
16.02.2022 Bulletin 2022/07

(21) Application number: 21180655.9

(22) Date of filing: 27.02.2014

(51) International Patent Classification (IPC):
*A61K 9/08* (2006.01) *A61K 47/06* (2006.01)
*A61K 47/08* (2006.01) *A61K 47/10* (2017.01)
*A61K 47/14* (2017.01) *A61K 47/20* (2006.01)
*A61K 47/22* (2006.01) *A61K 47/26* (2006.01)
*A61K 31/135* (2006.01) *A61K 31/166* (2006.01)
*A61K 31/365* (2006.01) *A61K 31/429* (2006.01)
*A61K 31/495* (2006.01) *A61K 31/506* (2006.01)
*A61K 31/573* (2006.01) *A61K 31/7048* (2006.01)

(52) Cooperative Patent Classification (CPC):
**A61K 47/14; A61K 9/0014; A61K 9/08; A61K 31/135; A61K 31/166; A61K 31/365; A61K 31/429; A61K 31/495; A61K 31/506; A61K 31/573; A61K 31/7048; A61K 47/06; A61K 47/08; A61K 47/10; A61K 47/20;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2013 US 201361770312 P**
**15.03.2013 US 201361793699 P**

(62) Document number(s) of the earlier application(s) in accordance with Art. 76 EPC:
**14756567.5 / 2 961 397**

(71) Applicant: **Argenta Innovation Limited**
**Auckland (NZ)**

(72) Inventors:
- **BATT, Laurie Robert**
**Auckland (NZ)**
- **WIN, Su**
**Auckland (NZ)**
- **DAVIES, Keryn**
**Auckland (NZ)**

(74) Representative: **Forresters IP LLP**
**Skygarden**
**Erika-Mann-Straße 11**
**80636 München (DE)**

Remarks:
•This application was filed on 21-06-2021 as a divisional application to the application mentioned under INID code 62.
•Claims filed after the date of filing of the application (Rule 68(4) EPC).

# (54) TRANSDERMAL FORMULATIONS

(57) The present invention relates to a non-aqueous composition that comprises a permeation enhancer such as a terpene, for delivering an active ingredient transdermally. The composition comprises at least on one active ingredient, a terpene, and a solvent, such as a non-hydroxyl containing solvent, non-heterocyclic ester solvent and/or a tripropylene glycol alkyl ether. The composition can be used to deliver a range of actives, such as anthelmintics. The present invention provides a platform composition and can be used to deliver a wide variety of active ingredients and combinations thereof transdermally to mammals.

EP 3 954 363 A1

(52) Cooperative Patent Classification (CPC): (Cont.)
**A61K 47/22; A61K 47/26**

## Description

### INCORPORATION BY REFERENCE

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### FIELD OF THE INVENTION

The present invention relates to an anhydrous transdermal formulation that contains a terpene, and more specifically a formulation suitable for transdermal delivery of active therapeutic agents to mammals. The present invention also relates to methods of manufacturing such compositions, and the use such compositions for treating animals.

### BACKGROUND TO THE INVENTION

Skin forms an excellent barrier against drug permeation, due to the rigid lamellar structure of the stratum corneum (SC) lipids. The SC limits the rate of drug transfer through the skin. The rate of transfer is generally too slow for massive systemic absorption, making transdermal application unsuitable for the delivery of large amounts of drug in short periods of time. Transdermal application is more commonly used for the sustained delivery of drugs over a prolonged period of time.

Transdermal formulations typically include permeation enhancers, which improve the rate of transfer of the drug across the skin. Permeation enhancers may act by disrupting the highly ordered structure of stratum corneum lipid, interacting with intercellular protein, and/or improving partition of the drug into the stratum corneum. A problem with transdermal formulations is that these permeation enhancers can cause skin irritation and inflammation.

Small molecules of moderate lipophilicity permeate through skin most easily. The partition co-efficient P in water and a hydrophobic organic solvent, such as octanol or hexane, is a useful measure lipophilicity. Molecules having a low log P (i.e. hydrophilic compounds) have low permeability because partitioning into the skin lipids is low. Permeability at high log P is also low. This may be due to the accumulation of lipophilic drugs in the stratum corneum due to low water solubility.

There is a need for new transdermal formulations that avoid one or more of the aforementioned disadvantages. It is an object of the present invention to go some way to meeting this need; and/or to at least provide the public with a useful choice.

Citation or identification of any document in this application is not an admission that such document is available as prior art to the present invention.

### SUMMARY OF THE INVENTION

In a first aspect the invention relates to **an anhydrous transdermal composition** which may comprise

at least one active ingredient,
a terpene, and
a non-hydroxyl containing solvent.

In another aspect the invention relates to **an anhydrous transdermal composition** which may comprise

at least one active ingredient,
a terpene, and
a non-heterocyclic ester solvent.

In another aspect the invention relates to **an anhydrous transdermal composition** which may comprise

at least one active ingredient,
a terpene, and
a tripropylene glycol alkyl ether.

In another aspect the invention relates to **an anhydrous transdermal composition** which may comprise

levamisole base,
a terpene, and
an anhydrous veterinarily acceptable carrier.

In another aspect the invention relates to an anhydrous transdermal composition comprising

at least one active ingredient having a log P in hexane and water of less than about 8 at pH 7.4,
a terpene, and
a non-hydroxyl containing solvent, a non-heterocyclic ester solvent or a combination thereof.

In another aspect the invention relates to an anhydrous transdermal composition comprising

at least one active ingredient having a log P in octanol and water of less than about 8 at pH 7.4,
a terpene, and
a non-hydroxyl containing solvent, a non-heterocyclic ester solvent or a combination thereof.

In another aspect the present invention relates to an anhydrous transdermal composition comprising

at least one active ingredient,
at least about 20% terpene, and
a non-heterocyclic ester solvent.

In another aspect the present invention relates to an anhydrous transdermal composition comprising

at least one anthelmintic,
a terpene, and
a non-heterocyclic ester solvent.

In another aspect the invention relates to **a method of manufacturing a transdermal composition** which may comprise

mixing a first composition which may comprise an active ingredient that is substantially insoluble in water, and a terpene, with a fatty acid ester, or
mixing a first composition comprising a terpene, with a second composition which may comprise an active ingredient that is substantially insoluble in water and a fatty acid ester, or
mixing a first composition which may comprise a first active ingredient that is substantially insoluble in water, and a terpene, with a second composition which may comprise a second active ingredient that is substantially insoluble in water, and a fatty acid ester,
thereby providing the transdermal composition.

In another aspect the present invention relates to a transdermal composition manufactured by a method of the present invention.
In another aspect the present invention relates to use of a composition of the present invention for treating an animal in need thereof.
In another aspect the present invention relates to a kit comprising a composition of the invention and instructions for use.
Any one or more of the following embodiments may relate to any of the above aspects.
In one embodiment the permeation enhancer may be present from at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 36, 38, 39, 40, 41, 42, 43, 44, 45, 46, 47, 48, 49, 50, 51, 52, 53, 54, 55, 56, 57, 58, 59, or 60% by weight of the composition, and useful ranges may be selected between any of these values.
In one embodiment the non-hydroxyl containing solvent may be a non-heterocyclic ester solvent.
In one embodiment at least one of the active ingredients may be lipophilic.
In one embodiment the active ingredient or ingredients may have a logP (partition coefficient in hexane and water) of less than about 10, 9, 8, 7, 6, 5, 4, 3 or 2. In one embodiment the active ingredient or ingredients may have a logP (partition coefficient in octanol and water) of less than about 10, 9, 8, 7, 6, 5, 4, 3 or 2.

In one embodiment the platform composition of the present invention may deliver active ingredients with a molecular weight of less than about 1000, 900, 800, 700, 600, 500, 400, 300 or 200 $gmol^{-1}$, and useful ranges may be selected between any of these values.

In one embodiment an active ingredient may be selected from

- an anthelmintic,
- a non-steroidal anti-inflammatory,
- a steroidal anti-inflammatory,
- a steroid hormone,
- an anti-histamine
- an anti-emetic,
- a metabolic regulator,
- a productivity regulator,
- a hypothyroidism treatment,
- a behavioural treatment,
- an analgesic,
- a parasiticide,
- an insecticide,
- an anti-microbial,
- an anti-biotic,
- an anti-fungal,
- an anti-viral,
- a coccidostat,
- a skin treatment agent, or
- any combination of one or more of the above.

In one embodiment the non-heterocyclic ester may be present at 1, 2, 3, 4, 5, 6, 7, 8, 9, 10, 11, 15, 13, 14, 15, 16, 17, 18, 19, 20, 21, 22, 23, 24, 25, 26, 27, 28, 29 or 30%, by weight of the composition, and useful ranges may be selected between any of these values.

In one embodiment the non-heterocyclic ester solvent may be a fatty acid ester.

In one embodiment the non-heterocyclic ester solvent is selected from a triglyceride, a glycerol ester or a combination thereof.

In one embodiment the anhydrous veterinarily acceptable carrier may be selected from a non-hydroxyl containing solvent, a non-heterocyclic ester solvent or a combination thereof.

In one embodiment the non-hydroxyl containing solvent or the non-heterocyclic ester solvent may be a fatty acid ester.

In one embodiment the non-hydroxyl containing solvent or the non-heterocyclic ester solvent may be selected from a triglyceride, glycerol ester or combination thereof.

In one embodiment, the composition comprises a tripropylene glycol alkyl ether and a non-hydroxyl containing solvent, a non-heterocyclic ester solvent or a combination thereof.

In one embodiment, the composition comprises a fatty acid ester and a solvent selected from a triglyceride, glycerol ester or combination thereof.

In one embodiment, the composition comprises a fatty acid ester and a glycol ether.

In one embodiment, the composition comprises a glycol ether and a solvent selected from a triglyceride, glycerol ester or a combination thereof.

In one embodiment, the composition comprises a glycol ether, a fatty acid ester and a solvent selected from a triglyceride, glycerol ester or a combination thereof.

In one embodiment, the composition comprises one or more surfactants.

In one embodiment the composition may comprise a surfactant having the following structure:

$$z\text{-}(o\text{-}CR_1R_2CR_3R_4]_n\text{-}OH$$

where

z is an optionally substituted $C_{14}$ to $C_{22}$ linear alkenyl,
$R_1$, $R_2$, $R_3$ and $R_4$ are each independently selected from methyl or hydrogen, and
n is an integer from 1 to 10.

In one embodiment $R_1$, $R_2$, $R_3$ or $R_4$ may be selected from the group consisting of methyl, ethyl and hydrogen.
In one embodiment $R_1$, $R_2$, $R_3$ or $R_4$ may be selected from the group consisting of methyl and hydrogen.
In one embodiment at least one of $R_1$, $R_2$, $R_3$ or $R_4$ may be hydrogen.
In one embodiment at least two of $R_1$, $R_2$, $R_3$ or $R_4$ may be hydrogen.
In one embodiment at least three of $R_1$, $R_2$, $R_3$ or $R_4$ may be hydrogen.
In one embodiment $R_1$, $R_2$, $R_3$ and $R_4$ may be hydrogen.
In one embodiment n may be an integer from 1 to 5, and more preferably from 1 to 3. In one embodiment n is 2.
In one embodiment Z may be mono, di or tri -unsaturated.
In one embodiment Z may be $C_{16}$ to $C_{22}$ linear alkenyl.
In one embodiment Z may be $C_{16}$ to $C_{20}$ linear alkenyl.
In one embodiment Z may be $C_{18}$ linear alkenyl.
In one embodiment Z may be selected from oleyl, elaidy, vaccenyl, linoeyl, linoelaidyl, $\alpha$-linolenyl.
In one embodiment Z may be oleyl.
In one embodiment Z may have four or more carbon-carbon double bonds.
In one embodiment the alkenyl may comprise from 1 to 3 carbon-carbon double bonds.
In one embodiment the alkenyl may have 1 or 2 carbon-carbon double bonds.
In one embodiment the alkenyl may have 1 carbon-carbon double bond.
In one embodiment at least one of the carbon-carbon double bonds of Z may have a cis configuration.
In one embodiment all of the carbon-carbon double bonds of Z may have a cis configuration.
In one embodiment at least one of the carbon-carbon bonds of Z may have a trans configuration.
In one embodiment at least one of the carbon-carbon double bonds may have a cis configuration and at least one of the carbon-carbon double bonds may have a trans configuration.
In one embodiment the composition may comprise more than one surfactant. For example, the composition may comprise two or more surfactants in which the surfactants differ in the number, position or configuration of the carbon-carbon double bonds present in Z.
In one embodiment the surfactant may have a polyoxyethylene (2) oleyl ether such as Brij 93.
In one embodiment the surfactant may provide a hydrophilic-lipophilic balance of about 4.0 to about 8.0, and more preferably about 4.0 to about 6.0.
In one embodiment the surfactant may provide a hydrophilic-lipophilic balance of about 4.5.
In one embodiment the composition comprising the surfactant is stable at 4°C.
In one embodiment the composition comprising the surfactant is stable at 4°C for at least 1, 2, 3, 4, 5, 6, 7, 8, 9, 10, 12, 18, 24, 36, 48, 72, 96, 120, 144, or 168 hrs.
In one embodiment, the composition comprises at least two active ingredients.
In one embodiment the composition may comprise at least two lipophilic active ingredients.
In one embodiment one of the active ingredients may be an imidazothiazole.
In one embodiment the imidazothiazole may be selected from levamisole base, pyrantel pamoate, butamisol or tetramisole.
In one embodiment, at least one of the active ingredients has a log P in hexane and water at pH 7.4 of at least about 4, at least about 5, or at least about 6.
In one embodiment the active may be, or may comprise, a macrocyclic lactone.
In one embodiment the macrocyclic lactone may be selected from avermectin, ivermectin, abamectin, eprinomectin, moxidectin, selamectin, doramectim, milbemycin, abamectin or cydectin.
In one embodiment, the macrocyclic lactone is selected from abamectin and moxidectin.
In one embodiment the composition may comprise

optionally about 1 to about 60% w/w levamisole base,
optionally about 0.1 to about 20% w/w macrocyclic lactone,
optionally about 1 to about 40% w/w fatty acid ester,
optionally about 1 to about 60% w/w terpene, and
optionally about 1 to about 25% w/w non-aqueous solvent.

In one embodiment the terpene may be selected from limonene or phellandrene. In one embodiment, the terpene is limonene.
In one embodiment the first composition may be heated to at least 20°C.
In one embodiment the composition may include a further penetration enhancer in addition to a terpene. In such embodiments the further penetration enhancer is present at 1, 2, 3, 4, 5, 6, 7, 8, 9, 10, 11, 15, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60%, by weight of the composition, and useful ranges may be selected

between any of these values.

In one embodiment the further penetration enhancer that is present with a terpene penetration enhancer is a non-heterocyclic ester. In further embodiments the further penetration enhancer is a fatty acid ester. Preferably the fatty acid ester that may be present as a further penetration enhancer to the terpene is isopropyl myristate, triacetin, propylene glycol octanoate decanoate (PGOD), polysorbate 20, or a mixture thereof.

In one embodiment the composition is administered at 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 1 mL/kg of live weight animal, and useful ranges may be selected between any of these values.

In one embodiment the fatty acid ester may have a $C_8$-$C_{20}$ alkyl chain.

In one embodiment the fatty acid ester may have a $C_{10}$-$C_{16}$ alkyl chain.

In one embodiment the fatty acid ester may be isopropyl myristate.

In one embodiment the non-aqueous solvent may be selected from a glycol ether, a triglyceride, a glycerol ester or a mixture thereof.

In one embodiment the composition may comprise an antioxidant. In one embodiment, the antioxidant stabilises the composition. In one embodiment, the antioxidant stabilises the active ingredient to chemical reaction, degradation, or decomposition in the composition.

In one embodiment the composition delivers at least one of the active ingredients transdermally at an average post-lag flux rate of at least 30, 40, 50, 60, 70, 80, 90, 100, 110, 120, 130, 140, 150, 160, 170, 180, 190, 200, 210, 220, 230, 240, 250, 260, 270, 280, 290 or 300 $\mu g/cm^2/h$, and useful ranges may be selected between any of these values.

In one embodiment the composition delivers a macrocyclic lactone at an average post-lag flux rate of at least 150, 200, 250, 300, 350, 400, 450, 500, 550, 600, 650, 700 $\mu g/cm^2/h$, and useful ranges may be selected between any of these values.

In one embodiment the composition delivers levamisole at an average post-lag flux rate of at least 300, 350, 400, 450, 500, 550, 600, 650, 700, 750, 800, 850, 900, 950, 1,000, 1,050, 1,100, 1,150, 1,200 $\mu g/cm^2/h$, and useful ranges may be selected between any of these values.

In one embodiment, the composition delivers abamectin at an average post lag flux rate of at least 0.1, 0.2, 0.3, 0.4, 0.5 ,0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, or 5.0 $\mu g/cm^2/h$.

In one embodiment, the composition is a veterinary composition.

In one embodiment, the composition is a pour on or spot on formulation.

In one embodiment, the composition is fast acting with respect to at least one active ingredient. In one embodiment, the composition is fast acting with respect to at least one active ingredient relative to at least one other active ingredient in the composition. In one embodiment, the fast acting active ingredient acts within at least 1, 2, 3, 4, 5, 6, 7, 8, 9, 10, 11, 12, 24, 48, 72, 96, 120 hours and useful ranges may be selected from within these values

In one embodiment, the action of at least one of the active ingredients is delayed relative to at least one other active ingredient in the composition. In one embodiment, the action is delayed by at least 1, 2, 3, 4, 5, 6, 7, 8, 9, 10, 11, 12, 13, 14, or 15 days, and useful ranges may be selected from within these values.

In one embodiment, the composition provides sustained or prolonged release of at least one of the active ingredients.

In one embodiment, the pH of the composition is from about 3 to about 12, about 3 to about 11, from about 4 to about 12, from about 4 to about 11, from about 5 to about 12, from about 5 to about 11, from about 5 to about 10, from about 5 to about 9, from about 5 to about 8, from about 6 to about 11, form about 6 to about 10, from about 6 to about 9, from about 7 to about 11, from about 7 to about 10, from about 7 to about 9, or from about 7 to about 8.

In one embodiment, the pH of the composition is at least about 3, 4, 5, 5.5, 6, 6.5, 7, 7.5, or 8.

In one embodiment, the log P of the at least one act ingredient at the pH of the composition is within at least about 3, 3.5, 2, 2.5, 1, 1.5, or 0.5 of the log P of the active ingredient at physiological pH (7.4).

In one embodiment the first composition may be formed from a mix of at least one active ingredient that is substantially insoluble in water, a terpene and a non-aqueous solvent.

In one embodiment the dissolved mixture may be formed from a mix of the first composition and any one or more of

i) an antioxidant,
ii) a non-aqueous solvent,
iii) a fatty acid ester, or
iv) a mixture of any one or more of (i) to (iii).

In one embodiment the non-aqueous solvent may be a glycol ether.

In one embodiment the glycol ether may be a tripropylene glycol alkyl ether.

In one embodiment the tripropylene glycol alkyl ether may be present at about 1, 2, 3, 4, 5, 6, 7, 8 ,9, 10, 11, 12, 13, 14, 15, 16, 17, 18, 19, 20, 21,22, 23, 24, 25, 26, 27, 28, 29, or 30% by weight of the platform composition, and useful ranges may be selected between any of these values.

In one embodiment the tripropylene glycol alkyl ether may be selected from tripropylene glycol methyl ether, tripropylene glycol mono-n-propyl ether or tripropylene glycol mono-n-butyl ether.

In one embodiment the second composition may also include a lipophilic organic antioxidant compound.

In one embodiment the lipophilic organic antioxidant compound is present at about 0.01, 0.02, 0.04 0.06, 0.08, 0.1, 0.15, 0.2, 0.25, 0.3, 0.35, 0.4, 0.45, 0.5, 0.55, 0.6, 0.65, 0.7, 0.75. 0.8, 0.85, 0.9, 0.95, 1.0, 1.5 or 2% by weight of the composition.

Preferably the lipophilic organic antioxidant compound is a phenol derivative such as butylated hydroxytoluene, BHA, tocopherol, propyl gallate, or any combination thereof.

In one embodiment the composition may comprise a reducing agent such as ascorbyl palmitate.

In one embodiment the composition may comprise a synergist such as sodium edentate or propyl gallate.

In one embodiment the heating may be performed for between 10 minutes to 12 hrs or 10 min to 8 hrs. In one embodiment the dissolved mixture is heated for 10, 30, 60, 90, 120, 150, 180, 210, 240, 270, 300, 330, 360, 390, 420, 450, 480, 540, 600, 660, or 720 minutes, and useful ranges may be selected between any of these values.

In one embodiment the dissolved mixture may be heated to 20, 25, 30, 32, 34, 36, 38, 40, 42, 44, 46, 48 or 50°C, and useful ranges may be selected between any of these values.

In one embodiment the heated mixture may be cooled.

In one embodiment the cooled mixture may be packaged.

In one embodiment the composition may have good physical and chemical stability, providing at least 12, 13, 14, 15, 16, 17, 18, 19, 20, 21, 22, 23, 24, 25, 26, 27, 28, 29 or 30 months shelf life, and useful ranges may be selected between any of these values.

In one embodiment the composition may be effective to reduce parasitic faecal egg count by at least 90, 95, 96, 97, 98 or 99%.

In another aspect the invention may be the use of any one or more of the compositions described above.

In one embodiment, the kit comprises a second composition comprising at least one active ingredient, wherein at least one of the active ingredients in the second composition is incompatible with at least one of the active ingredients in the composition of the invention.

In one embodiment, the instructions comprise mixing the composition of the invention and the second composition, and immediately administering the mixture to an animal in need thereof.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7).

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

In this specification, where reference has been made to external sources of information, including patent specifications and other documents, this is generally for the purpose of providing a context for discussing the features of the present invention. Unless stated otherwise, reference to such sources of information is not to be construed, in any jurisdiction, as an admission that such sources of information are prior art or form part of the common general knowledge in the art.

The term "alkenyl" employed alone or in combination with other terms means, unless otherwise stated, a monovalent straight chain hydrocarbon group including a carbon-carbon double bond.

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed

Description.

## BRIEF DESCRIPTION OF THE DRAWINGS

The following detailed description, given by way of example, but not intended to limit the invention solely to the specific embodiments described, may best be understood in conjunction with the accompanying drawings.

Figure 1 depicts a representative schematic of the invention.

Figure 2 depicts a formulation absent surfactant that was examined for moxidectin permeability with n = 5. The results show the permeability of moxidectin over 72 hours. The moxidectin had a flux rate of 475 ± 185.2 ng/cm$^2$/hr from linear section of profile ($R_2$ = 89.4%). Error bars are standard deviation.

Figure 3 depicts formulation absent stability agent that was examined for levamisole permeability with n = 5. The results show the permeability of levamisole over 72 hours. Levamisole has a flux rate of 949.6 ± 80.8 μg/cm$^2$/hr from linear section of profile ($R_2$ = 99.4%). Error bars are standard deviation.

Figure 4 depicts testing of a commercial formulation. The Eclipse PO formulation contains abamectin and levamisole. The results show the permeability of abamectin in Eclipse over 72 hours with n =3. The abamectin had a flux rate of 33.9 ± 26.3 μg/cm$^2$/hr. Error bars are standard deviation.

Figure 5 depicts the permeability of levamisole in Eclipse over 72 hours with n = 3. The levamisole had a flux rate of 204.4 ± 17.1 μg/cm$^2$/hr. Error bars are standard deviation.

Figure 6 depicts the transport of diphenhydramine across rabbit skin (flux rate of 41.47 ± 10.18 μg/cm$^2$/hr).

Figure 7 depicts the transport of diphenhydramine across horse skin (flux rate 25.3 ± 1.9 μg/cm$^2$/hr).

Figure 8 depicts the transport of diphenhydramine across bovine skin (flux rate 95.1 ± 33.1 μg/cm$^2$/hr).

Figure 9 depicts the transport of cetirizine across rabbit skin (flux rate of 4.6 ± 2.7 ± 10.18 μg/cm$^2$/hr).

Figure 10 depicts the transport of cetirizine across horse skin (flux rate 254.4 ± 1.5 μg/cm$^2$/hr).

Figure 11 depicts the transport of cetirizine across bovine skin (flux rate 77.9 ± 14.5 μg/cm$^2$/hr).

Figure 12 depicts the transport of hydrocortisone across rabbit skin (flux rate of 2.9 ± 0.9 μg/cm$^2$/hr).

Figure 13 depicts the transport of hydrocortisone across horse skin (flux rate 10.3 ± 5.8 μg/cm$^2$/hr).

Figure 14 depicts the transport of hydrocortisone across bovine skin (flux rate 61.3 ± 19.1 μg/cm$^2$/hr).

Figure 15 depicts the transport of metoclopramide across rabbit skin (flux rate of 38.6 ± 9.2μg/cm$^2$/hr).

Figure 16 depicts the transport of metoclopramide across horse skin (flux rate 67.0 ± 24.6 μg/cm$^2$/hr).

Figure 17 depicts the transport of metoclopramide across bovine skin (flux rate 109.4 ± 11.8 μg/cm$^2$/hr).

Figure 18 depicts Permeability of abamectin across split bovine skin. Data points are mean, n = 3 ± SD. Triangle = eclipse, Circle = Formulation of Table 11. Square = formulation of Table 12.

Figure 19 depicts Permeability of levamisole across split bovine skin. Data points are mean, n = 3 ± SD. Triangle = eclipse, Circle = Formulation of Table 11. Square = formulation of Table 12.

Figure 20 depicts a FA typical IR spectrum for untreated bovine skin where A = Amide II (weak), B = Amide I, C = $CH_2$ symmetrical stretching, D = $CH_2$ asymmetrical stretching, and E = water content.

## DETAILED DESCRIPTION OF THE INVENTION

The present invention relates to a non-aqueous composition that may comprise a permeation enhancer such as a terpene, for delivering an active ingredient or ingredients transdermally to mammals.

The composition may comprise at least on one active ingredient, a terpene, and a solvent, such as a non-hydroxyl containing solvent, non-heterocyclic ester solvent and/or a tripropylene glycol alkyl ether.

The composition may be used to deliver a range of actives, such as anthelmintics. A non-limiting example of suitable anthelmintics includes levamisole base and macrocyclic lactones. Compositions prepared in accordance with the present invention for delivering animal remedies such as anthelmintics may also include the present of an anhydrous veterinarily acceptable carrier.

The present invention provides a platform composition and may be used to deliver a wide variety of active ingredients transdermally.

### 1. Active Ingredient

The platform composition of the present invention delivers a therapeutic quantity of the active ingredient or ingredients. The active ingredient or ingredients, once applied to the skin of the recipient animal, may be absorbed into the systemic circulation.

A wide range of active ingredients may be delivered transdermally by the platform composition of the present invention.

For example, the active ingredient or ingredients may be lipophilic in nature. Preferably the active ingredient has a logP (partition coefficient in hexane and water at pH 7.4) of less than about 10, 9, 8, 7, 6, 5, 4, 3 or 2 and useful ranges may be selected between any of these values (for example, from about 2 to about 10, from 2 to about 8, from 2 to about 6, from 2 to about 4, from 3 to about 10, from 3 to about 8, from 3 to about 7, from 3 to about 6, from 4 to about 10, from 4 to about 8, from 4 to about 7, from 4 to about 6). Preferably the active ingredient has a logP (partition coefficient in octanol and water at pH 7.4) of less than about 10, 9, 8, 7, 6, 5, 4, 3 or 2 and useful ranges may be selected between any of these values (for example, from about 2 to about 10, from 2 to about 8, from 2 to about 6, from 2 to about 4, from 3 to about 10, from 3 to about 8, from 3 to about 7, from 3 to about 6, from 4 to about 10, from 4 to about 8, from 4 to about 7, from 4 to about 6). Examples of possible lipophilic active ingredients that can be delivered by the platform composition of the present invention include anthelmintics, non-steroidal and steroidal anti-inflammatory agents, anti-emetics, hypothyroidism treatment agents, behavioural treatment agent, or analgesics.

In one embodiment, at least one of the active ingredients has a log P in hexane and water at physiological pH (pH 7.4) of at least about -1, at least about 0, at least about 1, at least about 2, at least about 3, at least about 4, at least about 5, at least about 6, or at least about 7. In one embodiment, at least one of the active ingredients has a log P in octanol and water at physiological pH (pH 7.4) of at least about -1, at least about 0, at least about 1, at least about 2, at least about 3, at least about 4, at least about 5, at least about 6, or at least about 7.

In one embodiment the platform composition of the present invention may deliver multiple lipophilic active ingredients. For example, 2, 3, 4 or 5 actives delivered transdermally in a single composition. Preferably the active ingredients account for 1, 5, 10, 15, 20, 25, 30, 35, 40, 45, 50, 55, 60, 65, 70, 75, 80, 85 or 90% of the composition, and useful ranges may be selected between any of these values (for example, from about 1 to about 90, about 1 to about 75, about 1 to about 60, about 5 to about 100, about 5 to about 80, about 5 to about 55, about 25 to about 90, about 25 to about 65, about 25 to about 50, about 30 to about 90, about 30 to about 80, about 30 to about 70, about 30 to about 55, about 50 to about 90, about 50 to about 75, about 65 to about 90, about 65 to about 75 or about 70 to about 90% of the composition).

In one embodiment the platform composition of the present invention may deliver active ingredients with a molecular weight of less than about 1000, 900, 800, 700, 600, 500, 400, 300, 200 $gmol^{-1}$ and useful ranges may be selected between any of these values (for example, from about 100 to about 1000, about 100 to about 900, about 100 to about 800, about 100 to about 600, about 100 to about 500, about 200 to about 1000, about 200 to about 900, about 200 to about 800, about 200 to about 700, about 200 to about 500, about 200 to about 400, about 300 to about 1000, about 300 to about 900, about 300 to about 700, about 300 to about 500, about 500 to about 1000, about 500 to about 800, about 500 to about 700, about 500 to about 600, about 700 to about 1000, about 700 to about 900, about 800 to about 1000).

In one embodiment, at least one of the active ingredients has a molecular weight of at least about 300, at least about 400, at least about 500, or at least about 600 $gmol^{-1}$.

As mentioned above, anthelmintic agents may be delivered transdermally by the platform composition of the present invention. A candidate anthelmintic agent includes imidazothiazoles. For example, the imidazothiazole may be selected from levamisole, pyrantel pamoate, butamisol or tetramisole. Macrocyclic lactones are also candidate agents for delivery transdermally. For example, the macrocyclic lactone may be selected from avermectin, ivermectin, abamectin,

eprinomectin, moxidectin, selamectin, doramectim, milbemycin, abamectin or cydectin.

If an imidazothiazole, such as levamisole, is incorporated into the platform composition for transdermal delivery, preferably the composition may contain 1, 5, 10, 15, 20, 25, 30, 35, 40, 45, 50, 55 or 60% imidazothiazole, and useful ranges may be selected between any of these values (for example, from about 1 to about 60, about 1 to about 40, about 1 to about 30, about 5 to about 60, about 5 to about 45, about 5 to about 35, about 5 to about 25, about 10 to about 60, about 10 to about 45, about 10 to about 30, about 10 to about 20, about 15 to about 60, about 15 to about 40, about 15 to about 30, about 15 to about 35, about 15 to about 30, about 15 to about 25, about 20 to about 60, about 20 to about 40, about 20 to about 30, about 25 to about 60, about 25 to about 45, about 25 to about 35, about 30 to about 60, about 30 to about 40, about 40 to about 60% imidazothiazole).

If a macrocyclic lactone, such as moxidectin, is incorporated into the platform composition for transdermal delivery, preferably the composition may contain 0.1, 0.5, 1, 2, 3, 4, 5, 6, 7, 8, 9, 10, 11, 12, 13, 14, 15, 16, 17, 18, 19 or 20% macrocyclic lactone, and useful ranges may be selected between any of these values (for example, from about 0.1 to about 20, about 0.1 to about 15, about 0.1 to about 12, about 0.1 to about 10, about 0.1 to about 5, about 0.1 to about 4, about 0.5 to about 20, about 0.5 to about 14, about 0.5 to about 10, about 0.5 to about 5, about 1 to about 20, about 1 to about 16, about 1 to about 11, about 1 to about 6, about 3 to about 20, about 3 to about 13, about 3 to about 7, about 5 to about 20, about 5 to about 14, about 5 to about 10, about 8 to about 20, about 8 to about 12, about 14 to about 20, about 16 to about 20% macrocyclic lactone).

It should be appreciated that the exemplification of anthelmintics such as levamisole and moxidectin is not intended to be limiting, and that other lipophilic active ingredients could also be delivered within the composition.

Anthelmintics include benzimidazoles, imidazothiazoles, tetrahydropyrimidines, macrocyclic lactones, salicylanilides, substituted phenols, aromatic amides, isoquinolines, amino acetonitriles, spiroindoles, and the like.

Anthelmintic benzimidazoles include mebendazole, flubendazole, fenbendazole, oxfendazole, oxibendazole, albendazole, albendazole sulfoxide, thiabendazole, thiophanate, febantel, netobimin, and triclabendazole. Further examples include mebendazole, and ricobendazole.

Benzimidazole based anthelmintics interfere with the worm's energy metabolism on a cellular level. They bind to a specific building block called beta tubulin and prevent its incorporation into certain cellular structures called microtubules, which are essential for energy metabolism. Interfering with energy metabolism is a much more basic mode of activity than that which occurs with other classes of anthelmintics. For this reason, benzimidazoles are also able to kill worm eggs. Benzimidazoles have a wide margin of safety and broad spectrum activity.

Imidazothiazoles and tetrahydropyrimidines and both nicotinic agonists. Anthelmintic imidathiazoles include levamisole, tetramisole, and butamisole. Tetrahydropyrimidine anthelmintics include, for example, morantel, oxantel, and pyrantel.

The tetrahydropyrimidines mimic the activity of acetylcholine, a naturally occurring neurotransmitter that initiates muscular contraction. The worm is unable to feed and quickly starves. Tetrahydroyrimidines only affect adult populations of worms. They do not have activity against the larval stages and are ineffective against cestodes (tapeworms) and trematodes (liver flukes).

Imidazothiaoles have a similar mode of action causing spastic paralysis of the worms. Levamisole has a broad spectrum of activity and is effective against many larval stages of parasites.

Macrocyclic lactones include abamectins, for example abamectin, doramectin, eprinomectin, ivermectin, and selamectin, and milbemycins, for example milbemycin oxime and moxidectin.

The macrocyclic lactones (avermectins and milbemycins) are products or chemical derivatives of soil microorganisms belonging to the genus *Streptomyces.* All of the macrocyclic lactone anthelmintics have the same mode of action. They interfere with GABA-mediated neurotransmission, causing paralysis and death of the parasite. Macrocyclic lactones are the most potent killer of worms and are more persistent in their effect. The duration of persistent activity varies according to the drug and formulation.

Macrocyclic lactones also have the unique quality of also killing several types of external parasite such as lice, mites, and ticks. They have a wide margin of safety for livestock and are effective against all stages of worms, including inactive forms.

Salicylanilides include brotianide, clioxanide, closantel, niclosamide, oxyclozanide, rafoxanide, substituted phenols include bithionol, disophenol, hexachlorophene, niclofolan, menichlopholan, nitroxynil, and aromatic amides include diamfenetide (diamphenethide).

Insoquinoline anthelmintics include praziquantel and epsiprantel. Praziquantel and epsiprantel have high efficacy against cestode parasites at relatively low dose rates but no effect on nematodes. Praziquantel is rapidly and almost completely absorbed from the GI tract.

Amino-acetonitrile derivatives include monepantel, which acts by paralyzing worms by attacking a previously undiscovered receptor HCO-MPTL-1, present only in nematodes.

Further examples of anthelmintics include piperazine and derivatives thereof such as piperazine and diethylcarbamazine (DEC, a derivative of piperazine), benzenesulfonamides such as clorsulon, amidines such as bunamidine,

isothiocyantes such as nitroscanate, and organophosphates such as dichlorvos, and spiroindoles such as derquantel (2- deoxoparaherquamide).

The active ingredient may be a non-steroidal or steroidal anti-inflammatory. Examples of non-steroidal anti-inflammatory drugs include, but are not limited to, acemetacin, acetylsalicylic acid (aspirin), alminoprofen, benoxaprofen, bucloxic acid, carprofen, celecoxib, clidanac, deracoxib, diclofenac, diflunisal, dipyrone, etodolac, fenoprofen, fentiazac, firocoxib, flobufen, flufenamic acid, flufenisal, flunixin, fluprofen, flurbiprofen, ibuprofen, indomethacin, indoprofen, isoxicam, ketoprofen, ketorolac, meclofenamic acid, mefenamic acid, meloxicam, miroprofen, nabumetone, naproxen, niflumic acid, oxaprozin, oxepinac, phenylbutazone, piroxicam, pirprofen, pramoprofen, sudoxicam, sulindac, suprofen, tepoxalin, tiaprofenic acid, tiopinac, tolfenamic acid, tolmetin, trioxaprofen, zidometacin, or zomepirac, pharmaceutically acceptable salts thereof and mixtures thereof.

Most commercially available veterinary NSAIDs belong to two broad classes: carboxylic acid and enolic acid derivatives. The main groups of enolic acids are the pyrazolones (phenylbutazone, oxyphenbutazone, and ramifenazone) and the oxicams (meloxicam, piroxicam, and tenoxicam). Carboxylic acid groups include the salicylates (aspirin), propionic acids (ibuprofen, naproxen, carprofen, ketoprofen, and vedaprofen), anthranilic acids (tolfenamic and meclofenamic acids), phenylacetic acids (acetaminophen), aminonicotinic acids (flunixin), and indolines (indomethacin).

Anti-inflammatory steroids include steroids which have an anti-inflammatory activity either locally or systemically. These are well-known within the art. Non-limiting examples of steroids include the adrenocorticoid steroids, whether endogenous or synthetic. These include, without limitation, hydrocortisone, betamethasone, cortisone, dexamethasone, prednisolone, prednisone, methylprednisilone, triamcinolone, flumethasone, and their pharmaceutically acceptable derivitives. In one embodiment, the steroid is hydrocortisone or cortisone. These steroids may be used at levels known in the art.

The composition may comprise a steroid hormone. Steroid hormones include growth promoters and production enhancers. In one embodiment, the steroid hormone is a natural steroid hormone, such as estradiol, progesterone, and testosterone, or a synthetic steroid hormone, such as trenbolone acetate, estradiol benzoate, estradiol 17$\beta$, and melengestrol acetate, and zeranol.

Steroid hormones include natural and synthetic steroid hormones, steroid hormone precursors, steroid hormone metabolites, and derivatives thereof that are structurally derived from cholesterol. Steroid hormones can be synthesized from cholesterol via pathways that involve cytochrome P450 (cP450) enzymes, which are heme-containing proteins. Exemplary steroid hormones, include, but are not limited to, androgens, estrogens, progestogens, mineralcorticoids, and glucocorticoids. Exemplary androgens include, but are not limited to, testosterone, dehydroepiandrosterone, dehydroepiandrosterone sulphate, dihydrotestosterone, androstenedione, androstenediol, androstanedione, androstanediol, and any combination thereof. Exemplary estrogens include, but are not limited to, estrone, estradiol, estriol, estetrol, equilin, equilenin, and any combination thereof. Exemplary progestogens include, but are not limited to, progesterone, 17-hydroxy- progesterone, pregnenolone, dihydroprogesterone, allopregnanolone, 17-hydroxy- pregnenolone, 17-hydroxy-dihydroprogesterone, 17-hydroxy-allopregnanolone, and any combination thereof. Exemplary mineralcorticoids include, but are not limited to, aldosterone, 11- deoxycorticosterone, fludrocortisones, 1 1-deoxy-cortisol, pregnenedione, and any combination thereof. Exemplary glucocorticoids, include, but are not limited to, cortisol (hydrocortisone), corticosterone, 18-hydroxy-corticosterone, cortisone, and any combination thereof.

The composition may comprise an anti-histamine. Non-limiting examples of suitable antihistamines include clemastine, clemastine fumarate (2(R)-[2-[1-(4-Chlorophenyl)-1-phenyl-ethoxy]ethyl-1-methylpyrrolidine), dexmedetomidine, doxylamine, loratidine, desloratidine and promethazine, and diphenhydramine, or pharmaceutically acceptable salts, solvates or esters thereof.

The composition may comprise an anti-emetic. Non-limiting examples of suitable anti-emetic agents include phenothiazines (e.g., prochloperazine, promethazine, thiethylperazine, perphenazine, chlorpromazine, metopimazine, acepromazine, etc.); 5HT-3 receptor antagonists such as ondansetron, granisetron, tropisetron, dolasetron, hydrodolasetron, azasetron, ramosetron, lerisetron, indisetron and palonosetron; and others such as dimenhydrinate, diphenhydramine (which can also act as an antihistamine), cyclizine, meclizine, promethazine, hyroxyzine, metoclopramide, domperidone, hyoscine, hyoscine hydrobromide, hyoscine hydrochloride, scopolamine, clebopride, alizapride, itopride, bromopride, droperidol, haloperidol, benzquinamide, cerium oxalate, diphenidol, dronabinol, nabilone, ginger, levosulpiride, butorphanol and aprepitant.

The composition may comprise a metabolic regulator, such a hypothyroidism treatment. Metabolic diseases may be inherited or acquired and are clinically important because they affect energy production or damage critical tissues. Storage diseases and inborn defects in metabolism may be either genetic or acquired. The diseases are characterized by the accumulation or storage of specific lysosomal enzyme substrates or byproducts within cells due to the partial or complete deficiency of those enzymes. The development of the metabolic diseases is largely related to production or management factors. However, the pathogenesis of the diseases is primarily related to alterations in metabolism. In many cases the basis of disease is not a congenital or inherited metabolic defect, but rather an increased demand for a specific nutrient that has become deficient. In one embodiment the metabolic regulator is a growth promoter, probotic

or prebiotic, antibiotic or anti-infective supplement, electrolyte, mineral, vitamin, or other nutritional additive.

The composition may comprise a productivity regulator, for example polyethers such as monensin. In one embodiment, the productivity regulator is a productivity enhancer.

The composition may comprise a hypothyroidism treatment. Suitable hypothyroidism treatments include treatment with thyroid hormones and derivatives thereof. Examples of thyroid hormones include thyroixines such as $T_2$, $T_3$ and $T_4$. "$T_3$" refers to the art recognized thyroid hormone, triiodothyronine (also known as (2S)-2-amino-3-[4-(4-hydroxy-3-iodo-phenoxy)-3,5-diiodo-phenyl]propanoic acid). "$T_4$" refers to the art recognized thyroid hormone, thyroxine, or 3,5,3',5'-tetraiodothyronine (also known as (2S)-2-amino-3-[4-(4-hydroxy-3,5-diiododophenoxy)-3,5-diiodophenyl]propanoic acid). "$T_2$" refers to the thyroid hormone iodothyronine or 3,5-diiodo-1-thyronine.

The composition may comprise a behavioural treatment agent. Behavioral treatments include, for example, serotonin reuptake inhibitors and tricyclic antidepressants, pheromones, nutritional products, and calming agents. Examples of serotonin reuptake inhibitors and tricyclic antidepressants include, clomipramine.

The composition may comprise an analgesic. Analgesics include the opioid analgesics such as buprenorphine, butorphanol, dextromoramide, dezocine, dextropropoxyphene, diamorphine, fentanyl, alfentanil, sufentanil, hydrocodone, hydromorphone, ketobemidone, levomethadyl acetate, mepiridine, methadone, morphine, nalbuphine, opium, oxycodone, papaveretum, pentazocine, pethidine, phenoperidine, piritramide, dextropropoxyphene, remifentanil, tilidine, tramadol, codeine, dihydrocodeine, meptazinol, dezocine, eptazocine and flupirtine. Analgesics also include non-opioid analgesics, for example, non-steroidal anti-inflammatories, such as those described herein.

The composition may comprise a parasiticide. Parasiticides include, for example the macrocyclic lactones such as abamectin, ivermectin, eprinomectin, doramectin, moxidectin, selamectin, milbemycin oxime. In one embodiment, the antiparasitic agents include, but are not limited to, endoparasiticidal agents, ectoparaciticidal agents, and endectoparaciticidal agents. Ectoparasiticides include, for example, organochlorines, organophosphates, carbamates, amidines, pyrethrins and synthetic pyrethroids, benzoylureas, juvenile hormone analogues, macrocyclic Lactones, neonicotinoids, phenylpyrazoles, and spinosyns. Endectoparaciticides include, for example, macrocyclic lactones, such as ivermectin. Endoparasiticides include, for example, anhelmintics, such as those described herein. In one embodiment, the antiparasitic agent is an avermectin, milbemycin, phenylpyrazole, nodulisporic acid, clorsulon, closantel, quinacrine, chloroquine, vidarabine, nitenpyram, ivermectin, milbemycine oxime, lufenuron, salimectin, moxidectin, or dorimectin. In a more particular embodiment, the antiparasitic agent is nitenpyram, ivermectin, milbemycine oxime, lufenuron, salimectin, moxidectin, dorimectin, or paraherquamide, or pharmaceutically acceptable salts, solvates or esters thereof.

In one embodiment, the composition comprises an insecticide. Examples of insecticides include, but are not limited to, pyrethrins, pyrethroids, and limonene. Insecticides also include certain parasiticides.

In one embodiment, the composition comprises a skin treatment agent. Skin treatment agents include skin conditioning agents, for example glycerine. Other skin conditioning agents may be used. Categories of skin conditioning agents include, but are not limited to emollients, humectants and plasticizers.

Humectants include, but are not limited to sorbitol, propylene glycol, alkoxylated glucose, hexanetriol, ethanol, and the like. Emollients include, but are not limited to, hydrocarbon oils and waxes; silicone oils; triglyceride esters, acetoglyceride esters, ethoxylated glycerides; alkyl esters; alkenyl esters; fatty acids, fatty alcohols; fatty alcohol ethers; ether-esters (fatty acid esters of ethoxylated fatty alcohols); lanolin and its derivitives; polyhydric alcohols and polyether derivatives; polyhydric alcohol esters; wax esters; beeswax derivatives; vegetable waxes; phospholipids; steroids; and amides. These may all be used at art-established levels.

Other examples of skin treatment agents include anti-ageing agents and wound care agents.

The composition may comprise an anti-microbial. Anti-microbials include antibiotics, antifungals, antivirals, anhelmintics, and the like. Anti-microbials also include

The composition may comprise an antibiotic, antifungal, or antiviral.

The anti-biotic may be an inhibitor of cell wall synthesis (e.g. penicllins, cephalosporins, bacitracin and vancomycin), inhibitor of protein synthesis (aminoglycosides, macrolides, lincosamides, streptogramins, chloramphenicol, tetracyclines), inhibitor of membrane function (e.g. polymixin B and colistin), an inhibitor of nucleic acid synthesis (e.g. quinolones, metronidazole, and rifampin), or an inhibitor of other metabolic processes (e.g. anti-metabolites, sulfonamides, and trimethoprim). Non-limiting examples of antibiotics include polyethers ionophores such as monensin and salinomycin, beta-lactams such as penicillins, aminopenicillins (e.g., amoxicillin, ampicillin, hetacillin, etc.), penicillinase resistant antibiotics (e.g., cloxacillin, dicloxacillin, methicillin, nafcillin, oxacillin, etc.), extended spectrum antibiotics (e.g., axlocillin, carbenicillin, mezlocillin, piperacillin, ticarcillin, etc.); cephalosporins (e.g., cefadroxil, cefazolin, cephalixin, cephalothin, cephapirin, cephradine, cefaclor, cefacmandole, cefmetazole, cefonicid, ceforanide, cefotetan, cefoxitin, cefprozil, cefuroxime, loracarbef, cefixime, cefoperazone, cefotaxime, cefpodoxime, ceftazidime, ceftiofur, ceftizoxime, ceftriaxone, moxalactam, etc.); monobactams such as aztreonam; carbapenems such as imipenem and eropenem; quinolones (e.g., ciprofloxacin, enrofloxacin, difloxacin, orbifloxacin, marbofloxacin, etc.); chloramphenicols (e.g., chloramphenicol, thiamphenicol, florfenicol, etc.); tetracyclines (e.g., chlortetracycline, tetracycline, oxytetracycline, doxycycline, minocycline, etc.); macrolides (e.g., erythromycin, tylosin, tlimicosin, clarithromycin, azithromycin, etc.); lincosamides (e.g., lincomycin, clindamycin, etc.); aminoglycosides (e.g., gentamicin, amikacin, kanamycin, apramycin, tobramycin, neomycin, dihydrostreptomycin, paromomycin, etc.); sulfonamides (e.g., sulfadmethoxine, sfulfamethazine, sulfaquinoxaline, sulfamerazine, sulfathiazole, sulfasalazine, sulfadiazine, sulfabromomethazine, suflaethoxypyridazine, etc.); glycopeptides (e.g., vancomycin, teicoplanin, ramoplanin, and decaplanin; and other antibiotics (e.g., rifampin, nitrofuran, virginiamycin, polymyxins, tobramycin, etc.).

Antifungals include polyenes, azoles, allylamines, morpholines, antimetabolites, and combinations thereof. For example, fluconazole, itraconazole, clotrimazole, ketoconazole, terbinafine, 5-fluorocytosine, and amphotericin B.

Non-limiting examples of antivirals include didanosine, lamivudine, stavudine, zidovudine, indinavir, and ritonavir.

The composition may comprise a coccidostat. Coccidiostats are antiprotozoal agents that acts on Coccidia parasites. Examples include, but are not limited to, amprolium, arprinocid, artemether, clopidol, decoquinate, diclazuril, dinitolmide, ethopabate, halofuginone, lasalocid, monensin, narasin, nicarbazin, oryzalin, robenidine, roxarsone, salinomycin, spiramycin, sulfadiazine, and toltrazuril.

In one embodiment, the active ingredient is stable in the composition. For example, the active ingredient(s) in the composition is typically stable to chemical reaction with other components in the reaction mixture or to degradation or decomposition by other means.

In one embodiment, the composition comprises levamisole base and the recovery of levamisole base after 3 months at 75% relative humidity and 40°C is at least about 95, 96, 97, 98, or 99%. In one embodiment, the recovery is at least about 95%.

In one embodiment, the composition comprises abamectin and the recovery of abamectin after 3 months at 75% relative humidity and 40°C is at least about 95, 96, 97, 98, or 99%. In one embodiment, the recovery is at least about 95%. In one embodiment, the composition comprises abamectin and at least one surfactant. In one embodiment, at least one of the surfactants Is a polyoxyethylene alkenyl ether as described herein.

In one embodiment, the composition comprises moxidectin and the recovery of moxidectin after 3 months at 75% relative humidity and 40°C is at least about 90, 91, 92, 93, 94, 95, 96, 97, 98, or 99%. In one embodiment, the recovery Is at least about 90%.

In one embodiment, the at least one active ingredient is soluble in at least one of the other liquid components of the composition.

In one embodiment, at least one of the active ingredients Is substantially insoluble in water.

### 2. Permeation enhancer

The platform composition of the present Invention may comprise a permeation enhancer, or combination of permeation enhancers. The permeation enhancer, or combination of permeation enhancers, helps to maximise the transport of the active ingredient(s) across the skin by improving partitioning of the active ingredients, and minimises the residency time of the active ingredient(s) on the surface of the skin, reducing the potential for Irritation. In some embodiments, the penetration enhancer acts as a sorption promoter or accelerant.

The permeation enhancer of the present invention may comprise at least a terpene. Terpenes are a diverse class of organic compounds produced from a wide variety of sources. The fundamental building block of terpenes Is the isoprene unit, $C_5H_8$. Larger structures are then formed from multiples of isoprene. Terpenes can be cyclic or acyclic. Some examples of cyclic terpenes are given below.

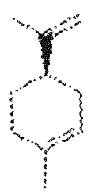
limonene

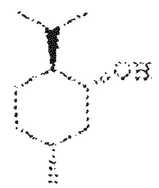
menthol

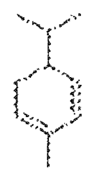
α-phellandrene

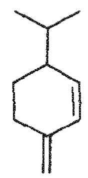
β-phellandrene

The terpene may be a terpene hydrocarbon, terpene alcohol, terpene ketone, or terpene oxide. In one embodiment, the terpene is a terpene hydrocarbon, terpene ketone, or terpene oxide. In another embodiment, the terpene is a terpene hydrocarbon.

In one embodiment, the terpene is a mono-terpene. In another embodiment, the terpene is mono-cyclic or bicyclic.

In one embodiment the terpene may be selected from limonene, menthol, α-phellandrene, β-phellandrene or a combination thereof. In another embodiment, the terpene is selected from limonene, α-phellandrene, and β-phellandrene. In yet another embodiment, the terpene is limonene.

In one embodiment, the composition does not comprise a terpene alcohol. In one embodiment, the composition does not comprise menthol.

In one embodiment, the terpene is the sole permeation enhancer in the composition.

In one embodiment, the composition comprises a single terpene.

In one embodiment the terpene permeation enhancer may be present from at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 36, 38, 39, 40, 41, 42, 43, 44, 45, 46, 47, 48, 49, 50, 51, 52, 53, 54, 55, 56, 57, 58, 59, or 60% by weight of the composition, and useful ranges may be selected between any of these values (for example, from about 2 to about 60, 2 to about 50, 2 to about 40, 2 to about 30, 2 to about 20, 2 to about 10, 5 to about 60, 5 to about 50, 5 to about 40, 5 to about 30, 5 to about 20, 5 to about 10, 10 to about 60, 10 to about 50, 10 to about 40, 10 to about 30, 10 to about 20, 15 to about 60, 15 to about 55, 15 to about 50, 15 to about 45,15 to about 40, about 15 to about 35, about 15 to about 30, about 15 to about 25, about 17 to about 60, about 17 to about 50, about 17 to about 40, about 17 to about 36, about 17 to about 29, about 17 to about 25, about 18 to about 60, about 18 to about 50, about 18 to about 40, about 18 to about 38, about 18 to about 32, about 18 to about 28, about 18 to about 26, about 18 to about 25, about 20 to about 60, about 20 to about 50, about 20 to about 40, about 20 to about 37, about 20 to about 35, about 20 to about 31, about 20 to about 30, about 20 to about 26, about 20 to about 25, about 22 to about 60, about 22 to about 50, about 22 to about 40, about 22 to about 34, about 22 to about 30, about 22 to about 28, about 22 to about 25, about 25 to about 60, about 25 to about 50, about 25 to about 40, about 25 to about 35, about 25 to about 30, about 28 to about 60, about 28 to about 50, about 28 to about 40, about 28 to about 36, about 32 to about 60, about 32 to about 50, about 32 to about 40, about 32 to about 38, about 35 to about 60, about 35 to about 50, about 35 to about 40, about 40 to about 60, about 40 to about 50, about 45 to about 60, about 45 to about 50, about 50 to about 60% by weight of the composition).

In addition to a terpene penetration enhancer, the composition may include a further penetration enhancer. The further penetration enhancer may work in combination with the terpene penetration enhancer.

In one embodiment, the combination of penetration enhancers (i.e. terpene and further penetration enhancer) may possess unique penetration enhancer properties, such as a synergistic interaction to increase the passage of the active ingredient across the skin or a reversible effect on skin lipids.

In one embodiment the further penetration enhancer may be present at 1, 2, 3, 4, 5, 6, 7, 8, 9, 10, 11, 15, 13, 14, 15, 16, 17, 18, 19, 20, 21, 22, 23, 24, 25, 26, 27, 28, 29 or 30%, by weight of the composition, and useful ranges may be selected between any of these values (for example, from about 1 to about 30, about 1 to about 25, about 1 to about 20, about 1 to about 15, about 1 to about 10, about 4 to about 30, about 4 to about 26, about 4 to about 21, about 4 to about 16, about 4 to about 10, about 5 to about 30, about 5 to about 25, about 5 to about 20, about 5 to about 15, about 5 to about 14, about 5 to about 13, about 5 to about 12, about 5 to about 10, about 7 to about 30, about 7 to about 27, about 7 to about 23, about 7 to about 15, about 7 to about 14, about 7 to about 13, about 7 to about 12, about 7 to about 10, about 10 to about 30, about 10 to about 21, about 10 to about 15, about 14 to about 30, about 14 to about 24, about 14 to about 20, about 14 to about 18, about 20 to about 30 or about 24 to about 30% by weight of the composition).

In one embodiment the further penetration enhancer that may be present with a terpene penetration enhancer may be a non-heterocyclic ester. In further embodiments the further penetration enhancer may be a fatty acid ester. In one embodiment the fatty acid ester may have a $C_8$-$C_{20}$ alkyl chain. In one embodiment the fatty acid ester may have a $C_{10}$-$C_{16}$ alkyl chain. In one embodiment the fatty acid ester may be isopropyl myristate.

An example of a fatty acid ester that may be present as a further penetration enhancer to the terpene may be isopropyl myristate, triacetin, propylene glycol octanoate decanoate (PGOD), polysorbate 20, or a mixture thereof.

### 3. Solvents

In one embodiment the platform composition may comprise an anhydrous veterinarily acceptable carrier selected from a non-hydroxyl containing solvent, a non-heterocyclic ester solvent or a combination thereof.

The platform composition of the present invention may comprise at least one solvent. In one embodiment the solvent may be a non-heterocyclic ester.

In one embodiment the non-heterocyclic ester may be present at 1, 2, 3, 4, 5, 6, 7, 8, 9, 10, 11, 15, 13, 14, 15, 16, 17, 18, 19, 20, 21, 22, 23, 24, 25, 26, 27, 28, 29 or 30%, by weight of the composition, and useful ranges may be selected between any of these values (for example, from about 1 to about 30, about 1 to about 25, about 1 to about 20, about 1 to about 15, about 1 to about 10, about 4 to about 30, about 4 to about 26, about 4 to about 21, about 4 to about 16, about 4 to about 10, about 5 to about 30, about 5 to about 25, about 5 to about 20, about 5 to about 15, about 5 to about 14, about 5 to about 13, about 5 to about 12, about 5 to about 10, about 7 to about 30, about 7 to about 27, about 7 to about 23, about 7 to about 15, about 7 to about 14, about 7 to about 13, about 7 to about 12, about 7 to about 10, about 10 to about 30, about 10 to about 21, about 10 to about 15, about 14 to about 30, about 14 to about 24, about 14 to about 20, about 14 to about 18, about 20 to about 30 or about 24 to about 30% by weight of the composition).

In one embodiment the non-heterocyclic ester may be a fatty acid ester. In one embodiment the fatty acid ester

may have a $C_8$-$C_{20}$ alkyl chain. In one embodiment the fatty acid ester may have a $C_{10}$-$C_{16}$ alkyl chain. Preferably the fatty acid ester may be selected from isopropyl myristate, triacetin, propylene glycol octanoate decanoate (PGOD), polysorbate 20, or a mixture thereof.

In one embodiment the platform composition of the present invention may include an additional solvent selected from a triglyceride, glycerol ester or combination thereof. In one embodiment this additional solvent is present only when particular active ingredients are to be delivered. For example, in one embodiment this additional solvent (i.e. triglyceride, glycerol ester or combination thereof) may be present when an active ingredients such as levamisole forms part of the composition. In one embodiment the additional solvent is present at 20, 22, 24, 26, 28, 30, 32, 34, 36, 38, 40, 42, 44, 46, 48, 50, 52, 54, 56, 58 or 50% by weight of the composition, and useful ranges may be selected between any of these values (for example, from about 20 to about 60, about 20 to about 50, about 20 to about 48, about 20 to about 46, about 20 to about 42, about 20 to about 38, about 26 to about 60, about 26 to about 52, about 26 to about 56, about 26 to about 50, about 26 to about 46, about 26 to about 42, about 26 to about 40, about 30 to about 60, about 30 to about 56, about 30 to about 50, about 30 to about 48, about 30 to about 46, about 30 to about 44, about 30 to about 42, about 36 to about 60, about 36 to about 52, about 36 to about 48, about 36 to about 46, about 36 to about 44, about 36 to about 42, about 40 to about 60, about 40 to about 50, about 40 to about 44, about 42 to about 60, about 42 to about 50, about 42 to about 46, about 42 to about 44, about 50 to about 60% by weight of the composition).

In one embodiment the solvents used in the platform composition of the present invention may be non-hydroxyl containing solvents.

In one embodiment the anhydrous veterinarily acceptable carrier may be a non-aqueous solvent. Preferably the non-aqueous solvent may be selected from a glycol ether, a triglyceride, a glycerol ester or a mixture thereof.

In one embodiment the platform composition may include a tripropylene glycol alkyl ether. In one embodiment the tripropylene glycol alkyl ether may be present at about 1, 2, 3, 4, 5, 6, 7, 8 ,9, 10, 11, 12, 13, 14, 15, 16, 17, 18, 19, 20, 21, 22, 23, 24, 25, 26, 27, 28, 29, or 30% by weight of the platform composition, and useful ranges may be selected between any of these values (for example, from about 1 to about 30, about 1 to about 25, about 1 to about 20, about 1 to about 18, about 1 to about 15, about 1 to about 12, about 1 to about 9, about 1 to about 6, about 2 to about 30, about 2 to about 25, about 2 to about 20, about 2 to about 18, about 2 to about 15, about 2 to about 13, about 2 to about 10, about 2 to about 8, about 2 to about 6, about 4 to about 30, about 4 to about 25, about 4 to about 20, about 4 to about 18, about 4 to about 15, about 4 to about 14, about 4 to about 11, about 4 to about 7, about 5 to about 30, about 5 to about 25, about 5 to about 20, about 5 to about 18, about 5 to about 15, about 5 to about 10, about 5 to about 8, about 5 to about 6, about 6 to about 30, about 6 to about 25, about 6 to about 20, about 6 to about 18, about 6 to about 15, about 6 to about 12, about 6 to about 9, about 9 to about 30, about 9 to about 25, about 9 to about 20, about 9 to about 18, about 9 to about 15, about 9 to about 14, about 9 to about 10, about 12 to about 30, about 12 to about 25, about 12 to about 20, about 12 to about 18, about 12 to about 15, about 12 to about 14 or about 13 to about 30, about 13 to about 25, about 13 to about 20, about 13 to about 18, about 13 to about 15, about 15 to about 30, about 15 to about 25, about 15 to about 20, about 15 to about 18, about 18 to about 30, about 18 to about 25, about 18 to about 20, about 20 to about 30, about 20 to about 25, about 25 to about 30% by weight of the platform composition). Preferably the tripropylene glycol alkyl ether is selected from tripropylene glycol methyl ether, tripropylene glycol mono-n-propyl ether, tripropylene glycol mono-n-butyl ether, or tripropylene glycol monomethyl ether (TPGME) or a combination of any two or more thereof.

In one embodiment the selection of the active ingredient may stipulate use of a particular solvent. For example, applicants have found that in those embodiments that contain levamisole base, use of a solvent such as glycerol formal, dimethyl isosorbate (DMI), tetraglycol or a mixture thereof is preferred.

The platform composition of the present invention provides for a high active loading in the composition. In one embodiment the platform composition will also include a co-solvent. Applicants have found that a benefit from the use of the co-solvent is the increased solvency powder of the composition allowing for a higher drug loading in the platform composition.

**4. Surfactant**

In one embodiment the composition may comprise a surfactant. In such an embodiment the surfactant assists maintaining the stability of the composition at low temperatures, for example at fridge storage temperatures of around 4°C.

Any suitable surfactant may be used. The composition may comprise one or more surfactants.

In one embodiment, at least one of the surfactant has the following structure:

$$z\text{-}(o\text{-}CR_1R_2CR_3R_4]_n\text{-}OH$$

where

z is an optionally substituted $C_{14}$ to $C_{22}$ linear alkenyl,

$R_1$, $R_2$, $R_3$ and $R_4$ are each independently selected from methyl or hydrogen, and
n is an integer from 1 to 10.

$R_1$, $R_2$, $R_3$ or $R_4$ may be selected from the group consisting of methyl, ethyl and hydrogen, and more preferably from methyl and hydrogen. Alternately, at least 1, 2, 3 or all of $R_1$, $R_2$, $R_3$ or $R_4$ are hydrogen.
In one embodiment n may be an integer from 1 to 5, preferably from 1 to 3, and more preferably is 2.
Z may be mono, di or tri -unsaturated. In one embodiment Z may have four or more double bonds.
In one embodiment the alkenyl may comprise at least 1 to 3 carbon-carbon double bonds.
Of the carbon-carbon double bonds in Z, at least one of them has a cis configuration. In some embodiments one of the carbon-carbon double bonds may have a trans configuration provided there remains at least one carbon-carbon double bond with a cis configuration. In one embodiment all of the carbon-carbon double bonds have a cis configuration.
In one embodiment Z may be an optionally substituted

- $C_{16}$ to $C_{22}$ linear alkenyl,
- $C_{16}$ to $C_{20}$ linear alkenyl, or
- $C_{18}$ linear alkenyl.

In one embodiment Z is selected from oleyl, elaidy, vaccenyl, linoeyl, linoelaidyl, $\alpha$-linolenyl. In one embodiment Z is oleyl.
The surfactant composition may comprise a mixture of compositions each having a different cis trans configuration. For example, the surfactant may be a mixture of a compound in which all of the carbon-carbon double bonds may have a cis configuration with some compounds in which some of the carbon-carbon double bonds may have a cis and some may have a trans configuration.
In one embodiment, the surfactant is a wetting agent. Examples of wetting agents include ethoxylated fatty alcohols, such as a Brij.
In one embodiment, the surfactant is a polyoxyethylene alkyl ether or a polyoxyethylene alkenyl ether.
In one embodiment the surfactant may be a polyoxyethylene (2) oleyl ether such as Brij 93.
In one embodiment the surfactant may have a hydrophilic-lipophilic balance (HLB) value of about 4.0 to about 8.0, and more preferably about 4.0 to about 6.0.
In one embodiment the surfactant may have a hydrophilic-lipophilic balance of about 4.5.
In one embodiment, the composition may comprise two or more surfactants that in combination provide a hydrophilic-lipophilic balance of about 4.0 to about 8.0, or about 4.0 to about 6.0, or about 4.5. The desired hydrophilic-lipophilic balance may be provided by combining surfactants having different hydrophilic-lipophilic balances, which may or may not be within the desired hydrophilic-lipophilic balance range, in appropriate proportions.
In some embodiments, the one or more surfactant stabilises the composition. In one embodiment, the surfactant inhibits crystallisation or precipitation of one or more components of the composition. In another embodiment, the surfactant inhibits phase separation in the composition.
In one embodiment the composition comprising the surfactant is stable at 4°C.
In one embodiment the composition comprising the surfactant is stable at 4°C for at least 1, 2, 3, 4, 5, 6, 7, 8, 9, 10, 12, 18, 24, 36, 48, 72, 96, 120, 144, or 168 hrs. In one embodiment, the composition may comprise at least one surfactant and is stable at 4 °C for at least 1 day, 2 days, 3 days, 4 days, 5 days, 6 days, 1 week, 2 weeks, 1 month, 2 months, or 3 months.
In one embodiment the composition may comprise at least 0.2, 0.4, 0.6, 0.8, 1, 1.5, 2, 2.5, 3, 3.5, 4, 4.5, 5, 5.5, 6, 6.5, 7, 7.5, 8, 8.5, 9, 9.5, or 10% surfactant by weight of the composition, and useful ranges may be selected between any of these values (for example, from about 0.2 to about 10, about 0.2 to about 8.5, about 0.2 to about 7, about 0.2 to about 6, about 0.2 to about 5, about 0.2 to about 4, about 0.2 to about 3.5, about 0.2 to about 3, about 0.2 to about 2.5, about 0.2 to about 2, about 0.2 to about 1, about 0.2 to about 0.8, about 0.4 to about 10, about 0.4 to about 8.5, about 0.4 to about 7, about 0.4 to about 6, about 0.4 to about 5, about 0.4 to about 4, about 0.4 to about 2, about 0.4 to about 1, about 0.6 to about 10, about 0.6 to about 8.5, about 0.6 to about 7, about 0.6 to about 6, about 0.6 to about 5, about 0.6 to about 4, about 0.6 to about 3.5, about 0.6 to about 2, about 0.6 to about 1.5, about 1 to about 10, about 1 to about 8.5, about 1 to about 7, about 1 to about 6, about 1 to about 5, about 1 to about 4, about 1 to about 3.5, about 1 to about 3, about 1 to about 2.5, about 1 to about 1.5, about 1.5 to about 10, about 1.5 to about 8.5, about 1.5 to about 7, about 1.5 to about 6, about 1.5 to about 5, about 1.5 to about 4, about 1.5 to about 3.5, about 1.5 to about 2.5, about 1.5 to about 2, about 2 to about 10, about 2 to about 8.5, about 2 to about 7, about 2 to about 6, about 2 to about 5, about 2 to about 4, about 2 to about 3.5, about 2 to about 3, about 2 to about 2.5, about 2.5 to about 10, about 2.5 to about 8.5, about 2.5 to about 7, about 2.5 to about 6, about 2.5 to about 5, about 2.5 to about 4, about 2.5 to about 3.5, about 2.5 to about 3.5, about 3 to about 10, about 3 to about 8.5, about 3 to about 7, about 3 to about 6, about 3 to about 5, about

3 to about 4, about 3 to about 3.5 or about 3.5 to about 4, about 4 to about 10, about 4 to about 8.5, about 4 to about 7, about 4 to about 6, about 4 to about 5, about 5 to about 10, about 5 to about 8.5, about 5 to about 7, about 5 to about 6, about 6 to about 10, about 6 to about 8.5, about 6 to about 7, about 7 to about 10, about 7 to about 8.5, about 8.5 to about 10% by weight of surfactant in the composition).

Surfactants suitable for use include ionic and non-ionic detergents, dispersing agents, wetting agents, emulsifiers and the like.

Examples of surfactants include those selected from the group consisting of anionic surfactants, nonionic surfactants, amphoteric surfactants, non-lathering surfactants, emulsifiers and mixtures thereof.

Examples of anionic surfactants include those selected from the group consisting of sarcosinates, sulfates, isethionates, taurates, phosphates, lactylates, glutamates, and mixtures thereof. Anionic surfactants also include fatty acid soaps.

Non-limiting examples of nonionic surfactants include those selected from the group consisting of alkyl glucosides, alkyl polyglucosides, polyhydroxy fatty acid amides, alkoxylated fatty acid esters and fatty alcohols, sucrose esters, amine oxides, and mixtures thereof.

Examples of amphoteric lathering surfactants include derivatives of aliphatic secondary and tertiary amines.

Non-limiting examples of zwitterionic surfactants are those selected from the group consisting of betaines, sultaines, hydroxysultaines, alkyliminoacetates, iminodialkanoates, aminoalkanoates, and mixtures thereof.

Non-limiting examples of non-lathering surfactants include polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryW isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

Surfactants also include silicone surfactants. Examples of silicone surfactants include dimethicone based surfactants, surfactant silicone elastomers, and combinations thereof.

### 5. Kits

The kit of the present invention comprises a composition of the present invention and instructions for use. The kit may comprise a second composition that comprises at least one active ingredient that it incompatible with at least component of the composition of the present invention.

Incompatibility between active ingredients or between active ingredients and excipients, for example, is well known in the art and may prevent the formulation of such components in a single, stable composition.

In one embodiment, the at least one active ingredient of the second composition is incompatible with at least one active ingredient in the composition of the present invention.

The instructions for use may include instructions to mix the composition of the present invention and second composition and to immediately administer the mixture to an animal in need thereof. The mixture is administered immediately to prevent or minimise the incompatibility causing adverse effects on the stability of the active ingredients or other components of the mixture.

In one embodiment, the mixture is administered within about 12, 11, 10, 9, 8, 7, 6, 5, 4, 3, 2, 1 hours after mixing. In another embodiment, the mixture is administered within about 60, 55, 50, 45, 40, 35, 30, 25, 20, 15, 10, or 5 minutes after mixing.

The composition of the invention and the second composition are separated from each other in the kit. The composition may be separated using, for example, a container, divided bottle or divided foil package.

### 6. Method of Manufacture

In one embodiment there is a method of manufacturing a transdermal composition which may comprise

1. mixing a first composition which may comprise an active ingredient that is substantially insoluble in water, and a terpene, with a fatty acid ester, or
2. mixing a first composition which may comprise a terpene, with a second composition which may comprise an active ingredient that is substantially insoluble in water and a fatty acid ester, or
3. a first composition which may comprise a first active ingredient that is substantially insoluble in water, and a terpene, with a second composition which may comprise a second active ingredient that is substantially insoluble in water, and a fatty acid ester,

thereby providing the transdermal composition.

In one embodiment the first composition may include a triglyceride or glycerol ester. Preferably the first composition may contain triacetin (glycerin triacetate).

In one embodiment the second composition may include a tripropylene glycol alkyl ether. Preferably the tripropylene glycol alkyl ether may be selected from tripropylene glycol methyl ether, tripropylene glycol mono-n-propyl ether, tripropylene glycol mono-n-butyl ether, or tripropylene glycol monomethyl ether (TPGME) or a combination of any two or more thereof.

In one embodiment the second composition may include a non-heterocyclic ester. In one embodiment the non-heterocyclic ester may be a fatty acid ester. In one embodiment the fatty acid ester may have a $C_8$-$C_{20}$ alkyl chain. In one embodiment the fatty acid ester may have a $C_{10}$-$C_{16}$ alkyl chain. Preferably the fatty acid ester may be selected from isopropyl myristate.

One example is of an anthelmintic composition. In this example the active ingredient of the first composition may be a lipophilic active such as levamisole base. Preferably the first composition may comprise a combination of levamisole base, a triglyceride or glycerol ester such as triacetin and a terpene penetration enhancer. Preferably the terpene penetration enhancer may be selected from limonene, menthol $\alpha$-phellandrene, $\beta$-phellandrene or a combination thereof. The first composition ingredients are mixed by stirring until a homogenous mixture if the ingredients is formed. The first composition may be combined with at least a fatty acid ester. In one embodiment the second composition may also include a further active ingredient. By way of example, the active ingredient of the second composition may be different to the active ingredient of the first composition. For example, the active ingredient of the second composition may be a macrocyclic lactone.

In one embodiment the second composition may include a triglyceride or glycerol ester. Preferably the second composition may contain triacetin (glycerin triacetate). The triglyceride or glycerol ester may be present in the first composition, the second composition or the first and the second compositions.

The second composition may therefore comprise a second active such as a macrocyclic lactone, a triglyceride or glycerol ester such as triacetin and a tripropylene glycol alkyl ether.

In one embodiment the second composition may also include a lipophilic organic antioxidant compound. In one embodiment the lipophilic organic antioxidant compound is present at about 0.01, 0.02, 0.04 0.06, 0.08, 0.1, 0.15, 0.2, 0.25, 0.3, 0.35, 0.4, 0.45, 0.5, 0.55, 0.6, 0.65, 0.7, 0.75. 0.8, 0.85, 0.9, 0.95, 1.0, 1.5 or 2% by weight of the composition, and useful ranges may be selected between any of these values (for example, from about 0.01 to about 2. 0.01 to about 1, about 0.01 to about 0.85, about 0.01 to about 0.35, about 0.010.2, about 0.01 to about 0.08, about 0.06 to about 2, about 0.06 to about 1.5, about 0.06 to about 0.85, about 0.06 to about 0.45, about 0.06 to about 0.25, about 0.08 to about 2, about 0.08 to about 1.5, about 0.08 to about 0.85, about 0.08 to about 0.45, about 0.08 to about 0.25, about 0.1 to about 2, about 0.1 to about 0.95, about 0.1 to about 0.5, about 0.1 to about 0.25, about 0.15 to about 2, about 0.15 to about 0.95, about 0.15 to about 0.5, about 0.15 to about 0.25, about 0.4 to about 2, about 0.4 to about 1, about 0.4 to about 0.9, about 0.4 to about 0.7, about 0.7 to about 2, about 0.7 to about 1, about 1 to about 2 or about 1.5 to about 2% by weight of the composition).

Preferably the lipophilic organic antioxidant compound may be a phenol derivative such as butylated hydroxytoluene.

In one embodiment the lipophilic organic antioxidant compound may be required to assist or enhance the stability of one or more of the active ingredients. For example, in an anthelmintic platform compositions the lipophilic organic antioxidant compound may enhance the stability of the levamisole base, the macrocyclic lactone or the levamisole base and the macrocyclic lactone.

In one embodiment the lipophilic organic antioxidant compound may assist or enhance the stability of the terpene penetration enhancer. For example, the lipophilic organic antioxidant compound may assist or enhance the stability of the limonene penetration enhancer.

In one embodiment the second composition may comprise an active ingredient, a triglyceride or glycerol ester, a tripropylene glycol alkyl ether, and a non-heterocyclic ester such as a fatty acid ester.

In one embodiment the second composition may comprise an active ingredient, a triglyceride or glycerol ester, a tripropylene glycol alkyl ether, a non-heterocyclic ester such as a fatty acid ester, and a lipophilic organic antioxidant compound.

The first and second compositions may be combined together. As mentioned above, in the instance where the second composition does not include an active ingredient, the second composition may only comprise 1 or more solvents. For example, the first composition may combine solely a fatty acid ester. When the second composition comprises an active ingredient then the second composition may also include additional solvents to the fatty acid ester, such as one or more of a triglyceride or glycerol ester or a tripropylene glycol alkyl ether. The second composition may also comprise the lipophilic organic antioxidant compound regardless of whether the second composition contains an active ingredient or not. It should be appreciated that where the second composition does not comprise an active ingredient, the presence of the lipophilic organic antioxidant compound may act to assist or enhance the stability of the active ingredient in the

first composition. The lipophilic organic antioxidant compound may also act to stabilise the terpene penetration enhancer.

In one embodiment, once combined the mixtures may be mixed until dissolved.

Following dissolution, in one embodiment additional terpene penetration enhancer may be added to the dissolved composition q.s.

Following the option addition of q.s. terpene penetration enhancer to the dissolved mixture, in one embodiment the mixture may then be incubated above room temperature. In one embodiment the incubation is carried out with stirring.

The dissolved mixture may be heated at any suitable temperature. The temperature may depend on the active ingredient(s) present in the mixture. In one embodiment the dissolved mixture may be heated to 20, 22, 24, 26, 28, 30, 32, 34, 36, 38, 40, 42, 44, 46, 48 or 50°C, and useful ranges may be selected between any of these values (for example, from about 20 to about 50, about 20 to about 44, about 20 to about 40, about 20 to about 30, about 20 to about 26, about 24 to about 50, about 24 to about 44, about 24 to about 40, about 24 to about 36, about 24 to about 30, about 30 to about 50, about 30 to about 46, about 30 to about 42, about 30 to about 40, about 34 to about 50, about 34 to about 48, about 34 to about 52, about 34 to about 48, about 34 to about 46, about 34 to about 44, about 34 to about 42, about 34 to about 40, about 36 to about 50, about 36 to about 46, about 36 to about 42, about 36 to about 30, about 38 to about 50, about 38 to about 44, about 38 to about 40, about, about 40 to about 50, about 40 to about 48, about 40 to about 46, about 40 to about 42 or about 44 to about 50°C). Higher temperatures may be suitable, depending on the active ingredient(s) present in the mixture.

In one embodiment the dissolved mixture may be heated for 10, 30, 60, 90, 120, 150, 180, 210, 240, 270, 300, 330, 360, 390, 420, 450, 480, 540, 600, 660, or 720 minutes, and useful ranges may be selected between any of these values (for example, from about 10 to about 720, about 10 to about 660, about 10 to about 600, about 10 to about 540, about 10 to about 480, about 10 to about 360, about 10 to about 240, about 10 to about 120, about 10 to about 60, about 60 to about 720, about 60 to about 660, about 60 to about 600, about 60 to about 540, about 60 to about 480, about 60 to about 390, about 60 to about 330, about 60 to about 240, about 60 to about 180, about 60 to about 120, about 120 to about 720, about 120 to about 660, about 120 to about 600, about 120 to about 540, about 120 to about 480, about 120 to about 420, about 120 to about 300, about 120 to about 270, about 120 to about 240, about 120 to about 180, about 210 to about 720, about 210 to about 660, about 210 to about 600, about 210 to about 540, about 210 to about 480, about 210 to about 390, about 210 to about 360, about 210 to about 270, about 270 to about 720, about 270 to about 660, about 270 to about 600, about 270 to about 540, about 270 to about 480, about 270 to about 420, about 270 to about 360, about 270 to about 300, about 360 to about 720, about 360 to about 660, about 360 to about 600, about 360 to about 540, about 360 to about 480, about 360 to about 420, about 420 to about 720, about 420 to about 660, about 420 to about 600, about 420 to about 540, about 420 to about 480 or about 450 to about 720, about 450 to about 660, about 450 to about 600, about 450 to about 540, about 450 to about 480, about 540 to about 720, about 540 to about 660, about 540 to about 600, about 600 to about 720, about 600 to about 660, about 660 to about 720minutes).

The heated composition may be then cooled and packaged for use.

Once cooled, in one embodiment the composition may be assayed for the active ingredient activity.

In one embodiment the composition may comprise

optionally about 1 to about 60% w/w levamisole base,
optionally about 0.1 to about 20% w/w macrocyclic lactone,
optionally about 1 to about 40% w/w fatty acid ester,
optionally about 1 to about 60% w/w terpene, and
optionally about 1 to about 25% w/w non-aqueous solvent.

In one embodiment the composition may have good physical and chemical stability, providing at least 12, 13, 14, 15, 16, 17, 18, 19, 20, 21, 22, 23, 24, 25, 26, 27, 28, 29 or 30 months shelf life, and useful ranges may be selected between any of these values (for example, from about 12 to about 30, about 12 to about 26, about 12 to about 20, about 12 to about 17, about 13 to about 30, about 13 to about 25, about 13 to about 17, about 13 to about 15, about 16 to about 30, about 15 to about 30, about 15 to about 27, about 15 to about 22, about 15 to about 19, about 14 to about 30, about 14 to about 28, about 14 to about 22, about 14 to about 18, about 14 to about 17, about 17 to about 30, about 17 to about 28, about 17 to about 23, about 17 to about 20, about 18 to about 30, about 18 to about 27, about 18 to about 24, about 18 to about 22, about 20 to about 30, about 20 to about 28, about 20 to about 26, about 25 to about 30, about 25 to about 28, about or about 27 to about 30 months shelf life).

In one embodiment the platform composition may be a solution, suspension, emulsion, microemulsion, or micelle composition.

In another embodiment, the composition may be a homogeneous or heterogeneous mixture, a solution, suspension (e.g. of submicron to micron particles), emulsion, microemulsion, micellar or encapsulated composition.

### 7. Use of the anhydrous transdermal platform composition

The platform composition is capable of delivering a range of drug candidates, including anthelmintics, as single entities or in combination. The platform composition delivers the actives to the systemic circulation by passive diffusion.

The composition is for treating an animal in need thereof. The suitability of the composition for treating a particular disease or condition, for example, depends on the active ingredients present in the composition.

The term "treatment", and related terms, such as "treating" and "treat" as used herein, relates generally to treatment, of either a human or a non-human animal, in which some desired therapeutic effect is achieved. The therapeutic effect may, for example, be the inhibition of progress of a disease or condition, including a reduction in the rate of progress, a halt in the rate of progress, amelioration, and cure. Treatment as a prophylactic measure is also included. Treatment also includes combination treatments and therapies, in which two or more treatments or therapies are used, for example, sequentially or simultaneously, in combination.

The present invention therefore provides use of a composition of the present invention for treating an animal in need thereof.

The present invention also provides a method of treating an animal in need thereof, comprising administering a therapeutically effective amount of a composition of the present invention.

The present invention also provides use of a composition of the present invention in the manufacture of a medicament for treating an animal in need thereof.

The animal to be treated may be human or non-human. Non-human animals include, for example, production animals, such as, cattle, sheep, swine, deer, and goats; companion animals, such as, dogs, cats, and horses; zoo animals, such as, zebras, elephants, giraffes, and large cats; research animals, such as, mice, rats, rabbits, and guinea pigs; fur-bearing animals, such as, mink; birds, such as, ostriches, emus, hens, geese, turkeys, and ducks.

In one embodiment, the animal is a non-human animal. In one embodiment, the animal is a non-human mammal. In one embodiment, the animal is a production animal or companion animal.

In one embodiment, the composition is for treating a helminth infection or infestation. The composition comprises at least one anthelmintic.

Helminths include, but are not limited to, cestodes (flatworms), nematodes (roundworms), and trematodes (flukes), such as, *Trichostrongyloidea,* inlcuding *Haemonchus contortus; Trichostrongylus* spp.; *Dictyocaulus* spp.; *Ascaridoidea,* including *Toxocara* spp.; *Strongylus* spp.; *Filarioidea,* including *Dirofilariaimmitis* and *Onchocerca* spp: *Trematoda,* including *Fasciolahepatica* and *Schistosoma* spp.; *Taenia* spp.; and *Moniezia* spp.; *Ostertagia* spp.; *Nematodirus* spp.; *Cooperia* spp.; *Bunostomum* spp.; *Oesophagostomum* spp.; *Chabertia* spp, *Trichuris* spp.; *Trichonema* spp.; *Capillaria* spp.; *Heterakis* spp.; *Toxocara* spp.;, *Oxyuris* spp.; *Ancylostoma* spp.; *Uncinaria* spp.; *Toxascaris* spp.; and *Parascaris* spp.

In one embodiment, the helminth is selected from *Haemonchus* spp.; Ostertagia spp.; Trichostrongylus spp.; Nematodirus spp.; and Cooperia spp.

The transdermal composition is for topically administration. The composition may be administered, for example, in the form of a sterile cream, gel, pour-on or spot-on formulation, suspension, lotion, ointment, dusting powder, spray, drug- incorporated dressing, skin patch, dip, spray, emulsion, jetting fluid, or shampoo.

In one embodiment, the composition is a pour-on or spot-on formulation. Such formulations may be prepared by the method described herein. Pour-on, spot-on or, spray formulations can be prepared to leave a residue of active agent on the surface of the animal.

Kits of the invention are suitable for administering different dosage forms of more than one anti-parasitic agent by separating the agents using, for example, a container, divided bottle or divided foil package.

A person skilled in the art will be able to readily determine the appropriate dosage of administration for treating an animal. The dosage will depend upon the active ingredient(s) present in the composition and may also depend on the frequency of administration, the sex, age, weight and general condition of the animal treated, the nature and severity of the condition treated, any concomitant diseases to be treated, and any other factors evident to those skilled in the art.

In one embodiment the platform composition may comprise doses at low volume. In one embodiment the composition is administered at 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, or 1 mL/kg of live weight animal and useful ranges may be selected between any of these values (for example, from about 0.005 to about 1, from 0.005 to about 0.8, from 0.005 to about 0.6, from 0.005 to about 0.5, from 0.005 to about 0.3, from 0.005 to about 0.2, from 0.005 to about 0.1, from 0.005 to about 0.05, from 0.005 to about 0.01, from 0.008 to about 1, from 0.008 to about 0.8, from 0.008 to about 0.6, from 0.008 to about 0.5, from 0.008 to about 0.4, from 0.008 to about 0.1, from 0.008 to about 0.09, from 0.008 to about 0.05, from 0.008 to about 0.01, from 0.01 to about 1, from 0.01 to about 0.8, from 0.01 to about 0.6, from 0.01 to about 0.5, from 0.01 to about 0.3, from 0.01 to about 0.1, from 0.01 to about 0.09, from 0.01 to about 0.06, from 0.01 to about 0.05, from 0.01 to about 0.04, from 0.03 to about 1, from 0.03 to about 0.8, from 0.03 to about 0.6, from 0.03 to about 0.5, from 0.03 to about 0.4, from 0.03 to about 0.2, from 0.03 to about 0.1, from 0.03 to about

0.09, from 0.03 to about 0.08, from 0.03 to about 0.06, from 0.03 to about 0.05, from 0.05 to about 1, from 0.05 to about 0.8, from 0.05 to about 0.6, from 0.05 to about 0.5, from 0.05 to about 0.4, from 0.05 to about 0.3, from 0.05 to about 0.1, from 0.05 to about 0.09, from 0.05 to about 0.07, from 0.1 to about 1, from 0.1 to about 0.8, from 0.1 to about 0.6, from 0.1 to about 0.5, from 0.1 to about 0.4, from 0.1 to about 0.3, from 0.3 to about 1, from 0.3 to about 0.8, from 0.3 to about 0.6, from 0.3 to about 0.5, from 0.5 to about 1, from 0.5 to about 0.8, from 0.5 to about 0.6, from 0.6 to about 1, from 0.6 to about 0.8, from 0.8 to about 1 mL/kg of live weight animal). Regardless of this low volume, the platform composition delivers the active ingredients within their therapeutic dose range to the target animal.

The volume of the dose may depend on the active ingredients present in the composition and also on the animal to be treated. For example, the composition may be administered at from about 0.025 mL/ kg to about 0.1 mL/kg for production animals, such as cattle, or from about 0.01 mL/kg to about 0.1 mL/kg for companion animals, such as cats and dogs.

The platform technology provides a number of advantages including

- good wetting/spreading properties,
- no, or very little, hair loss or skin damage,
- no, or very little, apparent residue/oil on skin, and/or
- no, or very little, apparent photosensitivity.

Spreadability can be measured directly by applying a known volume and measuring wetted area. UV light can be used to visualize certain formulations. Residue on skin can be measured by swabbing/extraction. Hair loss and photosensitivity may be evaluated by field observation.

In one embodiment the composition delivers at least one of the active ingredients transdermally at an average post-lag flux rate of at least 100, 110, 120, 130, 140, 150, 160, 170, 180, 190, 200, 210, 220, 230, 240, 250, 260, 270, 280, 290 or 300 $\mu g/cm^2/h$, and useful ranges may be selected between any of these values (for example, from about 100 to about 300, about 100 to about 280, about 100 to about 250, about 100 to about 200, about 100 to about 180, about 120 to about 300, about 120 to about 260, about 120 to about 200, about 120 to about 150, about 160 to about 300, about 160 to about 270, about 160 to about 240, about 160 to about 200, about 190 to about 300, about 190 to about 280, about 190 to about 240, about 190 to about 200, about 210 to about 300, about 210 to about 280, about 210 to about 260, about 210 to about 230, about 240 to about 300, about 240 to about 280, about 240 to about 260, about 260 to about 300, about 260 to about 290, about 280 to about 300 $\mu g/cm^2/h$).

In one embodiment the composition delivers a macrocyclic lactone at an average post-lag flux rate of at least 150, 200, 250, 300, 350, 400, 450, 500, 550, 600, 650, 700 $\mu g/cm^2/h$, and useful ranges may be selected between any of these values (for example, from about 150 to about 700, about 150 to about 600, about 150 to about 500, about 150 to about 400, about 150 to about 200, about 250 to about 700, about 250 to about 600, about 250 to about 500, about 250 to about 400, about 300 to about 700, about 300 to about 650, about 300 to about 450, about 300 to about 400, about 400 to about 700, about 400 to about 650, about 400 to about 600, about 400 to about 500, about 450 to about 700, about 450 to about 650, about 450 to about 500, about 500 to about 700, about 500 to about 600, about 550 to about 700, about 550 to about 650 $\mu g/cm^2/h$).

In one embodiment the composition delivers levamisole at an average post-lag flux rate of at least 300, 350, 400, 450, 500, 550, 600, 650, 700, 750, 800, 850, 900, 950, 1,000, 1,050, 1,100, 1,150, 1,200 $\mu g/cm^2/h$, and useful ranges may be selected between any of these values (for example, from about 300 to about 1200, about 300 to about 1100, about 300 to about 850, about 300 to about 700, about 300 to about 550, about 400 to about 1200, about 400 to about 1050, about 400 to about 950, about 400 to about 650, about 500 to about 1200, about 500 to about 1100, about 500 to about 1000, about 500 to about 950, about 500 to about 850, about 500 to about 700, about 550 to about 1200, about 550 to about 1150, about 550 to about 1000, about 550 to about 700, about 550 to about 600, about 650 to about 1200, about 650 to about 1000, about 650 to about 800, about 650 to about 700, about 750 to about 1200, about 750 to about 1100, about 750 to about 1000, about 750 to about 900, about 750 to about 800, about 800 to about 1200, about 800 to about 1000, about 800 to about 900 950 to about 1200, about 950 to about 1150, about 950 to about 1100, about 950 to about 1000, about 1000 to about 1200, about 1000 to about 1150, about 1000 to about 1100, about 1100 to about 1200 $\mu g/cm^2/h$).

In one embodiment the composition delivers moxidectin at an average post-lag flux rate of at least 300, 350, 400, 450, 500, 550, 600, 650, 700, 750, 800, 850, 900, 950, 1,000, 1,050, 1,100, 1,150, 1,200 $ng/cm^2/h$, and useful ranges may be selected between any of these values (for example, from about 300 to about 1200, about 300 to about 1100, about 300 to about 850, about 300 to about 700, about 300 to about 550, about 400 to about 1200, about 400 to about 1050, about 400 to about 950, about 400 to about 650, about 500 to about 1200, about 500 to about 1100, about 500 to about 1000, about 500 to about 950, about 500 to about 850, about 500 to about 700, about 550 to about 1200, about 550 to about 1150, about 550 to about 1000, about 550 to about 700, about 550 to about 600, about 650 to about 1200, about 650 to about 1000, about 650 to about 800, about 650 to about 700, about 750 to about 1200, about 750

to about 1100, about 750 to about 1000, about 750 to about 900, about 750 to about 800, about 800 to about 1200, about 800 to about 1000, about 800 to about 900 950 to about 1200, about 950 to about 1150, about 950 to about 1100, about 950 to about 1000, about 1000 to about 1200, about 1000 to about 1150, about 1000 to about 1100, about 1100 to about 1200 $ng/cm^2/h$).

In one embodiment the platform composition may be effective to reduce faecal egg count by at least 90, 95, 96, 97, 98 or 99%.

Although the present invention and its advantages have been described in detail, it should be understood that various changes, substitutions and alterations can be made herein without departing from the spirit and scope of the invention as defined in the appended claims.

The present invention will be further illustrated in the following Examples which are given for illustration purposes only and are not intended to limit the invention in any way.

## EXAMPLE 1-STABILITY STUDIES

### 1. Solvent Stability

The purpose of these studies was to identify solvents and co-solvents to optimise solubility of actives and the physical/chemical stability of product.

The solubility, and recovery after one month, of levamisole base and/or abamectin were measured in a range of solvents, as shown in **Table 1.**

**Table 1. Solubility data for solvents which were used individually to check the stability of the two actives levamisole base and abamectin**

| Solvent | Active | Maximum Solubility %w/w | % Recovery after 1 month at 50°C |
|---|---|---|---|
| Tripropylene glycol methylether (TPGME) | Lev. Base | 38 | 97.7 |
| | Abamectin | 8 | 78.7 |
| Dimethyl Isosorbate (DMI) | Lev. Base | 37 | 101.3 |
| Isosorbide | Abamectin | At least 1 | 89.3 |
| Triacetin | Lev. Base | 13 | 98.1 |
| | Abamectin | 3 | 83.8 |
| Isopropyl Mirystate | Lev. Base | 4 | 115.2 |
| | Abamectin | At least 1 | 96.9 |
| AGNIQUE AMD 810 | Lev. Base | At least 20 | 102.6 |
| | Abamectin | At least 1 | 85 |
| AGNIQUE AMD 10 | Lev. Base | At least 20 | 94.6 |
| | Abamectin | At least 1 | 84 |
| Teric 169 | Lev. Base | 19 | 101.8 |
| Glycerol Formal | Lev. Base | More than 50 | 101.5 |
| Isopropyleneglycol | Lev. Base | 62 | 101.2 |
| Estol 1526 | Lev. Base | 9 | 101.2 |
| Tetraglycol | Lev. Base | 49 | 100.9 |
| DMSO | Lev. Base | At least 20 | 98.9 |
| Propylene Glycol | Lev. Base | 19 | 96.7 |
| Ecoteric T80 | Lev. Base | At least 20 | 96.6 |
| Dipropylenglycol dimethylether | Lev. Base | 16 | 94.9 |
| Cremophor | Lev. Base | At least 20 | 91.1 |

(continued)

| Solvent | Active | Maximum Solubility %w/w | % Recovery after 1 month at 50°C |
|---|---|---|---|
| Tersperse 4894 | Lev. Base | At least 20 | 91 |
| Soy Bean Oil with mixed tocopherols | Lev. Base | At least 20 | 87.5 |
| PGOD Propylene Glycol Octanoate Decanoate | ML | 2 | 92.9 |
| Crodamol | ML | At least 1 | 89.9 |
| Polysorbate 20 | Lev. Base | At least 20 | 68.3 |
| | Abamectin | At least 1 | 76.6 |
| DMSO | Lev. Base | At least 20 | 98.9 |
| | Abamectin | At least 1 | 76.5 |
| Ecoteric T80 | Lev. Base | At least 20 | 96.6 |
| | Abamectin | At least 1 | 71.6 |
| Dipropylenglycol dimethylether | Lev. Base | 16 | 94.9 |
| | Abamectin | At least 1 | 70.6 |
| Isopropyleneglycol | Lev. Base | 62 | 101.2 |
| | Abamectin | 7 | 69.1 |
| Tetraglycol | Lev. Base | 49 | 100.9 |
| | Abamectin | 8 | 68.9 |
| Glycerol Formal | Lev. Base | More than 50 | 101.5 |
| | Abamectin | 19 | 59.1 |
| Cremophor | Lev. Base | At least 20 | 91.1 |
| | Abamectin | At least 1 | 58.7 |
| Span 80 | Lev. Base | At least 20 | 72.1 |
| | Abamectin | At least 1 | 79.1 |
| Soy Bean Oil with mixed tocopherols | Lev. Base | At least 20 | 87.5 |
| Mineral oil | Lev. Base | 0.51 | 117.6 |
| PEG400 | Abamectin | At least 1 | 81.3 |
| Span 80 | Abamectin | At least 1 | 79.1 |

**2. Abamectin stability**

The purpose of this study was to examine the stability of abamectin in glycerol formal (GF) alone and solvent systems containing complexing (e.g. PVP), acidifiers (e.g. malic acid) and chelating agents (e.g. EDTA) in combination with glycerol formal.

**Table 2. Stability data for Levamisol base and abamectin in solvent system containing glycerol formal (GF).**

| No. | Formulation | % Abamectin recovery After 1 week at 50°C |
|---|---|---|
| 1 | Lev + Aba + GF | 89.1 |
| 2 | Lev + Aba + GF + Malic (0.5%) | 94.5 |
| 3 | Lev + Aba + GF + oxalic (0.5%) | 94.0 |

(continued)

| No. | Formulation | % Abamectin recovery After 1 week at 50°C |
|---|---|---|
| 4 | Lev + Aba + GF + citric (2.0%) | 93.3 |
| 5 | Lev + Aba + GF + PVP (5.0%) | 93.4 |
| 6 | Lev + Aba + GF + EDTA (0.1%) | 91.2 |
| 7 | Lev + Aba + GF + methionine (2.0%) | 83.9 |

As shown in **Table 2,** the percentage recovery of abamectin after one week at 50°C was highest in those formulations that contained malic, oxalic, and citric acid, and PVP. A formulation comprising levamisole, abamectin and glycerol formal did not perform as well as the formulations containing malic, oxalic, and citric acid, and PVP.
Other solvent systems comprising tripropylene glycol methylether (TPGME), isopropylenglycol and tetraglycol to replace glycerol formal were evaluated.
TPGME was found to be a good candidate.

**3. Carrier stability**

These studies looked at the stability of compositions of the invention which may comprise the carriers dimethyl isosorbate (DMI), triacetin, agnique 810, and isopropyl mirystate (IPM) if necessary as a penetration enhancer.
This study also looked at tripropylene glycol methylether (TPGME) or tetraglycol or isopropyleneglycol.
This study also further analysed the effect of the following on stability.

- The addition of acidifiers. e.g. Malic acid
- The addition of complexing agents. e.g. PVP.
- Use of non polar solvents. e.g. limonene.

**Table 3. Formulation stability (Stability data after two weeks stored at 50°C)**

| # | Ingredients | | | | | | | | | | % Recovery after 2 weeks | |
|---|---|---|---|---|---|---|---|---|---|---|---|---|
| | GF | DMI | Triacetin | PVP + malic acid 5%-0.5% | EDTA | PGOD | Isopropyl Myristate | Limonene | Agnique 810 | Agnique 10 | Levamisole base | Abamecti |
| 1 | 12% | 60% | | | | QS | | | | | 101 | 83 |
| 2 | 12% | 60% | | √ | | QS | | | | | 99 | 81 |
| 3 | 12% | 60% | | | | | | QS | | | 102 | 94 |
| 4 | 12% | 60% | | √ | | | | QS | | | 111 | 88 |
| 5 | 32% | | 35% | | | QS | | | | | 100 | 70 |
| 6 | 32% | | 35% | √ | | QS | | | | | 94 | 68 |
| 7 | 12% | 30% | 30% | | | | | QS | | | 97 | 87 |
| 8 | 12% | 30% | 30% | √ | | | | QS | | | 96 | 82 |
| 9 | 12% | 30% | 30% | √ | 0.1% | | | QS | | | 101 | 82 |
| 10 | 22% | | 35% | | | | | QS | 10% | | 105 | 79 |
| 11 | 22% | | 35% | √ | | | | QS | 10% | | 99 | 76 |
| 12 | 12% | | | | | | 10% | | QS | | 91 | 70 |
| 13 | 32% | | | | | | 10% | | | QS | 103 | 78 |

**Table 4. Formulation stability (stability data after two months stored at 30°C, 40°C and 50°C)**

| Formulation # | Ingredients | | | | | | Active | % Recovery after 2 months | | |
|---|---|---|---|---|---|---|---|---|---|---|
| | Tetraglycol | IPM | TPGME | Isopropylene glycerol | Triacetin | Limonene | | 30°C | 40°C | 50°C |
| 14 | 20% | | | | 42 | QS | Levamisole | | | 97.3 |
| | | | | | | | Abamectin | 96.7 | 94.5 | 64.8 |
| 15 | | 10% | 6% | | 42 | QS | Levamisole | | | 99.7 |
| | | | | | | | Abamectin | 95.8 | 87.4 | 68.4 |
| 16 | | | 12% | 7% | 42 | QS | Levamisole | | | 97.5 |
| | | | | | | | Abamectin | 94.6 | 89.1 | 68.5 |
| 17 | | | | 19% | 42 | QS | Levamisole | | | 98.1 |
| | | | | | | | Abamectin | 91.2 | 85.7 | 62.2 |

As shown in **Table 3,** DMI, triacetin and limonene demonstrated good recovery of levamisole and abamectin when incorporated into the formulation.

As shown in **Table 4** each of the formulations tested demonstrated good recovery of levamisole and abamectin after two months at 30°C, reduced but still good recovery of abamectin after two months at 40°C, and reduced abamectin recovery after two months at 50°C.

**4. Stability with formulations comprising surfactants**

The purpose of this study was to examine the stability of the formula which may comprise surface active agents such as surfactants.

The formulation of the platform composition is given in **Table 5.**

**Table 5. Test composition**

| Ingredient | Classification | Quantity (%) |
|---|---|---|
| Abamectin or Moxidectin | Macrocyclic lactone | 1 |
| Levamisole (Base) | Imidazothiazole derivative | 20 |
| Triacetin | glycerin triacetate / glycerol triester | 42 |
| Isopropyl mirystate (IPM) | FA ester | 10 |
| tripropylene glycol monomethyl ether (TPGME) | Glycol ether | 6 |
| Limonene | cyclic terpene | QS (21) |
| **TOTAL** | | **100** |

A screen of a various surfactants was conducted to improve the formulation stability of the test composition containing 20% w/w Levamisole base in combination with either 1 % w/w Abamectin or 1 % w/w Moxidectin. Three temperatures (RT, 4.5°C and -20°C) were used to monitor for phase separation and/or drug crystal formation every one or two days from 6 days. Each surfactant was added to the test composition as a percentage of weight, therefore changing the concentrations of the original ingredients. However, most of the tests used less than 4% of each surfactant. Only in the 5% and 10% Brij 93 samples was the higher concentration of surfactant present likely to have an effect on the test composition.

The formulations were studied at room temperature (24 ± 1°C) and fridge temperature (4.2-4.5°C).

As shown in Table 6 each of the surfactants tested maintained the solubility of the test composition at room temperature.

Brij 93 (polyoxyethylene (2) oleyl ether) was effective in maintaining the stability of the test composition at fridge temperature (~4°C).

**Table 6. List of selected and tested surfactants for stability of test composition**

| Agent | HLB** | RT | Fridge |
|---|---|---|---|
| - | - | S | US |
| Brij 93 | 4.5 ± 0.4 | S | S* |
| Brij 78 | 15.3 | S | US |
| Brij 72 | 4.9 | S | US |
| Brij 52 | 5.0 | S | US |
| Tocopherol | 6.0 | S | US |
| Span 20 | 8.6 | S | US |
| Span 60 | 4.7 | S | US |
| Span 80 | 4.3 | S | US |
| Lauroglycol | 5.0 | S | US |
| Pluronic P 123 | 7 - 9 | S | US |

(continued)

| Agent | HLB** | RT | Fridge |
|---|---|---|---|
| Tween 80 | 15 | S | US |
| Labrafil M | 4.0 | S | US |
| PC-3 (lethicin) | 8.5 | ND | - |

KEY
US = unstable, phase separation, crystals
S = stable, no crystals
ND = not dissolvable
* = stable for at least 12 days
** = at RT

**Table 2. Stability study using Brij 93 for the test composition**

| % weight | RT | Fridge (2/3 days) | Fridge (4 days) | Fridge (6 days) | Freezer (2 days) | Freezer (4 days) |
|---|---|---|---|---|---|---|
| 0.2 | S | US - 1 | US - 1 | US - 1 | - | - |
| 0.5 | S | US - 1 | US - 1 | US - 1 | - | - |
| 1.0 | S | US - 1 | US - 1 | US - 1 | - | - |
| 1.5 | S | US | US - 1 | US - 1 | - | - |
| 2.0 | S | S | US - 1 | US - 1 | US - 1 | - |
| 3.0 | S | S | US - 1 | US - 1 | US | - |
| 3.5 | S | S | US - 2 | US - 1 | - | US - 3 |
| 4.0 | S | S | US - 2 | US - 1 | - | US - 3 |

KEY Stability and type;
US = unstable, two phases, minimal crystal formation, reversible on shaking
US - 1 = unstable, phase separation, irreversible crystal formation
US - 2 = unstable, no phase separation, irreversible crystal formation
US - 3 = unstable, phase separation, but no crystal formation
S = stable, no phase separation, no crystal formation

**EXAMPLE 2-EX VIVO STUDIES**

**5. Permeability studies**

This example describes the use of the platform composition to deliver a range of different actives across the skin of a range of different animals (cow, horse, rabbit).

The actives investigated in this example are

- levamisole base (anthelmintic),
- macrocyclic lactones (abamectin and moxidextin) (anthelmintics),
- hydrocortisone (steroidal anti-inflammatory),
- metoclopramide (antiemetic),
- cetirizine (antihistamine), and
- diphenhydramine (anti-histamine).

The characteristics for each of the actives tested are shown below in **Table 7.**

**Table 7. Characteristics of the APIs tested**

| API | Solubility (Aq) | Mol Weight | logP | pKa (acidic) | pKa (acidic) | pKa (basic) | MP (°C) |
|---|---|---|---|---|---|---|---|
| Abamectin | Insoluble | 873 | 4.4 | | | | 156 |
| Levamisole (base) | Insoluble | 204 | 1.8 | 8 | | | 60 |
| Moxidectin | Insoluble | 640 | 6.0 | | | | 150 |
| Diphenhydramine HCl | Insoluble | 292 | 3.5 | 8.9 | | | 168 |
| Cetirizine 2HCl | 10mg/mL | 462 | 3.0 | 2.2 | 2.9 | 7.8 | 112.5 |
| Metacloprimide HCl | Freely soluble | 300 | 2.6 | 9.3 | | | 147 |
| Hydrocortisone (base) | Soluble | 263 | 1.6 | 12.6 | | -2.8 | 220 |

The formulation of the platform composition is given in **Table 8.**

**Table 8. Formulation for abamectin/levamisole base formulation**

| Ingredient | Classification | Quantity (%) |
|---|---|---|
| Abamectin | anthelmintic active | 1 |
| Levamisole base | anthelmintic active | 20 |
| Triacetin | glycerin triacetate / glycerol triester | 42 |
| Isopropyl mirystate (IPM) | FA ester | 10 |
| tripropylene glycol monomethyl ether (TPGME) | glycol ether | 6 |
| Limonene | cyclic terpene | QS (21) |
| **TOTAL** | | **100** |

**Table 9. Formulation for moxidectin/levamisole base formulation**

| Ingredient | Classification | Quantity (%) |
|---|---|---|
| Moxidectin | anthelmintic active | 1 |
| Levamisole base | anthelmintic active | 20 |
| BHT (Butylated hydroxytoluene) | anti-oxidant | 0.1 |
| Ethanol (EtOH) | solvent | 1 |
| Butyl cellosolv acetate (BCA) | solvent | 5 |
| tripropylene glycol monomethyl ether (TPGME) | glycol ether | 6 |
| Isopropyl mirystate (IPM) | FA ester | 10 |
| Triacetin | glycerin triacetate / glycerol triester | 50 |
| Limonene | cyclic terpene | QS (~12) |
| **TOTAL** | | **100** |

**Table 10. Formulation for non-anthelmintic actives**

| Ingredient | Classification | Quantity (%) |
|---|---|---|
| API | Active | 1 |
| Triacetin | glycerin triacetate / glycerol triester | 54.31 |

(continued)

| Ingredient | Classification | Quantity (%) |
|---|---|---|
| Isopropyl mirystate (IPM) | FA ester | 6.47 |
| BHT (Butylated hydroxytoluene) | Anti-oxidant | 0.13 |
| Brij 93 | Surfactant | 6.47 |
| tripropylene glycol monomethyl ether (TPGME) | Glycol ether | 7.76 |
| Limonene | cyclic terpene | QS (~24) |
| **TOTAL** | | **100** |

A further formulation as follows was examined.

**Table 11. Formulation for abamectin/levamisole combination**

| Ingredient | Classification | Quantity (%) |
|---|---|---|
| Abamectin | Active | 1 |
| Levamisole base | Active | 20 |
| TPGME | Glycol ether | 6 |
| IPM | FA ester | 10 |
| Triacetin | glycerin triacetate / glycerol triester | 42 |
| BHT | Anti-oxidant | 0.1 |
| Limonene | cyclic terpene | q.s. (~23 |

A further formulation as follows was examined.

**Table 12. Modified formulation for abamectin/levamisole combination**

| Ingredient | Classification | Quantity (%) |
|---|---|---|
| Abamectin | Active | 1 |
| Levamisole base | Active | 20 |
| TPGME | Glycol ether | 6 |
| IPM | FA ester | 5 |
| Triacetin | glycerin triacetate / glycerol triester | 42 |
| Brij 93 | Surfactant | 5 |
| BHT | Anti-oxidant | 0.1 |
| Limonene | cyclic terpene | q.s. (~23 |

### 5.1 Methods

#### 5.1.1 Skin Sample Preparation

Full thickness rabbit or horse skin samples and half thickness bovine (~500 μm) skin samples were excised from different animals above the scapular and over the withers regions. The bovine samples were obtained from 2-5 year old steers, horse samples were obtained from the abattoir, and rabbit skin from euthanized animals after research studies. Immediately after excision, the skin was wrapped in aluminium foil, put into plastic bags, and stored at below 6°C for delivery to the research laboratory. Hair of the collected skin was cut using a hair clipper with surgical blade 50 (0.4 mm) and the underlying excessive fat layer was also carefully removed from the hypodermis using a scalpel (size 20) and discarded. The skin samples were then frozen at ≤ -20°C for further preparation and stored for no more than

16 weeks prior to use.

Before each experiment, partially thawed skin was sheared to remove the entire hair layer and skin samples of 500 ± 30 μm thickness were cut using a dermatome. The dermatome was fitted with duplex blades from Stericut, Reference S11-103. Skin samples with epidermis/dermis were then finally cut into approximately 2 to 2.5 $cm^2$ and placed on a Franz cell between the donor (containing 1 mL formulation) and receptor compartments (12 mL).

The *ex vivo* permeation of each drug was determined by using Franz diffusion cell; VTC 200 by Logan Instruments Corporation (New Jersey, USA), which takes its name from Dr. Thomas Franz who first popularised this method. This method has been used in many skin permeation studies, including topical and transdermal drug delivery formulations, as well as opthalmics, cosmetics, skin care products and pesticides. This system is approved by the FDA. See "Guidance for Industry Nonsterile Semisolid Dosage Forms Scale-Up and Postapproval Changes: Chemistry, Manufacturing, and Controls; In Vitro Release Testing and In Vivo Bioequivalence Documentation", U.S. Department of Health and Human Services, Food and Drug Administration, Center for Drug Evaluation and Research (CDER), May 1997, SUPAC-SS, CMC 7.

Briefly described, the Franz Cell chamber is an in vitro skin permeation assay that consists of two primary chambers separated by a membrane. Bovine skin was used as the membrane. The sub-cutaneous layer was first removed by scalpel having a thickness of 0.1 to 1 mm. The skin sample was placed in propylene glycol 40% v/v (q.s. Milli Q water) and phosphate buffer to pH 7.4 at a temperature of 39°C ± 1°C and equilibrated for 12 hours. The bovine epidermis hair was sheared to 0.4 cm and the remaining hair sheared to just above the stratum corneum layer. The epidermis and some dermis was removed (approximately 500 μm) and sections were cut to 2 cm by 2 cm for each Franz cell unit. The test composition was loaded into a loading volume of 1.0 mL and applied to the membrane via the top chamber. The bottom chamber contains fluid from which samples are taken at regular intervals for analysis. This testing determines the amount of active that has permeated the membrane at each time point. The chamber is maintained at a constant temperature of 37° C.

The moxidectin, abamectin and levamisole tests on bovine skin was tested using the Franz cell system FDC-6 (Logan Instruments, USA). The reminder of the APIs were tested using a new Franz cell system DHC-6T (Logan Instruments, USA). Further more, the new test included the use of dry heated cell as opposed to a water bath, syringe sample collection and the use of Teflon (TFE) coated cells to prevent bubble formation.

The samples were taken up to 58 hrs post-administration and drug concentrations were measure by UHPLC.

The *in vitro* method (Franz Cell) differs to *in vivo* because it uses dead skin with no functioning vascular system. Therefore, the drug needs to permeate through the entire piece of skin before being collected in the receptor fluid for analysis. To compare the actual flux rates of the drugs, the lag time before drug enters the receptor fluid was ignored, and the rate of increasing concentration was calculated from when the active was detected in the fluid. This removes some of the variability due to skin thickness.

The bovine skin was mounted on the receptor compartment with the SC side facing upwards into the donor compartment (12 mL and 1 mL, respectively). Receptor medium was composed of propylene glycol (PG) 39%, ethanol (1%), and buffer phosphate 0.1M (19% $NaH_2PO_4$ 0.1M and 81% $Na_2HPO_4$ 0.1M, pH 7.4). Ethanol was added to the receptor medium to maintain sink condition for the lipophilic drugs which only partially mimics *in vivo* where blood flow provides constant elimination by transport of the drug away from the site of delivery. The medium was proved to have no drug degradation at 40°C for 12 days. Buffer was used due to the presence of basic the drug: LEV ($pK_a$ = 8). The available diffusion area of cell was 1.77 $cm^2$. The receptor compartment was maintained at 39 ± 1°C and stirred by a magnetic bar at 600 rpm, which also helps improve the problem of a static diffusion cell system (i.e. increased drug dissolution). At periodic intervals, samples (400 μL) were withdrawn from the sampling port and immediately replaced by an equal volume of fresh receptor medium which was pre-warmed at 39 ± 1°. The samples were then analyzed by an HPLC method with UV detection. All formulations were tested at least in triplicate.

### 5.1.2 Moxidectin Skin Deposition Study

The amount of moxidectin accumulating in the bovine skin was determined using whole skin thickness (approx. 4 cm) and split skin thickness (approx. 500 μM) after 72 hours. The skin tissue was physically broken down using a Gentle MACS dissociator (Miltenyi Biotec GmbH, Germany) with 5 mL of methanol added into each sample. The dissociator setting was RNA-02.01 with M-type tubes for 90 seconds. Centrifuged samples had the supernatant collected and diluted 2 times with ACN before being analysed by the HPLC method.

### 5.2 Results

### 5.2.1 Permeability Studies

The formulation of **Table 9** was tested for its flux across bovine skin with n = 5. The results are shown in Figure

2 that shows the permeability of moxidectin over 72 hours. The moxidectin had a flux rate of 475 ± 185.2 ng/cm$^2$/hr from linear section of profile ($R^2$ = 89.4%). Error bars are standard deviation.

The formulation of **Table 9** was tested for its flux across bovine skin with n = 5. The results are shown in Figure 3 that shows the permeability of levamisole over 72 hours. Levamisole has a flux rate of 949.6 ± 80.8 μg/cm$^2$/hr from linear section of profile ($R^2$ = 99.4%). Error bars are standard deviation.

A commercial formulation was also tested. The Eclipse PO formulation contains abamectin and levamisole. The results are shown in Figure 4 that shows the permeability of abamectin in Eclipse over 72 hours with n = 3. The abamectin had a flux rate of 33.9 ± 26.3 μg/cm$^2$/hr. Error bars are standard deviation. The results are shown in Figure 5 that shows the permeability of levamisole in Eclipse over 72 hours with n = 3. The levamisole had a flux rate of 204.4 ± 17.1 μg/cm$^2$/hr. Error bars are standard deviation.

The formulation containing diphenhydramine exhibited a flux rate of 41.47 ± 10.18 μg/cm$^2$/hr in rabbit skin (see Figure 6), 25.3 ± 1.9 μg/cm$^2$/hr in horse skin (see Figure 7), and 95.1 ± 33.1 μg/cm$^2$/hr in bovine skin (see Figure 8) as shown below in **Table 13.**

**Table 13. Diphenhydramine permeability through rabbit, horse and bovine skin.**

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 0 | 0 | 0 | 0 | 0 | 0.0 | 0.0 |
| 2 | 0 | 0 | 0 | 0 | 132 | 114 |
| 4 | 0 | 0 | 0 | 0 | 278 | 52.1 |
| 6 | 0 | 0 | 0 | 0 | 388 | 77.5 |
| 8 | 0 | 0 | 0 | 0 | 621 | 100 |
| 10 | 9.25 | 16.0 | 9.25 | 16.0 | | |
| 20 | 378 | 242 | 375 | 97.2 | 1765 | 624 |
| 24 | 455 | 286 | 466 | 144 | 2290 | 816 |
| 26 | 542 | 267 | 482 | 136 | 2514 | 872 |
| 28 | 594 | 236 | 529 | 124 | | |
| 44 | 1025 | 196 | 1011 | 50.0 | 3157 | 1256 |
| 46 | 1168 | 330 | 1052 | 85.9 | | |
| 48 | 1320 | 340 | 1113 | 86.3 | 3218 | 1326 |
| 50 | 1414 | 376 | 1127 | 52.9 | | |
| 52 | 1529 | 429 | 1208 | 103 | 3272 | 1331 |
| 68 | 2425 | 749 | 1514 | 62.8 | 3318 | 1394 |
| 70 | 2539 | 782 | 1560 | 57.2 | 3229 | 1411 |
| 72 | 2547 | 787 | 1569 | 82.3 | 3408 | 1428 |

The formulation containing cetirizine exhibited a flux rate of 4.6 ± 2.7 μg/cm$^2$/hr in rabbit skin (see Figure 9), 4.4 ± 1.5 μg/cm$^2$/hr in horse skin (see Figure 10), and 77.9 ± 14.5 μg/cm$^2$/hr in bovine skin (see Figure 11) as shown below in **Table 14.**

**Table 14. Cetirizine permeability through rabbit, horse and bovine skin.**

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 0 | 0.00 | 0.00 | 0.0 | 0.0 | 0 | 0 |
| 2 | 0.00 | 0.00 | 0.0 | 0.0 | 67.5 | 55.9 |
| 4 | 0.00 | 0.00 | 0.0 | 0.0 | 86.0 | 69.7 |

(continued)

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 6 | 9.13 | 15.82 | 0.0 | 0.0 | 26.7 | 11.5 |
| 8 | 6.18 | 10.70 | 0.0 | 0.0 | 452.1 | 29.9 |
| 10 | 22.6 | 7.60 | 13.9 | 0.0 | | |
| 20 | 30.6 | 4.64 | 43.1 | 3.4 | 1152 | 67.1 |
| 24 | 37.9 | 6.80 | 48.8 | 8.7 | 1201 | 101.4 |
| 26 | 40.0 | 4.48 | 50.3 | 11.8 | 1484 | 88.9 |
| 28 | 43.6 | 4.12 | 61.1 | 21.5 | | |
| 44 | 147 | 81.9 | 155 | 50.9 | 3726 | 799 |
| 46 | 161 | 87.8 | 176 | 65.6 | | |
| 48 | 162 | 82.3 | 183 | 76.6 | 3726 | 799 |
| 50 | 171 | 81.8 | 192 | 72.1 | | |
| 52 | 186 | 91.7 | 206 | 70.7 | 3879 | 829 |
| 68 | 280 | 148 | 261 | 83.2 | 4619 | 994 |
| 70 | 285 | 133 | 262 | 81.4 | 4799 | 1277 |
| 72 | 275 | 123 | 263 | 87.6 | 4723 | 1154 |

The formulation containing hydrocortisone exhibited a flux rate of 2.9 ± 0.9 μg/cm$^2$/hr in rabbit skin (see Figure 12), 10.3 ± 5.8 μg/cm$^2$/hr in horse skin (see Figure 13), and 61.3 ± 19.1 μg/cm$^2$/hr in bovine skin (see Figure 14) as shown below in **Table 15.**

**Table 15. Hydrocortisone permeability through rabbit, horse and bovine skin.**

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 0 | 0 | 0 | 0 | 0 | 0 | 0 |
| 2 | 0 | 0 | 0 | 0 | 1.16 | 2.02 |
| 4 | 0 | 0 | 0 | 0 | 57.8 | 84.8 |
| 6 | 4.63 | 8.02 | 0 | 0 | | |
| 8 | 12.41 | 14.58 | 0 | 0 | 287 | 189 |
| 10 | 4.12 | 0.88 | 2.33 | 2.02 | | |
| 20 | 19.83 | 6.49 | 19.8 | 7.76 | 981 | 187 |
| 24 | 43.41 | 35.17 | 27.0 | 14.6 | 1227 | 296 |
| 26 | 65.56 | 47.41 | 29.3 | 17.5 | 1470 | 382 |
| 28 | 47.36 | 34.05 | 41.1 | 31.7 | | |
| 44 | 112 | 52.5 | 160 | 141 | 2645 | 760 |
| 46 | 113 | 56.2 | 179 | 155 | | |
| 48 | 116 | 55.2 | 200 | 162 | 2686 | 1029 |
| 50 | 131 | 62.1 | 245 | 208 | | |
| 52 | | | | | 2870 | 1050 |

(continued)

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 68 | 157 | 49.1 | 423 | 300 | 3084 | 1279 |
| 70 | 158 | 46.0 | 438 | 300 | 3270 | 1349 |
| 72 | 166 | 51.4 | 448 | 294 | 3016 | 1101 |

The formulation containing metoclopramide exhibited a flux rate of 38.6 ± 9.2 μg/cm$^2$/hr in rabbit skin (see Figure 15), 67.0 ± 24.6 μg/cm$^2$/hr in horse skin (see Figure 16), and 109.4 ± 11.8 μg/cm$^2$/hr in bovine skin (see Figure 17) as shown below in **Table 16.**

**Table 16. Metoclopramide permeability through rabbit, horse and bovine skin.**

| Time | Rabbit | | Horse | | Bovine | |
|---|---|---|---|---|---|---|
| | Mean (μg/cm2) | SD | Mean (μg/cm2) | SD | Mean (μg/cm2) | SD |
| 0 | 0 | 0 | 0 | 0 | 0 | 0 |
| 2 | 0 | 0 | 0 | 0 | 16.2 | 28.0 |
| 4 | 0 | 0 | 0 | 0 | 251 | 83.6 |
| 6 | 0 | 0 | 0 | 0 | 456 | 205 |
| 8 | 0 | 0 | 68.1 | 118 | 960 | 378 |
| 10 | 32.3 | 28.0 | 679 | 596 | | |
| 20 | 685 | 235 | 1372 | 181 | 2188 | 322 |
| 24 | 772 | 214 | 1533 | 404 | 2252 | 440 |
| 26 | 831 | 191 | 1477 | 296 | 2718 | 482 |
| 28 | 925 | 233 | 1640 | 370 | | |
| 44 | 1537 | 167 | 2604 | 722 | 4779 | 502 |
| 46 | 1612 | 231 | 2784 | 831 | | |
| 48 | 1567 | 257 | 2910 | 986 | 5169 | 653 |
| 50 | 1782 | 398 | 2930 | 1041 | | |
| 52 | 1968 | 346 | 3078 | 929 | 5394 | 340 |
| 68 | 2391 | 569 | 3350 | 948 | 5336 | 459 |
| 70 | 2387 | 469 | 3440 | 678 | 5613 | 405 |
| 72 | 2515 | 643 | 3454 | 1043 | 5346 | 711 |

A summary of the lag T, flux rate, APC and extent (72 hrs) for diphenhydramine, cetirizine, hydrocortisone and metoclopramide is shown in **Table 17** below.

**Table 17. A summary of the lag T, flux rate, APC and extent (72 hrs) for diphenhydramine, cetirizine, hydrocortisone and metoclopramide.**

| Active | Skin type | lag T (hrs) | Flux μg/cm$^2$/hr | APC cm/s × $10^{-8}$ | Extent 72h μg/cm$^2$ |
|---|---|---|---|---|---|
| **Diphenhydramine** | **Rabbit** | 13.6 (2.01) | 41.5 (10.2) | 46.3 (14.3) | 2547 (787) |
| | **Horse** | 6.03 (4.17) | 25.3 (1.91) | 28.5 (1.49) | 1569 (82.3) |
| | **Bovine** | 0.72 (0.27) | 95.1 (33.1) | 61.9 (25.9) | 3408 (1428) |

(continued)

| Active | Skin type | lag T (hrs) | Flux $\mu g/cm^2/hr$ | APC cm/s $\times 10^{-8}$ | Extent 72h $\mu g/cm^2$ |
|---|---|---|---|---|---|
| **Cetirizine** | **Rabbit** | 10.3 (4.55) | 4.61 (2.69) | 4.99 (2.24) | 275 (123) |
| | **Horse** | 8.83 (2.32) | 4.41 (1.53) | 4.78 (1.59) | 263 (87.6) |
| | **Bovine** | 2.61 (1.42) | 77.9 (14.5) | 85.8 (21.0) | 4723 (1154) |
| **Hydrocortisone** | **Rabbit** | 8.14 (5.08) | 2.92 (0.96) | 3.01 (0.93) | 166 (51.4) |
| | **Horse** | 21.6 (16.9) | 10.3 (5.83) | 8.14 (5.35) | 448 (294) |
| | **Bovine** | 2.44 (1.27) | 61.2 (19.1) | 54.8 (20.0) | 3016 (1101) |
| **Metoclopramide** | **Rabbit** | 4.84 (4.18) | 38.6 (9.21) | 45.7 (11.7) | 2515 (643) |
| | **Horse** | 3.11 (3.10) | 67.0 (24.6) | 62.7 (18.9) | 3454 (1043) |
| | **Bovine** | 1.07 (0.50) | 109 (11.8) | 97.1 (12.9) | 5346 (711) |

The cumulative amount (Qt) of active permeated through the skin was calculated using the following equation:

$$Qt = [VrCt + \sum_{t=0}^{t-1} VnCn]\frac{1}{A}$$

where *Vr* is the volume of the receptor chamber (12 mL), *Ct* is the drug concentration in the receptor chamber at each time interval, *Vn* and *Cn* are the volume and concentration for the cumulated number of samples withdrawn and A is the relative diffusion surface area (1.77 $cm^2$). The amount of active permeated over 24 hours was plotted over time (hours). Regression analysis was carried out on linear regions of each plot. The lag time, $Lag_t$, was then calculated using the steady state flux (Jss) by measuring the linear portion of the cumulative penetration curve to the time axis where drug release was equal to zero, such that the following formula can be deducted:

$$Lag_t = \frac{h^2}{6D}$$

where, h is the skin membrane thickness ($\mu$m) and D is the diffusion coefficient provided that the membrane thickness is available. On the permeation profile the Flux ($\mu g \cdot cm^{-2} \cdot h^{-1}$) was represented by the *y*-axis and time (t) was plotted on the x-axis. The apparent permeability coefficient ($P_{app}$) was calculated using the following equation.

$$P_{app} = (dX_r / dt)\frac{1}{A} \cdot C_o$$

where $P_{app}$ is determined with the final units as $cm \cdot s^{-1}$, $X_r$ is the amount of active in the receptor chamber, A is the surface area of skin exposed ($cm^2$), and $C_0$ is the initial active concentration at specific time point ($\mu g \cdot mL^{-1}$).

The formulations of **Table 11** and **Table 12** were also tested against Eclipse.

Both abamectin and levamisole were able to permeate across *in vitro* bovine split skin over 72 hours. Figures 18 and 19 show the cumulative drug mass ($\mu g/cm^2$) over time (hrs) for abamectin and levamisole, respectively.

**Table 18. Permeability parameters for abamectin**

| Abamectin | Lag T (hrs) | Extent 48 hrs ($\mu$g/cm2) | Extent 72 hrs ($\mu$g/cm2) | Flux ($\mu$g/cm2/hr) | Papp (cm/s x 10-8) |
|---|---|---|---|---|---|
| Eclipse PO | 6.12 $\pm$ 0.58 | 105.45 $\pm$ 33.71 | 198.92 $\pm$ 72.39 | 2.77 $\pm$ 0.71 | 3.61 $\pm$ 1.32 |
| Formulation of Table 11 | 6.91 $\pm$ 1.46 | 186.34 $\pm$ 30.95 | 186.34 $\pm$ 30.95 | 2.66 $\pm$ 0.38 | 3.34 $\pm$ 0.56 |

(continued)

| Abamectin | Lag T (hrs) | Extent 48 hrs (μg/cm2) | Extent 72 hrs (μg/cm2) | Flux (μg/cm2/hr) | Papp (cm/s x 10-8) |
|---|---|---|---|---|---|
| Formulation of Table 12 | 21.15 ± 9.56 | 85.94 ± 10.66 * | 166.96 ± 38.79 | 4.73 ± 1.76 | 3.03 ± 0.71 |
| * = 60 hrs | | | | | |

**Table 19. Permeability parameters for levamisole.***

| LEV | Lag T (hrs) | Extent 48 hrs (μg/cm2) | Extent 72 hrs (μg/cm2) | Flux (μg/cm2/hr ) | Papp (cm/s x 10-8) |
|---|---|---|---|---|---|
| Eclipse PO | 0.31 ± 0.23 | 47659.57 ± 2821.42 | 77606.34 ± 11011.15 | 991.83 ± 35.46 | 1409.64 ± 200.01 |
| Formulati on of Table 11 | 2.62 ± 1.24 | 49697.63 ± 6850.21 | 83933.69 ± 17451.81 | 982.76 ± 112.13 | 1524.57 ± 316.99 |
| Formulati on of Table 12 | 2.54 ± 0.78 | 34080.96 ± 8486.37 | 68480.85 ± 15841.05 | 784.71 ± 84.17 | 1262.05 ± 287.84 |
| * Data from split skin samples | | | | | |

Table 18 presents the permeability parameters for ABM from each formulation. At steady-state, ABM was observed to have a $P_{app}$ of 3.3 ± 0.6 cm/s × $10^{-8}$ from the Formulation of **Table 11,** while the Formulation of **Table 12** had a $P_{app}$ of 3.0 ± 0.7 cm/s × $10^{-8}$. The mean lag time for the Formulation of Table 11 was significantly shorter to that of the Formulation of **Table 12,** 6.9 hours compared to 21.2 hours, respectively (p-value < 0.01). A shorter lag time is ideal, indicating that steady state is reached more quickly. In this case, although the lag time for the Formulation **of Table** 12was much longer than the Formulation of **Table 11** it was able to almost reach a similar mean permeability extent after 72 hours, 166.9 and 186.3 μg/$cm^2$, respectively. Furthermore, this delayed ABM permeability but similar extent of permeability across the bovine skin, explains the improved flux observed from the Formulation of Table 12. Interestingly, the Formulation of Table 12 had a higher flux (permeability rate) of 4.6 ± 1.8 μg/$cm^2$/hr, while Formulation of Table 11 had a flux of 2.7 ± 0.4 μg/$cm^2$/hr, although these results were not significantly different (p-value > 0.05). For clarity, flux is the slope of the amount permeated over time, hence the delayed ABM permeation from Formulation of Table 12 compared to Formulation of **Table 11,** yet similar permeation extent, meant the slope (flux) from Formulation of Table 12 was steeper compared to that of Bola (original). Finally, the Formulation of Table 11 and Eclipse PO had generally similar ABM permeability parameters, see Table 2. The one way ANOVA results are given in Table 3.

For Levamisole, the Formulation of Table 11 had a lag time of 2.6 ± 1.2 hours and the Formulation of Table 12 had a similar lag time of 2.5 ± 0.8 hours (p-value > 0.05). A summary of the permeability parameters describing the *in vitro* permeability of LEV are given in Table 4. The one way ANOVA results are given in Table 5.

**5.2.2 Moxidectin Deposition Study**

The proportion of the drug at the three compartments, donor, skin and receptor at 72 hours is shown below.

**Table 20. Table showing the proportion of the drug in the three compartments: donor, skin and receptor at 72 hours.**

| | Receptor | Skin | Donor |
|---|---|---|---|
| Whole skin | 3% | 32% | 65% |
| 500 μm skin | 31% | 18% | 51% |

**6. Upper skin permeability**

The purpose of this study is to test the permeability of the active ingredients through the upper skin layer only. The upper skin provides the "real" barrier to the transport of actives across the skin. *In vivo,* once an active penetrates

the skin it is then transported away by a network of blood vessels.

When testing whole skin *in vitro,* the whole skin provides a variable result and probably gives an artificially low estimate of permeability for lipophilic compounds that tend to be trapped in the subcutaneous fat when no blood vessels are present to transport the actives away. More hydrophilic compounds progress faster into the receptor fluid.

## 6.1 Method

A skin sample is prepared for the Franz cell technique, which has been described above. The skin sample is prepared absent the subcutaneous fat.

The test composition is used to determine the flux rate of moxidectin and levamisole.

## 6.2 Result

A result showing a higher flux rate that for whole skin moxidectin and/or levamisole demonstrates that moxidectin and/or levamisole pass the upper skin barrier easily and that the sub cutaneous layers limit their passage in vitro.

## 7. Effect of limonene on skin disruption

The purpose of these studies was to examine the effects of limonene on skin disruption.

## 7.1 Method

The Franz chamber, as described above, was used to determine permeability.

The test composition was carried out with three different concentrations of limonene: 6, 12 and 24% by weight.

### 7.1.1 Histology

Histology was carried out to look at the effect of the formulation on the degree of stratum corneum disruption. The skin tissue was sectioned and stained and the examined under a light microscope or electron microscope to visualize or differentially identify microscopic structures through the use of histological stains.

### 7.1.2 FTIR

Fourier transform infrared spectroscopy (FTIR) was used to measure how well the samples absorbed light at each wavelength.

Lipid disruption was monitored by IR spectrum. A change in water content observed in IR spectrum was used as an indication of lipid disruption.

## 8. Results

### 8.1.1 Histology

Microscopic analysis of the membrane exposed to 6% limonene showed the top layer begins to lift from the epidermis. Membrane exposed to 12% limonene exhibited a broken layer of epidermis. Membrane exposed to 24% limonene exhibited a highly disrupted and mashed layer of epidermis.

The results show that the top layer of the epidermis is damaged. However, this damage is reversible as the top layer is refreshed almost constantly. The results also showed that there was no damage to the dermis or the epidermis.

Histological analysis also showed that there was increased oil in the epidermis, which suggests that the limonene may result in pushing cells apart to create channels through which the active ingredients can more easily pass.

### 8.1.2 FTIR

A typical IR spectrum for untreated bovine skin is shown as Figure 18 where A = Amide II (weak), B = Amide I, C = $CH_2$ symmetrical stretching, D = $CH_2$ asymmetrical stretching, and E = water content.

Shown below in Table 21 is a comparison of IR spectrum data for untreated and treated skin.

**Table 21. Comparison of IR spectrum data for untreated and treated skin**

| Formulation | E (AUC) | D (cm-1) | C (cm-1) | B stretching (cm-1) | A stretching (cm-1) |
|---|---|---|---|---|---|
| Untreated skin | 302.5 ± 13.3 | 2920.2 ± 0.7 | 2850.9 ± 0.4 | 1634.7 ± 0.6 | 1547.0 ± 7.5 |
| PE 6% PO | 250.4 ± 8.2 | 2929.1 ± 3.7 | 2855.1 ± 2.0 | 1636.0 ± 0.5 | 1555.7 ± 0.7 |
| PE 12% PO | 244.1 ± 8.8 | 2929.2 ± 4.3 | 2855.4 ± 1.4 | 1643.0 ± 6.0 | 1555.7 ± 0.3 |
| 24 % PO | 250.6 ± 9.2 | 2929.0 ± 2.0 | 2852.9 ± 7.2 | 1639.0 ± 6.1 | 1556.1 ± 0.2 |

The data suggests that the formulation does cause a significant disruption to the structure of the SC probably by causing changes to the lipid layers. There does not appear to be any apparent cellular damage of the SC. This (transient) disruption of the lipid layer will potentially "open" a passage for the diffusion of drug. This is confirmed by the FTIR studies below.

**EXAMPLE 3-CLINICAL STUDIES**

Two clinical efficacy studies were carried out. The study design for each study is summarised below.

- Study 1-Winter coat
  - Control
  - Test composition with no rain
  - Test composition with rain 2 hours after application
- Study 2-Summer coat
  - Control
  - Test composition
  - Comparator product (Eclipse-combination dual pour-on containing abamectin and levamisole)
  - Single-active comparator product

The purpose of study 1 was to evaluate the efficacy of the test composition against gastrointestinal parasites in cattle with a winter coat, and to determine the effect of rain, after application of the composition to the skin of the cattle, on the composition's efficacy.

The purpose of study 2 was to evaluate the efficacy of the test composition against gastrointestinal parasites in cattle with a summer coat, and to compare against the comparator product Eclipse.

The test composition is shown below in **Table 22.**

**Table 22. Composition for efficacy study**

| Ingredient | Classification | Quantity (%) |
|---|---|---|
| Abamectin | Macrocyclic lactone | 1 |
| Levamisole (Base) | Imidazothiazole derivative | 20 |
| Triacetin | glycerin triacetate / glycerol triester | 42 |
| Isopropyl mirystate (IPM) | FA ester | 10 |
| tripropylene glycol monomethyl ether (TPGME) | Glycol ether | 6 |
| Limonene | cyclic terpene | QS (21) |
| **TOTAL** | | **100** |

The test composition was observed to be a clear straw-coloured solution with a citrus like smell, with good syringability. It was found to be free flowing, to readily wet the hair coat and rapidly passed down the hair coat to the skin, without leaving any oily residue.

## 9. Efficacy study - Winter coat

### 9.1 Treatment groups

The study comprised six infected beef and dairy calves per treatment group.

Treatment Groups consisted of Group 1 that remained as untreated controls, Group 2 were animals treated with the test composition applied to a dry coat then showered with 10 mL simulated rain 2 hours after treatment, then protected from any rain for at least 24 hours and Group 3 were animals treated with the test composition applied to a dry coat and then protected from any rain for at least 24 hours.

**Table 23. Treatment groups**

| Group Number | Treatment | Animal Number |
|---|---|---|
| 1 | Untreated Control | 6 |
| 2 | Test composition<br>10 mL Simulated Rain at 2 hours | 6 |
| 3 | Test composition<br>No rain for 24 hours | 6 |
| TOTAL | | 18 |

Each animal was treated at a rate of 1 ml/20 kg body weight, which equates to 500 μg macrocyclic lactone and 10 mg levamisole per kg.

### 9.2 Study design

This study was carried out as a randomised, stratified controlled study on cattle, less than 12 months of age, with winter coats and a mean weight of 118.5 kg.

The test composition was administered as a single topical treatment to two groups of dry cattle at standard label dose rates, based on individual body weight. Two hours after treatment, one group was sprayed with 10 mL of simulated rain by overhead nozzles, and then kept dry until 24 hours after treatment. The other pour-on group was kept dry for 24 hours.

Efficacy of treatment was measured by faecal egg counts at 6 and 10 days post treatment and at slaughter on Day 13. Efficacy was also measured 13 days after treatment by abomasal, small intestinal and large intestinal worm counts, assessed by genus and stage, relative to the parasite burden in the control group animals, with speciation of appropriate worm genera. Lungworm burden in the control group was investigated by counting lungworm in three animals to determine if a suitable lungworm burden was present, and if further lungworm counts were justified.

Clinical behavioural observations and measurements and pour-on site inspections post treatment were made. Two separate studies were performed. The first study was carried out on beef and dairy calves with a winter coat. Testing was carried out without and with rain 2 hours after application The second study was carried out on beef and dairy calves with a summer coat. Testing was carried out comparing the test composition to a comparator product and to single-active comparator product.

The animals were slaughtered at day 13/14 after treatment and faecal egg count (FEC) and worm count measured.

The study protocol is shown below in Table 24.

**Table 24. Study protocol**

| Activity | Trial Day | Dates performed/Details |
|---|---|---|
| Clean out drench 2 | -46 | Oxfendazole +Levamisole (Scanda) oral 10 ml/calf+ Metacam 2 ml Subcut Inj/calf |
| Clean out drench 3 + lice treatment | -45 | Ivermectin (Ivomec liquid) oral (22 ml/calf).<br>Bendicarb (Niltime) pour on for lice (10 ml/calf). |
| Artificially oral infection | -39, -36, - 29, -20, - 12 | Orally dose calves with infective larvae via plastic syringe on D -39, -36, -29, -20 and D-12. See detail larval dosing/artificial infection. |

(continued)

| Activity | Trial Day | Dates performed/Details |
|---|---|---|
| Faecal sampling | -6, -2, 6, 10, 13 | Day -6, Day -2, then post treatment at Days 6, 10, 13. FEC at all points, lungworm larvae at Day 10. Quantative larval culture Day 13 |
| Weighing | -1 | Weigh, Day -1 |
| Treatment | 0 | Day 0. T=0 Controls (Group 1), Test composition treated (Group 2 & 3) |
| Simulated rain | 0 | 2 hours post treatment (Group 2 only). 10 mm in approximately 30 min. |
| Skin observations | -1, 0, 1, 4, 12 | Pre-allocation Day, 4 hours, 24 hours, |
| Clinical Observations | 0, 1, 12 | Pre-treatment, 1 hours, 4 hours, 24 hours and 12 days |
| Clinical measurements | 0, 1 | Pre-treatment (0), 4 hours and 24 hours post treatment |
| Weather and general observations | -6 to 13 | Activities log then Daily log Day-6 until Slaughter (Day 13) including weather from treatment day |
| Slaughter | 13 | Recover lungs for lungworm, collect and ligate abomasum, small intestine and large intestine for worm count. Collect faeces for egg count/ larval culture. Collect hides and also fixed skin sections (4 animals). |

To supplement natural infection the number of larvae orally dosed was increased as shown in Table 25.

**Table 25. Total number of infective larvae dosed per calf by worm genera**

| Dose day | Total | Genera *Haemonchus* | *Ostertagia* | *Trichostrongylus* | *Cooperia* | *Oesph/Chab* |
|---|---|---|---|---|---|---|
| Dose 1 (D -39) | 1600 | 32 | 208 | 48 | 1232 | 80 |
| Dose 2 (D -36) | 2500 | 41 | 424 | 75 | 1844 | 116 |
| Dose 3 (D -29) | 4333 | 0 | 390 | 0 | 3943 | 0 |
| Dose 4 (D -20) | 2167 | 0 | 195 | 0 | 1972 | 0 |
| Dose 5 (D - 12) | 3000 | 0 | 750 | 80 | 1530 | 660 |
| Total (1-5) | 13600 | 73 | 1967 | 183 | 10521 | 856 |

**9.3 Statistics**

The primary data in the study were the individual worm counts. The worm count data was tabulated and statistically analysed including tests for normal distribution (Bartlett's test of equal variance) and tests for significance between the means of treated and control groups compared using One Way Analysis of Variance (ANOVA). Efficacy of treatment on worm count and egg counts (for each sampling) was calculated according to the following equation using both arithmetic and geometric means:

$$\%\ \text{Reduction (Efficacy\%)} = \frac{GroupMean(untreated) - GroupMean(treated)}{GroupMean(untreated)} \times 100$$

Secondary data including the observations was tabulated including totals and means to determine if there were any treatment effects.

### 9.4 Results

#### 9.4.1 Faecal egg counts

The faecal egg counts showed that the untreated control animals (n=6) were uniformly positive (150-500 epg) over the trial period. At allocation (Day -2) they had a mean of 317 epg AM (296 epg GM) with the mean egg counts remaining at 300 epg AM (284-293epg) GM at Days 6, 10 and 13. The mean of Groups 2 (n=6) and 3 (n=6), had similar means at the time of allocation at 325 epg (AM) or 304epg (GM) for Group 2 and 308 epg (AM) or 291 (GM) for Group 3. After treatment both test composition treated groups had uniformly negative egg counts at 6, 10 and 13 days after treatment. There was no significant difference in group mean faecal egg counts at the time of allocation, but the differences between control and treated groups at the post-treatment counts was highly significant (>0.0001). There was no difference in FEC between Group 2 calves and showered with simulated rain 2 hours post-treatment, or the Group 3 calves that were treated and remained dry with no rain for over 24 hours, see Table 26.

**Table 26. Mean faecal egg count (eggs per gram) and significance**

| | FEC (Day-2) | FEC (Day 6) | FEC (Day 10) | FEC (Day 13) |
|---|---|---|---|---|
| Group 1 - Untreated Controls | | | | |
| 3 | 150 | 200 | 200 | 200 |
| 5 | 400 | 250 | 500 | 350 |
| 9 | 300 | 200 | 350 | 250 |
| 11 | 500 | 350 | 250 | 400 |
| 13 | 400 | 400 | 300 | 300 |
| 15 | 400 | 400 | 200 | 300 |
| AM | 316.7 | 300.0 | 300.0 | 300 |
| GM | 296.3 | 287.1 | 284.1 | 292.8 |
| Group 2 - Test composition with simulated rain (10 mm) at 2 hours | | | | |
| AM | 325.0 | 0 | 0 | 0 |
| GM | 303.9 | 0 | 0 | 0 |
| % Reduction | | 100 | 100 | 100 |
| P-value | | >0.0001 | >0.0001 | >0.0001 |
| Group 3 - Test composition with no rain for 24 hours | | | | |
| AM | 308.3 | 0 | 0 | 0 |
| GM | 291.1 | 0 | 0 | 0 |
| % Reduction | | 100 | 100 | 100 |
| P-value | | >0.0001 | >0.0001 | >0.0001 |

AM= Arithmetic mean GM = Geometric mean

#### 9.4.2 Larval cultures

Larval cultures (see Table 27) confirmed that at the time of slaughter a mixed worm infection including large intestinal worms were present. Quantitative larval culture analysis confirmed that no larvae could be detected in either test composition treated group. This result indicates it is a highly effective anthelmintic and also that the levamisole component of the test composition was effective as no surviving *Cooperia* larvae (resistant) were detected.

**Table 27. Larval cultures pre-treatment (-6) and 13 days after treatment (quantitative culture)**

| Group Trial Day | Pre-Allocation -6 | Group 1 13 | Group 2 13 | Group 3 13 |
|---|---|---|---|---|
| *Haemonchus* (%) | 0 | 0 | 0 | 0 |
| *Ostertagia* (%) | 17 | 13 | 0 | 0 |
| *Trichostrongylus* (%) | 0 | 0 | 0 | 0 |
| *Cooperia* (%) | 83 | 72 | 0 | 0 |
| *Oesophagostomum/Chabertia* | 0 | 15 | 0 | 0 |
| Total larvae | NQ | 2400 | 0 | 0 |
| Gram of faeces cultured | NA | 50 g | 50 g | 50 g |

(continued)

| Group Trial Day | Pre-Allocation -6 | Group 1 13 | Group 2 13 | Group 3 13 |
|---|---|---|---|---|
| Larvae per gram | NQ | 48 | 0 | 0 |
| NQ = Not quantified, NA = Not applicable | | | | |

### 9.4.3 Worm counts

***Abomasum***

Worm counts confirmed that the control animals were uniformly infected with adult *Ostertagia* (1483 AM, 1335 GM) and smaller numbers of L4 stages (100AM, 49GM) in the abomasum with only occasional *Trichostrongylus axei* and *Haemonchus contortus* present. The test composition treatments gave complete (100%) reductions, with no worms found. These reductions were highly significant for *Ostertagia* (adult and L4 stages), but there were insufficient *T. axei* or *Haemonchus* in the controls to allow assessment and statistical analysis. The *Ostertagia* present were confirmed as *Ostertagia ostertagi* (94.7%)and *Ostertagia lyrata* (5.3%), with a total of 57 male worms available for speciation from the controls.

**Table 28. Abomasal worm counts**

| | Ostertagia spp. | | | Trichostrongylus axei | | Haemonchus contortus | |
|---|---|---|---|---|---|---|---|
| | 5th Stage | L4 | E4 | 5th Stage | L4/E4 | 5th Stage | L4/E4 |
| Group 1 - Untreated Controls | | | | | | | |
| 3 | 1000 | 0 | 0 | 0 | 0 | 50 | 0 |
| 5 | 2500 | 150 | 0 | 150 | 0 | 0 | 0 |
| 9 | 700 | 100 | 0 | 0 | 0 | 0 | 0 |
| 11 | 1150 | 50 | 0 | 0 | 0 | 0 | 0 |
| 13 | 2300 | 200 | 0 | 0 | 0 | 0 | 0 |
| 15 | 1200 | 100 | 0 | 100 | 0 | 0 | 0 |
| AM | 1483.3 | 100.0 | 0 | 41.7 | 0 | 8.3 | 0 |
| GM | 1335.3 | 49.1 | 0 | 4.0 | 0 | 0.9 | 0 |
| Group 2 - Test composition with simulated rain (10 mm) at 2 hours | | | | | | | |
| AM | 0 | 0 | 0 | 0 | 0 | 0 | 0 |
| GM | 0 | 0 | 0 | 0 | 0 | 0 | 0 |
| % Red | 100 | 100 | NA | 100 | NA | 100 | NA |
| P-value | <0.0001 | 0.0003 | NA | 0.07297 | NA | 0.2313 | NA |
| Group 3 - Test composition with no rain for 24 hours. | | | | | | | |
| AM | 0 | 0 | 0 | 0 | 0 | 0 | 0 |
| GM | 0 | 0 | 0 | 0 | 0 | 0 | 0 |
| % Red | 100 | 100 | NA | 100 | NA | 100 | NA |
| P-value | <0.0001 | 0.0003 | NA | 0.07297 | NA | 0.2313 | NA |

P-value for Groups 2 & 3 combined, to give greater power as the two groups are indistinguishable with the same means and distribution of values. 2% aliquot over a 38μm sieve. AM = Arithmetic mean, GM = Geometric mean, NA = Not applicable, % Red= % Reduction.

***Small intestine***

Small intestinal worm counts confirmed that the control animals were uniformly infected with moderate *Cooperia* burdens, mainly adults (10,142 AM, 5912 GM) and smaller numbers of L4 (258AM, 107GM) and E4 stages (50 AM, 16 GM) with only occasional *Trichostrongylus spp* and *Nematodirus spp* present. The test composition treatment gave complete (100%) reductions, with no worms found (Group 2 and Group 3). These reductions were highly significant for *Cooperia* (adult, L4, and E4 stages) but there were insufficient *Trichostrongylus* or *Nematodirus* in the controls to allow assessment and statistical analysis. The *Cooperia* present were confirmed as *Cooperia oncophora* (99.6%) with very small numbers of *Cooperia punctata* (0.4%), with a total of 252 male worms available for identification from the controls.

Only two male *Nematodirus* were found in one animal (Tag #13) both identified as *N. helvetianus,* and no *male Trichostrongylus spp* were found so species identification could not be performed.

**Table 29. Small intestine worm counts**

| | Cooperia spp | | | Trichostrongylus spp | | Nematodirus spp | |
|---|---|---|---|---|---|---|---|
| | 5th Stage | L4 | E4 | 5th Stage | L4/E4 | 5th Stage | L4/E4 |
| Group 1 - Untreated Controls | | | | | | | |
| 3 | 13100 | 100 | 50 | 50 | 0 | 0 | 0 |
| 5 | 10200 | 400 | 50 | 50 | 0 | 0 | 0 |
| 9 | 200 | 0 | 0 | 0 | 0 | 0 | 0 |
| 11 | 7950 | 450 | 0 | 0 | 0 | 0 | 0 |
| 13 | 18700 | 250 | 50 | 0 | 0 | 150 | 0 |
| 15 | 10700 | 350 | 150 | 0 | 0 | 0 | 0 |
| AM | 10141.7 | 258.3 | 50.0 | 16.7 | 0 | 25.0 | 0 |
| GM | 5912.0 | 107.3 | 15.5 | 2.7 | 0 | 1.3 | 0 |
| Group 2 - Test composition with simulated rain (10mm) at 2 hours | | | | | | | |
| AM | 0 | 0 | 0 | 0 | 0 | 0 | 0 |
| GM | 0 | 0 | 0 | 0 | 0 | 0 | 0 |
| % Reduction | 100 | 100 | 100 | 100 | NA | 100 | NA |
| P-value | <0.0001 | 0.00029 | 0.00354 | 0.07266 | NA | 0.2313 | NA |
| Group 3 - Test composition with no rain for 24 hours. | | | | | | | |
| AM | 0 | 0 | 0 | 0 | 0 | 0 | 0 |
| GM | 0 | 0 | 0 | 0 | 0 | 0 | 0 |
| % Reduction | 100 | 100 | 100 | 100 | NA | 100 | NA |
| P-value | <0.0001 | 0.00029 | 0.00354 | 0.07266 | NA | 0.2313 | NA |

P-value for Groups 2 & 3 combined to give greater power, as the two groups are indistinguishable with the same means and distribution of values. 2% aliquot over 38μm sieve. Animal #9 was recounted using another 2% aliquot to confirm count. Retest (400 5th Stage *Cooperia spp,* only). AM = Arithmetic mean, GM = Geometric mean, NA = Not applicable.

***Large intestine***

Large intestinal worm counts confirmed that the control animals were uniformly infected with small numbers of *Trichuris* adults (15 AM, 13.6 GM) and greater numbers of *Oesophastomum* adults (42AM, 39.5GM). The test composition treatment gave complete (100%) reductions, with no worms found (Group 2, and Group 3). These reductions were highly significant for both *Trichuris* and *Oesophagostomum.* Differentiation for *Trichuris* is not performed routinely and is referred to as *Trichuris* species. *Oesophagostomum* in cattle is assumed to be *Oesophagostomum radiatum.*

**Table 30. Large intestinal worm counts**

| | Oesophagostomum 5th Stage | Trichuris 5th Stage |
|---|---|---|
| Group 1 - Untreated Controls | | |
| 3 | 30 | 10 |
| 5 | 50 | 10 |
| 9 | 30 | 30 |
| 11 | 40 | 10 |
| 13 | 30 | 20 |
| 15 | 70 | 10 |
| AM | 41.7 | 15.0 |
| GM | 39.5 | 13.6 |
| Group 2 - Test composition with simulated rain (10mm) at 2 hours | | |
| AM | 0 | 0 |
| GM | 0 | 0 |
| % Red | 100 | 100 |
| P-value | <0.0001 | <0.0001 |
| Group 3 - Test composition with no rain for 24 hours | | |
| AM | 0 | 0 |
| GM | 0 | 0 |
| % Red | 100 | 100 |
| P-value | <0.0001 | <0.0001 |

P-value for Groups 2 & 3 combined to give greater power, as the two groups are indistinguishable with the same means and distribution of values. 10% aliquot over 150 mesh sieve. AM = Arithmetic mean, GM = Geometric mean, NA = Not applicable, % Red= % Reduction.

***Lung worm***

Lung worm counts of three controls animals and also pooled faeces of the control animals cultured for *Dictyocaulus* demonstrated no evidence of lungworm infection in the control animals so further processing of lungs for lungworm was abandoned.

**Table 31. *Dictyocaulus spp.* counts on three control animals**

| | 5th Stage Adult | JuvenileL5 (<2omm) | InhibitedL5 (<1.5mm) |
|---|---|---|---|
| 3 | 0 | 0 | 0 |
| 5 | 0 | 0 | 0 |
| 11 | 0 | 0 | 0 |
| AM | 0 | 0 | 0 |
| GM | 0 | 0 | 0 |

**9.4.4 Skin observations**

Skin observations at the pour-on site were graded prior to pour-on application, at 4 hours, 24 hours, 4 days and 12 days after treatment (just prior to slaughter). Prior to pour-on application the treated calves had no lesions with back scores of zero for hair loss, scurf, redness or inflammation, and there were no findings at 4 or 24 hours post treatment. At 3 days post-treatment some mild reaction was noted in treated calves and the skin was assessed at both Day 4 and prior to slaughter. The results are summaries in **Table 32.**

**Table 32. Skin observations at pour-on site (number and score of treatment animals)**

| Time after treatment | Hairloss 0 | 1 | 2 | 3 | Scurf 0 | 1 | 2 | 3 | Inflammation 0 | 1 | 2 | 3 |
|---|---|---|---|---|---|---|---|---|---|---|---|---|
| Group 1: Untreated control | | | | | | | | | | | | |
| -1 day | 6 | 0 | 0 | 0 | 6 | 0 | 0 | 0 | 6 | 0 | 0 | 0 |
| 4 hours | 6 | 0 | 0 | 0 | 6 | 0 | 0 | 0 | 6 | 0 | 0 | 0 |
| 24 hours | 6 | 0 | 0 | 0 | 6 | 0 | 0 | 0 | 6 | 0 | 0 | 0 |
| 4 days | 6 | 0 | 0 | 0 | 6 | 0 | 0 | 0 | 6 | 0 | 0 | 0 |
| 12 days | 6 | 0 | 0 | 0 | 6 | 0 | 0 | 0 | 6 | 0 | 0 | 0 |
| Group 2: Test composition with simulated rain at 2 hours | | | | | | | | | | | | |
| -1 day | 6 | 0 | 0 | 0 | 6 | 0 | 0 | 0 | 6 | 0 | 0 | 0 |
| 4 hours | 6 | 0 | 0 | 0 | 6 | 0 | 0 | 0 | 6 | 0 | 0 | 0 |
| 24 hours | 6 | 0 | 0 | 0 | 6 | 0 | 0 | 0 | 6 | 0 | 0 | 0 |
| 4 days | 6 | 0 | 0 | 0 | 2 | 4 | 0 | 0 | 0 | 2 | 4 | 0 |
| 12 days | 6 | 0 | 0 | 0 | 5 | 1 | 0 | 0 | 5 | 1 | 0 | 0 |
| Group 3: Test composition with no rain for 24 hours | | | | | | | | | | | | |
| -1 day | 6 | 0 | 0 | 0 | 6 | 0 | 0 | 0 | 6 | 0 | 0 | 0 |
| 4 hours | 6 | 0 | 0 | 0 | 6 | 0 | 0 | 0 | 6 | 0 | 0 | 0 |
| 24 hours | 6 | 0 | 0 | 0 | 6 | 0 | 0 | 0 | 6 | 0 | 0 | 0 |
| 4 days | 6 | 0 | 0 | 0 | 3 | 2 | 1 | 0 | 1 | 0 | 5 | 0 |
| 12 days | 6 | 0 | 0 | 0 | 0 | 4 | 2 | 0 | 5 | 1 | 0 | 0 |

(0=no lesions, 1 =mild, 2- moderate, 3 = severe, 6 animals/group)

At Day 4 some mild-moderate exudate (mean score 1.67) was noted in 11/12 calves treated with the Test composition pour-on without noted skin reddening, with some early developing scurf and some crusts (mean score 1.67) forming. The exudate often had small white lines/fissures, which appeared to be early lifting of the outer layer of the epidermis and formation of scurf/scale (exfoliation). This reaction was not uniform. It was focused particularly in the first 10 cm near the withers and an area some 20-30 cm from the withers (mid-back) suggesting some types of skin are more reactive. The back midline of the calves was approximately 90-100cm long and other quite large areas were unaffected despite the pour-on being applied evenly along the entire length. At Day 12 after application the exudate had largely resolved (score 0.17) with lifting epidermal flakes/scurf (score 1 to 1.33) growing out in the hair with the underlying skin intact. These epidermal flakes varied from very small 1-2 mm up to flakes 2x3 cm in the hair coat. There was no hair loss, and the changes were considered mild and within the range observed for some other similar registered pour-ons and could be easily missed without close observation. The underlying skin also appeared intact, with no reddening and there was rapid measured resolution even at 12 days without any treatment and no undue animal distress or changes in behaviour. It was considered that these skin changes may be in part related to one of the active components rather than simply the solvent system, as almost identical lesions with the same distribution pattern were observed with another registered levamisole base pour on formulation - see Eclipse pour on skin findings in Efficacy Study AR-CSR-0004.

Excluding the four hides sampled for histology the remaining hides were recovered at slaughter on Day 13, identified to treatment group by punching 1, 2 or 3 holes in the hide in the area of the tail head and delivered to Warren Bell, Acting Plant Manager, Graeme Lowe Corporation, Fellmonger/Tannery, Onehunga, Auckland. The skins were processed to the wet blue stage of tanning and graded by the plant manager for quality as 1st, 2nd or 3rd grade hides, with the results shown in Table 33. Light discolouration (darkening) corresponding to the pour-on site as occurred with the test composition treatments is often seen on "wet-blue" hides and was not graded as a fault.

**Table 33. Hide grade assessment by treatment group**

| | 1st grade | 2nd grade | 3rd grade | Total hides |
|---|---|---|---|---|
| Controls | 5 | 0 | 0 | 5 |
| Group 2 | 4 | 1 | 0 | 5 |
| Group 3 | 2 plus 1 graded as 1-2 1 | | 0 | 4 |

Key: 1st grade hide does not have any serious defect, 2nd grade hide has moderate sized or moderately severe defect(s), 3rd grade hide has a severe defect(s). The defects observed comprised a roughened surface.

As shown above eleven of the 14 hides were graded as 1st, with all Controls assessed as 1st Grade (5 of 5) while 4 of the 5 test composition treated hides were 1st Grade in Group 2 and 2 of 4 in Group 3. Those graded as 2nd

Grade included 1 of 5 in Group 2 and 1 of 4 in Group 3. One hide in Group 3 was graded mid way as a 1st-2nd grade.

Causes for the 2nd grades included: Two small areas of roughened hide, 5x5 cm, one at withers and one in the lumbar region on one of the five Group 2 hides and two similar areas of roughened hide in the withers and lumbar region on one of the four Group 3 hides. A second Group 3 hide was classified as borderline grade 1st-2nd due to a single 4x5 cm area of slightly roughened hide. Two or three of 9 hides recording a 2nd grade were regarded by the plant manager as a typical grading result for a line of hides.

Hides from the calves were recovered and processed 13 days post treatment, 22 days earlier than would occur with a withhold period of 35 days. The observation that a majority of hides in the test composition treated groups were 1st Grade, the lesions were Grade 2 and that the skin observations indicated a rapid resolution of the epidermal flakes/scurf following application is expected to further reduce any affects of treatment on the quality of tanned hides when harvested at the normal withhold time. No lesions, epidermal flakes or scurf were reported when the test composition was administered in older 300 kg animals in the Residue Study ARAB2679 at Armidale Australia, and detailed observations from the Safety Study AR-CSR-0003 noted the following "There was mild superficial non painful scurf formation along midline areas of the back, particularly the withers in all treated animals 6 days after treatment, but without reddening, oedema or ulceration or hair loss. There was a reduction and early resolution in this scurf reaction by 14 days after treatment and the scurf had virtually completely resolved in all animals by Day 35 without treatment".

### 9.5 Conclusions

The Test composition Pour-on (abamectin and levamisole base) when applied along the midline of the back in beef calves with winter coats, at a dose rate of 1 ml per 20 kg, gave complete, and highly significant ($p<0.0001$) reductions in egg count and worm count relative to untreated controls. Efficacy was not affected by 10 ml of simulated rain applied 2 hours after application.

The reductions of roundworm numbers were significant ($p<0.05$) or highly significant ($p<0.01$) for the parasites present in the various organs including: *Ostertagia spp* (adult and L4 stage) in the abomasum, *Cooperia spp* (adult, L4 and E4 stage) in the small intestine and *Oesophagostomum radiatum* and *Trichuris spp* in the large intestine.

Speciation of the worm types showed predominantly *Ostertagia ostertagi* with small numbers of *Ostertagia lyrata,* and mainly *Cooperia oncophora* with small numbers of *Cooperia puntata.*

The reductions in worm numbers were in excess of >98% AM or GM, and are consistent with a highly effective anthelmintic as defined in both ACVM and VICH guidelines.

The Test composition was well tolerated. Some initial reaction and awareness to the application of the pour on including licking or kicking at the back was seen in the first 10 minutes but this passed rapidly, and by 30 minutes all calves were grazing normally. Mild scurf and some exfoliation of the superficial epidermis was seen at Day 3-4 post treatment, more particularly at the withers or sometimes mid back, but this was resolving and the underlying skin was intact at the time of slaughter (Day 13 post treatment). The skin reaction was considered mild and within the range of skin reactions observe with other similar pour ons. All behavioural observations and clinical measurements showed no difference to untreated controls.

Two of nine Test composition treated hides were found with moderate defects following slaughter at Day 13 and processing to the wet blue stage and one hide had a lesser fault, an acceptable and typical industry result which is expected to improve further when hides are harvested at or after the proposed withhold time of 35 days.

## 10. Efficacy study - Summer coat

### 10.1 Treatment groups

The study comprised six infected beef and dairy calves per treatment group (group 2a contained two animals).

Treatment Groups consisted of Group 1 that remained as untreated controls, Group 2 were animals treated with the test composition applied to a dry coat then showered with 10 mL simulated rain 2 hours after treatment, then protected from any rain for at least 24 hours and Group 3 were animals treated with the test composition applied to a dry coat and then protected from any rain for at least 24 hours.

**Table 34. Treatment groups**

| Group Number | Treatment | Animal Number |
|---|---|---|
| 1 | Untreated Control | 6 |
| 2 | Test composition.<br>Applied midline, withers to tail head | 6 |

(continued)

| Group Number | Treatment | Animal Number |
|---|---|---|
| 2b | Test composition. Applied midline, mid-back to tail head | 2 |
| 3 | RP1 (Bomectin Gold pour-on) as per label | 6 |
| 4 | RP2 (Eclipse pour-on) as per label | 6 |
| | TOTAL | 26 |

Each animal was treated at a rate of 1 ml/20 kg body weight, which equates to 500 μg macrocyclic lactone and 10 mg levamisole per kg.

### 10.2 Study design

The study protocol is shown below in **Table 35.**

**Table 35. Study protocol**

| Activity | Trial Day | Dates performed/Details |
|---|---|---|
| Clean out drench 1 | -68 | Levamisole+Oxfendazole oral (Scanda), 10 ml/calf |
| Artificial oral infection 1 | -64 | Orally dose 27 calves with larvae (10 ml/calf) |
| Artificial oral infection 2 | -56 | Orally dose 27 calves with larvae (20 ml/calf) |
| Treat skin with iodine (ringworm) | -54 | Wash with Vetadine (iodine) to assist in control of ringworm. |
| Artificial oral infection 3 | -29 | Orally dose 27 calves with larvae (10 ml/calf) |
| Faecal sampling | -5, 6,9, 12 | Day -5, then post treatment at Days 6, 9, 12, larval culture at Day -5 and 12, and lungworm larvae at Day 6 in controls. |
| Weighing | -4 | Weigh for calculation of treatment dose. |
| Treatment | 0 | Day 0. T=0 Controls (Group 1), Test composition pour-on (Groups 2 & 2b, Abamectin+Levamisole), Group 3-Bomectin Gold pour-on (Abamectin), Group 4-Eclipse pour-on (Abamectin+Levamisole). Protect all pour-on groups from rain for 24 hours. |
| Skin observations | 0, 1, 6, 11 | Pre-treatment, 4 hr, 24hr, 6 and 11 days in all groups |
| Clinical Observations | 0, 1, 11 | Pre-treatment, 1hr, 4hr, 24 hr and 11 days in all groups |
| Clinical measurements | 0 | Pre-treatment (0), 4hrs in Groups 1 & 2, 2b |
| Weather and general observations | -6 to 12 | Activities log then Daily log Day -6 until Slaughter (Day 12) including weather from treatment day |
| Slaughter | 12 | Collect and ligate abomasum, small intestine and large intestine for worm count. Collect faeces for egg counts. |

To ensure suitable infection of trial animals both natural and artificial infection (oral dosing) were used.

An estimated total of 15,912 larvae of 5 genus were dosed per calf as per Table 36. Details of larval dosing

are summarised below.

- Dose 1. Twenty-seven trial calves individually orally dosed with 10 ml of larval culture (Approximately 3110 larvae/calf).
- Dose 2. Twenty-seven trial calves individually orally dosed with 20 mL of larval culture (Approximately 7302 larvae/calf).
- Dose 3. Twenty-seven trial calves individually orally dosed with 11 ml of larval culture (Approximately 5500 larvae/calf.

**Table 36. Total number of infective larvae dosed per calf by worm genera**

| | **Total** | **Genus** | | | | |
|---|---|---|---|---|---|---|
| | | ***Haemonchus*** | ***Ostertagia*** | ***Tri chostrongylus*** | ***Cooperia*** | ***Oesph/Chab*** |
| Dose 1 | 3110 | 100 | 640 | 70 | 1830 | 470 |
| Dose 2 | 7301 | 292 | 1314 | 0 | 5111 | 584 |
| Dose 3 | 5500 | 0 | 4345 | 0 | 1155 | 0 |
| Total dose | 15911 | 392 | 6299 | 70 | 8096 | 1054 |

Calves were orally dosed using a plastic syringe with the liquid larval culture administered over the back of the tongue.

Natural roundworm infection was acquired from calves grazing infective pasture.

**10.3 Statistics**

The primary data in the study were the individual worm counts. The worm count data was tabulated and statistically analysed including tests for normal distribution and tests for significance between the means of treated and control groups compared using One Way Analysis of Variance (ANOVA). Efficacy of treatment on worm count and egg counts (for each sampling day) was calculated according to the following equation using both arithmetic and geometric means:

$$\text{\% Reduction (Efficacy \%)} = \frac{GroupMean(untreated) - GroupMean(treated)}{GroupMean(untreated)} \times 100$$

Secondary data including the observations was tabulated including totals and means to determine if there were any treatment effects.

**10.4 Results**

**10.4.1Faecal egg counts**

The faecal egg counts showed that the untreated control animals (n=6) were uniformly positive with eggs counts varying from 150-500 epg over the trial period. The pour-on treatment groups including Groups 2, 2b, 3 and 4 had similar mean egg counts at Day -5 (the counts that were used for allocation to treatment). There was no significant difference in group mean faecal egg counts at the time of allocation, but the differences between control and all treated groups at all times post treatment was highly significant (>0.01). All animals treated with the abamectin+levamisole pour-ons, (Test composition and Eclipse in Groups 2, 2b and 4) gave complete control of egg output. In contrast Group 3 animals treated with the single active abamectin pour-on (Bomectin Gold pour-on) gave incomplete reductions in egg count, with a reduction relative to controls of 97.3% at 6 days post treatment and 93.1% at Day 12 post treatment. This finding was consistent with the larval culture and worm count findings for this group discussed later (Section 15.3), and the selection criteria of farms with a history of ML resistant *Cooperia.*

**Table 37. Mean faecal egg count (eggs per gram) and significance**

| Ear tag | FEC (Day-5) | FEC (Day 6) | FEC (Day 9) | FEC (Day 12) |
|---|---|---|---|---|
| Group 1 - Untreated Controls | | | | |
| 44 | 350 | 300 | 200 | 300 |
| 70 | 250 | 300 | 100 | 200 |
| 74 | 550 | 450 | 150 | 150 |
| 92 | 500 | 250 | 200 | 250 |
| 127 | 300 | 200 | 200 | 200 |
| 128 | 150 | 250 | 250 | 350 |
| AM | 350.0 | 291.7 | 183.3 | 241.7 |
| GM | 321.6 | 283.3 | 176.3 | 232.4 |
| Group 2&2b - Test composition pour on, withers to base of tail | | | | |
| AM | 393.8 | 0 | 0 | 0 |
| GM | 349.3 | 0 | 0 | 0 |
| % Reduction (AM) | | 100 | 100 | 100 |
| P-value | 1.0 | >0.001 | >0.001 | >0.001 |
| Group 3. Abamectin pour on (Bomectin pour-on) | | | | |
| 30 | 500 | 0 | 0 | 0 |
| 39 | 400 | 50 | 0 | 0 |
| 46 | 250 | 0 | 0 | 0 |
| 49 | 300 | 0 | 0 | 0 |
| 58 | 200 | 0 | 0 | 0 |
| 94 | 200 | 0 | 0 | 0 |
| AM | 308.3 | 8.3 | 0 | 16.7 |
| GM | 291.5 | 1.9 | 0 | 3.6 |
| % Reduction (AM) | | 97.3 | 100 | 93.1 |
| P-value | 1.0 | >0.001 | >0.001 | >0.001 |
| Group 4. Abamectin +Levamisole pour on (Eclipse pour-on) | | | | |
| AM | 358.3 | 0 | 0 | 0 |
| GM | 333.5 | 0 | 0 | 0 |
| % Reduction | | 100 | 100 | 100 |
| P-value | 1.0 | >0.001 | >0.001 | >0.001 |

AM= Arithmetic mean GM = Geometric mean

### 10.4.1 Larval cultures

Larval cultures 5 days before treatment confirmed that at the time of treatment a mixed worm infection including large intestinal worms were present. Pooled Quantitative larval culture analysis (40 g/group) conducted on faecal samples collected at the time of slaughter confirmed that a mixed worm population was present with high numbers of larvae detected (13,000 larvae/40 g). Low numbers of larvae (140 larvae/ 40 g) were also found in the cattle treated with Abamectin pour-on alone (Bomectin Gold). The larvae recovered from this group were all *Cooperia spp* (100%). This is consistent with an ML resistant *Cooperia* worm strain as also discussed under worm counts. Abamectin is more potent on gastrointestinal parasites than ivermectin so the apparent level of resistance in this study would be more pronounced with a product containing ivermectin. In contrast, no larvae could be detected in a pooled 40 g sample from either of the Abamectin+Levamisole pour-on groups, including the Test composition treatment (Groups 2&2b) and Eclipse treatment (Group 4). The sensitivity of this test was increased further by separately culturing 40 g of faeces from each of the two calves treated with the Test composition on the lower back only (Group 2b). Again no larvae were recovered from either animal in Group 2b (80 g total). This result is consistent with the Test composition having very high efficacy with effective removal of adult worm stages, and that the levamisole component of the Test composition (as with Eclipse pour-on) is effective in removing ML resistant adult *Cooperia* stages which was confirmed in worm count results discussed in Section 15.3.1.

**Table 38. Larval cultures 12 days post treatment (quantitative culture)**

| Group | Group 1 | Group 2&2b | Group 3 | Group 4 |
|---|---|---|---|---|
| Trial Day | 12 | 12 | 12 | 12 |
| *Haemonchus* (%) | 2 | 0 | 0 | 0 |
| *Ostertagia* (%) | 9 | 0 | 0 | 0 |
| *Trichostrongylus* (%) | 0 | 0 | 0 | 0 |

(continued)

| Group<br>Trial Day | Group 1<br>12 | Group 2&2b<br>12 | Group 3<br>12 | Group 4<br>12 |
|---|---|---|---|---|
| *Cooperia* (%) | 73 | 0 | 100 | 0 |
| *Oesophagostomum*/*Chabertia* | 16 | 0 | 0 | 0 |
| Total larvae | 13000 | 0 | 140 | 0 |
| Gram of faeces cultured | 40 g | 40 g +80g (2a+2b) | 40 g | 40 g |
| Larvae per gram | 325 | 0 | 3.5 | 0 |

**10.4.2Worm counts**

***Abomasum***

Worm counts confirmed that the control animals were uniformly infected with adult *Ostertagia* (3650 AM, 2815 GM) and smaller numbers of L4 stages (158AM, 137GM), adult *Trichostrongylus axei* (292 AM, 278 GM) and almost uniformly infected (5/6) with adult *Haemonchus contortus*. The Test composition treatment gave complete (100%) reductions, with no worms found. These reductions were highly significant for *Ostertagia* (adult and L4 stages), *T. axei* adults and significant for *Haemonchus contortus.* This was also true for those treated with the Test composition on the lower back only (Group 2b), and also for those calves treated with Eclipse pour-on (Group 4). What was notable however was that while the reduction in abomasal worm numbers was still effective with Bomectin Gold pour-on, it did not completely remove *Ostertagia* with an adult stage and L4 stage found in two separate animals, while nothing was detected in any of those treated with an abamectin+levamisole pour-on. Typically ML's are highly effective against *Ostertagia* in cattle, and levamisole frequently less effective.

The *Ostertagia spp* from the controls was speciated using 50 male worms and confirmed as *Ostertagia ostertagi* (98%) and *Ostertagia lyrata* (2.0%). The 2 male worms found in the Group 3 were both identified as *Ostertagia ostertagi.* Abomasal *Trichostongylus spp* and *Haemonchus spp* in cattle are considered monospecific in New Zealand which has been confirmed in other studies, so were not typed.

**Table 39. Abomasal worm counts**

| | Ostertagia spp. | | | Trichostrongylus axei | | Haemonchus contortus | |
|---|---|---|---|---|---|---|---|
| | 5th Stage | L4 | E4 | 5th Stage | L4/E4 | 5th Stage | L4/E4 |
| Group 1 - Untreated Controls | | | | | | | |
| 44 | 5900 | 250 | 0 | 250 | 0 | 150 | 0 |
| 70 | 8250 | 100 | 0 | 350 | 0 | 100 | 0 |
| 74 | 2200 | 250 | 0 | 350 | 0 | 100 | 0 |
| 92 | 1150 | 200 | 0 | 400 | 50 | 0 | 0 |
| 127 | 1300 | 100 | 0 | 150 | 0 | 200 | 0 |
| 128 | 3100 | 50 | 0 | 250 | 0 | 100 | 0 |
| AM | 3650.00 | 158.3 | 0 | 291.7 | 0 | 108.3 | 0 |
| GM | 2815.0 | 136.9 | 0 | 278.8 | 0 | 56.1 | 0 |
| Group 2&2b - Test composition pour-on | | | | | | | |
| AM | 0 | 0 | 0 | 0 | 0 | 0 | 0 |
| GM | 0 | 0 | 0 | 0 | 0 | 0 | 0 |
| % Red AM | 100 | 100 | NA | 100 | NA | 100 | NA |
| P-value | <0.001 | <0.001 | NA | <0.001 | NA | <0.05 | NA |
| Group 3 - Abamectin pour on (Bomectin Gold pour-on) | | | | | | | |
| 30 | 0 | 0 | 0 | 0 | 0 | 0 | 0 |
| 39 | 0 | 0 | 0 | 0 | 0 | 0 | 0 |
| 46 | 100 | 0 | 0 | 0 | 0 | 0 | 0 |
| 49 | 0 | 0 | 0 | 0 | 0 | 0 | 0 |
| 58 | 0 | 0 | 0 | 0 | 0 | 0 | 0 |
| 94 | 0 | 50 | 0 | 0 | 0 | 0 | 0 |
| AM | 16.7 | 8.3 | 0 | 0 | 0 | 0 | 0 |
| GM | 2.2 | 1.9 | 0 | 0 | 0 | 0 | 0 |
| % Red AM | 95.4 | 94.8 | NA | 100 | NA | 100 | NA |
| P-value | <0.001 | <0.001 | NA | <0.001 | NA | <0.05 | NA |
| Group 4 - Abamectin+Levamisole pour-on ( Eclipse pour-on) | | | | | | | |
| AM | 0 | 0 | 0 | 0 | 0 | 0 | 0 |
| GM | 0 | 0 | 0 | 0 | 0 | 0 | 0 |
| % Red | 100 | 100 | NA | 100 | NA | 100 | NA |
| P-value | <0.001 | <0.001 | NA | <0.001 | NA | <0.05 | NA |

P-value for Groups 2 & 2b combined, to give greater power as the two groups are indistinguishable with the same means and distribution of values. 2% aliquot over a 38 μm sieve. AM = Arithmetic mean, GM = Geometric mean, NA = Not applicable, % Red= % reduction.

***Small intestine***

Small intestinal worm counts confirmed that the control animals were uniformly infected with moderate-high *Cooperia* burdens, mainly adults (26092 AM, 21692GM) and moderate numbers of L4 (3408AM, 2596GM) and no E4 stages. There were no *Trichostrongylus* detected in the small intestine but the controls were almost uniformly (5/6) infected with L4 stages of *Nematodirus spp* (200 AM, 83GM) and slightly less uniformly infected with small numbers of adult stages (75 AM, 11 GM). The Test composition treatment (Group 2 and Group 2b) gave complete (100%) reductions of these worms and worm stages, with no difference detected in those receiving the pour on only on the lower back (Group 2b). These reductions were highly significant for *Cooperia* (adult, L4 stages) and *Nematodirus* L4 and significant for *Nematodirus* adults. Interestingly a small number of E4 Cooperia larvae were detected in 4/8 Test composition treated animals (AM 112), but not seen in the controls, indicating very early re-infection post treatment and possibly suggesting less persistent activity than the positive control abamectin pour ons in this study. Eclipse pour-on treated animals in Group 4 gave similar reductions but no E4 larvae were seen. Bomectin Gold pour-on containing abamectin alone did not give full reductions against *Cooperia* stages, with only 89.4% control of adult stages (AM) and 98.8% reductions of L4 stages. While the *Cooperia* reduction for this treatment remained statistically highly significant, the product achieved only moderate efficacy (80-89%), which was lower than its "effective" label claim. Experience with abamectin versus ivermectin via topical treatment in cattle, suggest that if a less potent ML such as ivermectin had been used the efficacy (inefficacy) would have been even lower. Fifty male *Cooperia* present in the controls were speciated and confirmed to consist of *Cooperia oncophora* (76%) and *Cooperia punctata* (24%), while 50 male worms in the Bomectin Gold pour on group (Group 3) were entirely *Cooperia oncophora* (100%). Only four male *Nematodirus* were found in two control animals and all four worms were identified as *N. helvetianus.*

**Table 40. Small intestine worm counts**

| | Cooperia spp | | | Trichostrongylus spp | | Nematodirus spp | |
|---|---|---|---|---|---|---|---|
| | 5th Stage | L4 | E4 | 5th Stage | L4/E4 | 5th Stage | L4 |
| Group 1 - Untreated Controls | | | | | | | |
| 44 | 20650 | 1500 | 0 | 0 | 0 | 50 | 0 |
| 70 | 42100 | 7700 | 0 | 0 | 0 | 0 | 400 |
| 74 | 31700 | 3800 | 0 | 0 | 0 | 150 | 100 |
| 92 | 37250 | 4750 | 0 | 0 | 0 | 250 | 400 |
| 127 | 5150 | 750 | 0 | 0 | 0 | 0 | 100 |
| 128 | 19700 | 1950 | 0 | 0 | 0 | 0 | 200 |
| AM | 26091.7 | 3408.3 | 0 | 0 | 0 | 75 | 200 |
| GM | 21692.2 | 2595.7 | 0 | 0 | 0 | 11.2 | 83.1 |
| Group 2&2b - Test composition pour-on | | | | | | | |
| AM | 0 | 0 | 0 | 0 | 0 | 0 | 0 |
| GM | 0 | 0 | 0 | 0 | 0 | 0 | 0 |
| % Red AM | 100 | 100 | NA | NA | NA | 100 | 100 |
| P-value | <0.001 | <0.001 | NA | NA | NA | <0.05 | <0.001 |
| Group 3 - Abamectin pour-on (Bomectin Gold pour-on) | | | | | | | |
| 30 | 500 | 0 | 0 | 0 | 0 | 0 | 0 |
| 39 | 4450 | 0 | 0 | 0 | 0 | 0 | 0 |
| 46 | 5350 | 200 | 0 | 0 | 0 | 0 | 0 |
| 49 | 3500 | 0 | 0 | 0 | 0 | 0 | 0 |
| 58 | 0 | 0 | 0 | 0 | 0 | 0 | 0 |
| 94 | 2800 | 50 | 0 | 0 | 0 | 0 | 0 |
| AM | 2766.7 | 41.7 | 0 | 0 | 0 | 0 | 0 |
| GM | 699.4 | 4.7 | 0 | 0 | 0 | 0 | 0 |
| % Red AM | 89.4 | 98.8 | NA | NA | NA | 100 | 100 |
| P-value | <0.001 | <0.001 | NA | NA | NA | <0.05 | <0.001 |
| Group 4 - Abamectin+Levamisole pour on ( Eclipse pour on) | | | | | | | |
| AM | 0 | 0 | 0 | 0 | 0 | 0 | 0 |
| GM | 0 | 0 | 0 | 0 | 0 | 0 | 0 |
| % Reduction | 100 | 100 | NA | NA | NA | 100 | 100 |
| P-value | <0.001 | <0.001 | NA | NA | NA | <0.05 | <0.001 |

P-value for Groups 2 & 2b combined to give greater power, as the two groups are indistinguishable with the same means and distribution of values. 2% aliquot over 38 μm sieve. AM = Arithmetic mean, GM = Geometric mean, NA = Not applicable.

***Large intestine***

Large intestinal worm counts confirmed that the control animals were uniformly infected with small numbers of *Oesophastomum* adults (113.3 AM, 105 GM) and variably infected with lower numbers of *Trichuris* adults (6.7AM, 4.9 GM). The Test composition pour on treatment (Groups 2&2b) and both Bomectin Gold pour on (Group 3) and Eclipse pour on (Group 4) gave complete (100%) reductions in both worm genera. These reductions were highly significant for the *Oesophagostomum spp,* but the reduction in *Trichuris* were not significant because of low worm numbers and variable infection in the controls. Differentiation for *Trichuris* is not performed routinely and is referred to as *Trichuris* species. *Oesophagostomum* in cattle is assumed to be *O. radiatum* as it is assumed to be monspecific in New Zealand.

**Table 41. Large intestinal worm counts**

| | Oesophagostomum 5th Stage | Trichuris 5th Stage |
|---|---|---|
| Group 1 - Untreated Controls | | |
| 44 | 100 | 10 |
| 70 | 130 | 10 |
| 74 | 110 | 10 |
| 92 | 210 | 0 |
| 127 | 70 | 0 |
| 128 | 60 | 10 |
| AM | 113.3 | 6.67 |
| GM | 105.0 | 4.9 |
| Group 2 & 2b - Test composition pour on | | |
| AM | 0 | 0 |
| GM | 0 | 0 |
| % Red | 100 | 100 |
| P-value | <0.001 | 0.2 |
| Group 3 - Abamectin pour on (Bomectin Gold pour-on) | | |
| AM | 0 | 0 |
| GM | 0 | 0 |
| % Reduction | 100 | 100 |
| P-value | <0.001 | 0.3 |
| Group 4 - Abamectin+Levamisole pour on (Eclipse pour-on) | | |
| AM | 0 | 0 |
| GM | 0 | 0 |
| % Red | 100 | 100 |
| P-value | <0.001 | 0.3 |

P-value for Groups 2 & 2b combined to give greater power, as the two groups are indistinguishable with the same means and distribution of values. 10% aliquot over 150 mesh sieve. AM = Arithmetic mean, GM = Geometric mean, NA = Not applicable, % Red= % Reduction.

***Lung worm***

Lung worm larval culture from the pooled faecal sample (25-30 g) of the control animals at Day 6 using a modified Baemann technique were negative for lungworm larvae and so no lungs were collected at slaughter for lungworm examination.

**10.5 Conclusions**

The test composition pour-on (abamectin + levamisole base) when applied along the midline of the back in dairy calves with summer coats, at a dose rate of 1 ml per 20 kg, gave complete and highly significant (p<0.001) reductions in egg count and worm count relative to untreated controls. The efficacy and safety of the pour-on did not appear affected by the product being applied only to the midline of the lower back (from mid-back to the base of tail) compared to application along the entire midline of the back, from the withers to the base of the tail

The reductions of roundworm numbers were significant (p<0.05) or highly significant (p<0.001) for the parasites present in the various organs including: *Ostertagia spp* (adult and L4 stage), *Trichostrongylus axei* (adult) and *Haemonchus contortus* (adult) in the abomasum, *Cooperia spp* (adult, L4 stages) and *Nematodirus spp* (adult and L4) in the small intestine and *Oesophagostomum radiatum* in the large intestine.

Speciation of the worm types showed predominantly *Ostertagia ostertagi* with small numbers of *Ostertagia lyrata,* and *Cooperia oncophora* and *Cooperia punctata.* The adult stages of *Nematodirus* were identified as *N. helvetianus.*

The reductions in worm numbers were in excess of >98% AM or GM, and are consistent with a highly effective anthelmintic as defined in both ACVM and VICH guidelines.

The reference product Eclipse Pour-on, which also delivers abamectin and levamisole base at the same dose rate as the investigational product gave similar complete reductions in worm count.

In contrast Bomectin Gold pour-on containing only abamectin was only moderately effective on adult *Cooperia oncophora* (89.4% AM) and did not achieve "effective" control as per its label claim. It appeared to give effective control of *Cooperia punctata.* It did not achieve complete control of L4 stages (98.8% AM) which the combinations achieved. It

is considered that if a less potent ML such as ivermectin had been used then the efficacy against this *Cooperia* strain would have been considerably lower and the level of resistance demonstrated substantial. The findings however are considered entirely consistent with an ML resistant strain of *Cooperia oncophora* and supports the test composition's claim of efficacy against ML resistant *Cooperia* strains. A less common finding with Bomectin Gold in this study was the incomplete control of *Ostertagia ostertagi* (95.4% adult, 94.8% L4, AM). Typically the efficacy of ML pour-ons including ivermectin against *Ostertagia spp* is extremely high (>98%). As the most pathogenic worm genera in cattle this finding is of concern, whether it is the effect of ineffective skin absorption, or evidence of emerging resistance or tolerance by the parasite. It does however demonstrate the benefit of combination anthelmintics in delivering very high efficacy, which reduces the selection for potentially resistant worm strains

The Test composition was well tolerated including at the pour-on site. Some initial reaction and awareness to the application of the pour-on including licking at the application site was seen in occasional animals but this passed rapidly, and by 10 to 15 minutes the test composition treated calves were grazing normally. Mild scurf and some exfoliation of the superficial epidermis was seen at Day 6 post treatment, more particularly at the withers or sometimes mid-back, but this was resolving and the underlying skin was intact, at the time of slaughter (Day 12 post-treatment) without treatment or negative effects on behaviour. The skin reaction was considered mild and within the range of skin reactions observed for the reference product Eclipse pour-on. It is speculated because of the similarly of the skin reactions in these pour-ons that this reaction may be related to the levamisole base which both pour-ons contain. All behavioural observations and clinical measurements relative to the controls showed no differences that could be attributed to the Test composition treatment. Despite Bomectin Gold pour-on being apparently a better tolerated, formulation based on skin observations and measurement, this was not supported by observational data. There appeared to be avoidance behaviour of bright sunlight by Bomectin Gold pour-on treated animals, which possibly negatively impacted grazing behaviour for up to 11 days. This was not observed for either the Test composition or the reference combination pour-on, or the untreated controls.

11. **Summary**

The studies have demonstrated that the test composition is

- highly effective (>99%) against resident parasites in the abomasum, small instestine and large intestine.
- effective on ML-resistant *Cooperia,*

The studies have also demonstrated that the test composition is not affected by

- rain 2 hours after treatment,
- breed, or
- summer/winter coat.

The results also showed that the test composition did not perform significantly different to the orally administered Eclipse product.

The following field observations were made on the studies. The test composition

- has good wetting/spreading properties,
- does not cause hair loss or skin damage,
- leaves no apparent residue/oil on skin,
- causes mild, transient scurf,
- does not cause any apparent photosensitivity,
- has a similar withholding period to equivalent registered products, and
- results in mild hide defects (wet blue stage) 13 days after treatment (cf 35 day WHP).

The field observations also noted that the calves grazed normally 30 minutes after treatment.

Where in the foregoing description reference has been made to elements or integers having known equivalents, then such equivalents are included as if they were individually set forth.

Although the invention has been described by way of example and with reference to particular embodiments, it is to be understood that modifications and/or improvements may be made without departing from the scope or spirit of the invention.

Having thus described in detail preferred embodiments of the present invention, it is to be understood that the invention defined by the above paragraphs is not to be limited to particular details set forth in the above description as many apparent variations thereof are possible without departing from the spirit or scope of the present invention.

The present invention will now be described by way of reference to the following clauses:

1. An anhydrous transdermal composition comprising

- at least one active ingredient having a log P in hexane and water of less than about 8 at pH 7.4,
- a terpene, and
- a non-hydroxyl containing solvent, a non-heterocyclic ester solvent or a combination thereof.

2. An anhydrous transdermal composition comprising

- at least one active ingredient,
- at least about 20% terpene, and
- a non-heterocyclic ester solvent.

3. An anhydrous transdermal composition comprising

- at least one active ingredient,
- a terpene, and
- a tripropylene glycol alkyl ether.

4. A composition of any one of clauses 1 to 3, wherein at least one of the active ingredients is selected from the group consisting of an anthelmintic, a non-steroidal anti-inflammatory, a steroidal anti-inflammatory, a steroid hormone, an antihistamine, an anti-emetic, a metabolic regulator, a productivity regulator, a hypothyroidism treatment, a behavioural treatment, an analgesic, a parasiticide, an insecticide, an anti-biotic, an anti-microbial, an anti-fungal, an anti-viral, a coccidostat, a skin-treatment agent, or any combination of two or more thereof.

5. A composition of any one of clauses 1 to 5, wherein at least one of the active ingredients is an anthelmintic.

6. An anhydrous transdermal composition comprising

- at least one anthelmintic,
- a terpene, and
- a non-heterocyclic ester solvent.

7. A composition of any one of clauses 4 to 6, wherein the anthelmintic is an imidazothiazole.

8. A composition of clause 7, wherein the imidazothiazole is selected from levamisole base, pyrantel pamoate, butamisol or tetramisole.

9. A composition of any one of clauses 4 to 8, wherein the anthelmintic is levamisole base.

10. A composition of any one of clauses 2 to 5 and 7 to 9, wherein at least one of the active ingredients has a log P in hexane and water of less than about 8 at pH 7.4.

11. A composition of any one of clauses 6 to 9, wherein at least one of the anthelmintics has a log P in hexane and water of less than about 8 at pH 7.4.

12. An anhydrous transdermal composition comprising :

- levamisole base,
- a terpene, and
- an anhydrous veterinarily acceptable carrier.

13. A composition of clause 12, wherein the anhydrous veterinarily acceptable carrier is selected from a non-hydroxyl containing solvent, a non-heterocyclic ester solvent or a combination thereof.

14. A composition of any one of clauses 3 to 5 and 7 to 11, comprising a tripropylene glycol alkyl ether and a non-hydroxyl containing solvent, a non-heterocyclic ester solvent or a combination thereof.

15. A composition of any one of clauses 1, 4, 5, 7 to 9, 11, 13, and 14 wherein the non-hydroxyl containing solvent or the non-heterocyclic ester solvent is a fatty acid ester.

16. A composition of any one of clauses 1, 4, 5, 7 to 9, 11, 13, and 14 wherein the non-hydroxyl containing solvent or the non-heterocyclic ester solvent is selected from a triglyceride, glycerol ester or combination thereof.

17. A composition of any one of clauses 2 and 4 to 11 wherein the non-heterocyclic ester solvent is a fatty acid ester.

18. A composition of any one of clause 2 and 4 to 11 wherein the non-heterocyclic ester solvent is selected from a triglyceride, glycerol ester or combination thereof.

19. A composition of clause 15 or 17, comprising a solvent selected from a triglyceride, a glycerol ester or a mixture thereof.

20. A composition of clause 16 or 18, comprising a fatty acid ester.

21. A composition of any one of clauses 15 to 20, comprising a glycol ether.

22. A composition of any one of clauses 1 and 3 to 21, wherein the composition comprises at least about 10% or at least about 20% w/w terpene.

23. A composition of any one of clauses 1 to 22, wherein the terpene is a terpene hydrocarbon, terpene alcohol, terpene ketone, or terpene oxide.

24. A composition of any one of clauses 1 to 23, wherein the terpene is a mono-terpene.

25. A composition of any one of clauses 1 to 24, wherein the terpene is monocyclic or bi-cyclic.

26. A composition of any one of clauses 1 to 25, wherein the terpene is a terpene hydrocarbon.

27. A composition of any one of clauses 1 to 26, wherein the terpene is limonene or phellandrene.

28. A composition of any one of clauses 1 to 27, wherein the terpene is limonene.

29. A composition of any one of clauses 1 to 28, comprising at least one surfactant.

30. A composition of any one of clauses 29, wherein at least one of the sufactants has the following structure:

$$z\text{-}(o\text{-}CR_1R_2CR_3R_4]_n\text{-}OH$$

where

z is an optionally substituted $Ci_4$ to C22 linear alkenyl,
Ri, R2, R3 and $R_4$ are each independently selected from methyl or hydrogen, and n is an integer from 1 to 10.

31. A composition of clause 30 wherein at least two of Ri, $R_2$, R3 and $R_4$ are hydrogen.

32. A composition of clause 30 or 31 wherein Ri, $R_2$, R3 and $R_4$ are all hydrogen.

33. A composition of any one of clauses 30 to 32 wherein n is an integer from 1 to 5.

34. A composition of any one of clauses 30 to 33 wherein at least one of the carbon-carbon double bonds in Z has a cis configuration.

35. A composition of any one of clauses 30 to 34 wherein Z is $Ci_6$ to C22 linear alkenyl.

36. A composition of any one of clauses 30 to 35 wherein the surfactant is a polyoxyethylene (2) oleyl ether.

37. A composition of any one of clauses 29 to 36, wherein the at least one surfactant provides a hydrophilic-lipophilic balance of about 4.0 to about 6.0.

38. A composition of any one of clauses 29 to 37 wherein the composition is stable at 4°C.

39. A composition of clause 38 wherein the composition is stable at 4°C for at least 72 hrs.

40. A composition of any one of clauses 1 to 39 wherein the composition comprises at least two active ingredients.

41. A composition of clause 40, wherein at least one of the active ingredients has a log P in hexane and water at pH 7.4 of at least about 4, at least about 5, or at least about 6.

42. A composition of any one of clauses 1 to 41, comprising a macrocyclic lactone.

43. A composition of clause 42 wherein the macrocyclic lactone is selected from avermectin, ivermectin, abamectin, eprinomectin, moxidectin, selamectin, doramectim, milbemycin, abamectin or cydectin .

44. A composition of clause 42 or 43, wherein the macrocyclic lactone is abamectin or moxidectin .

45. A composition of any one of clauses 1 to 44 comprising

- optionally about 1 to about 60% w/w levamisole base,
- optionally about 0.1 to about 20% w/w macrocyclic lactone,
- optionally about 1 to about 40% w/w fatty acid ester,
- optionally about 1 to about 60% w/w terpene, and
- optionally about 1 to about 25% w/w non-aqueous solvent.

46. A composition of any one of clauses 15, 17, and 20 to 45 wherei n the fatty acid ester has a $C_8$-$C_2$o alkyl chain .

47. A composition of any one of clauses 15, 17, and 20 to 46 wherei n the fatty acid ester has a $Ci_0$-$Ci_6$ alkyl chain .
48. A composition of any one of clauses 15, 17, and 20 to 47 wherei n the fatty acid ester is isopropyl myristate.
49. A composition of any one of clauses 1 to 48 wherein the composition comprises an antioxidant.
50. A composition of any one of clauses 9 to 49 wherein the composition delivers levamisol base transdermally at an average flux rate of at least 300pg/$cm^2$/n.
51. A composition of any one of clauses 9 to 50, wherein the composition is administered in an amount less than about 0.1 mL/kg of live animal; and wherein the composition delivers levamisole base within its therapeutically effective does range to the target animal.
52. A method of manufacturing a composition comprising

i) mixing a first composition comprising an active ingredient that is substantially insoluble in water, and a terpene, with a fatty acid ester, or
ii) mixing a first composition comprising a terpene, with a second composition comprising an active ingredient that is substantially insoluble in water and a fatty acid ester, or
iii) mixing a first composition comprising a first active ingredient that is substantially insoluble in water, and a terpene, with a second composition comprising a second active ingredient that is substantially insoluble in water, and a fatty acid ester,

thereby providing the transdermal composition.
53. A method of clause 52 wherein the first composition is formed from a mix of at least one active ingredient that is substantially insoluble in water, a terpene and a nonaqueous solvent.
54. A method of clause 52 or 53 wherein the dissolved mixture is formed from a mix of the first composition and any one or more of

- an antioxidant,
- a non-aqueous solvent,
- a fatty acid ester, or
- a mixture of any one or more of (i) to (iii).

55. A method of any one of clauses 52 to 54 wherein the non-aqueous solvent is a glycol ether.
56. A method of any one of clauses 55 wherein the glycol ether is a tripropylene glycol alkyl ether.
57. A method of clause 56 wherein the tripropylene glycol alkyl ether is selected from tripropylene glycol methyl ether, tripropylene glycol mono-n-propyl ether or tripropylene glycol mono-n-butyl ether.
58. A method of any one or more of clauses 52 to 57 wherein the heating is performed for between 30 min to 8 hrs.
59. A method of any one or more of clauses 52 to 58 wherein the heated mixture is cooled.
60. A method of clause 59 wherein the cooled mixture is packaged.
61. Use of a composition of any one of clauses 1 to 51 for treating an animal in need thereof.
62. A kit comprising a composition of any one of clauses 1 to 51 and instructions for use.
63. The kit of clause 62, comprising a second composition comprising at least one active ingredient, wherein at least one of the active ingredients in the second composition is incompatible with at least one of the active ingredients in the composition of any one of clauses 1 to 51.
64. The kit of clause 62 or 63, wherein the instructions comprise mixing the composition of any one of clauses 1 to 51 and the second composition, and immediately administering the mixture to an animal in need thereof.

## Claims

**1.** An anhydrous transdermal composition comprising

at least one active ingredient having a log P in hexane and water of less than about 8 at pH 7.4,
a terpene, where the terpene is limonene, and
a non-aqueous solvent system comprising a tripropylene glycol alkyl ether and a non-hydroxyl containing solvent, a non-heterocyclic ester solvent or a combination thereof;
wherein the composition is in the form of a solution.

**2.** A composition of claim 1 comprising 15% to 60% by weight of terpene.

**3.** A composition of claims 1 or 2 wherein the non-heterocyclic ester solvent is a fatty acid ester.

4. A composition of claim 3 comprising a) a fatty acid ester and a glycol ether, the glycol ether being the tripropylene glycol alkyl ether; or b) a glycol ether, the glycol ether being the tripropylene glycol alkyl ether, a fatty acid ester and a solvent selected from a triglyceride, glycerol ester or a combination thereof; or c) a fatty acid ester and a further solvent selected from a triglyceride, glycerol ester or combination thereof.

5. A composition of any one of claims 1 to 4 wherein the non-hydroxyl containing solvent or the non-heterocyclic ester solvent is a fatty acid ester.

6. A composition of any one of claims 1 to 4 wherein the non-hydroxyl containing solvent or the non-heterocyclic ester solvent is selected from a triglyceride, glycerol ester or combination thereof.

7. A composition of claim 1 or 2 comprising a glycol ether, the glycol ether being the tripropylene glycol alkyl ether, and a solvent selected from a triglyceride, glycerol ester or a combination thereof.

8. A composition of any one of claims 3 to 5 wherein the fatty acid ester has a $C_{10}$-$C_{16}$ alkyl chain.

9. A composition of any one of claims 3 to 5 wherein the fatty acid ester is selected from isopropyl myristate, triacetin, propylene glycol octanoate decanoate (PGOD), polysorbate 20, or a mixture thereof.

10. A composition of claim 1 or 2 wherein the non-heterocyclic ester solvent is selected from a triglyceride, a glycerol ester or a combination thereof.

11. A composition of any one of claims 1 to 8 wherein the non-hydroxyl containing solvent is selected from DMSO or DMI.

12. A composition of claim 3 or 5, comprising a solvent selected from a triglyceride, a glycerol ester or a mixture thereof.

13. A composition of claim 6 or 10, comprising a fatty acid ester.

14. A composition of any one of claims 1 to 13, comprising at least one surfactant, and wherein at least one of the sufactants has the following structure:

$$Z_[o_CR_1R_2CR_3R_4]_n_OH$$

where

z is an optionally substituted $C_{14}$ to $C_{22}$ linear alkenyl,
$R_1$, $R_2$, $R_3$ and $R_4$ are each independently selected from methyl or hydrogen, and
n is an integer from 1 to 10.

15. A composition of any one of claims 1 to 14 wherein the composition comprises an antioxidant.

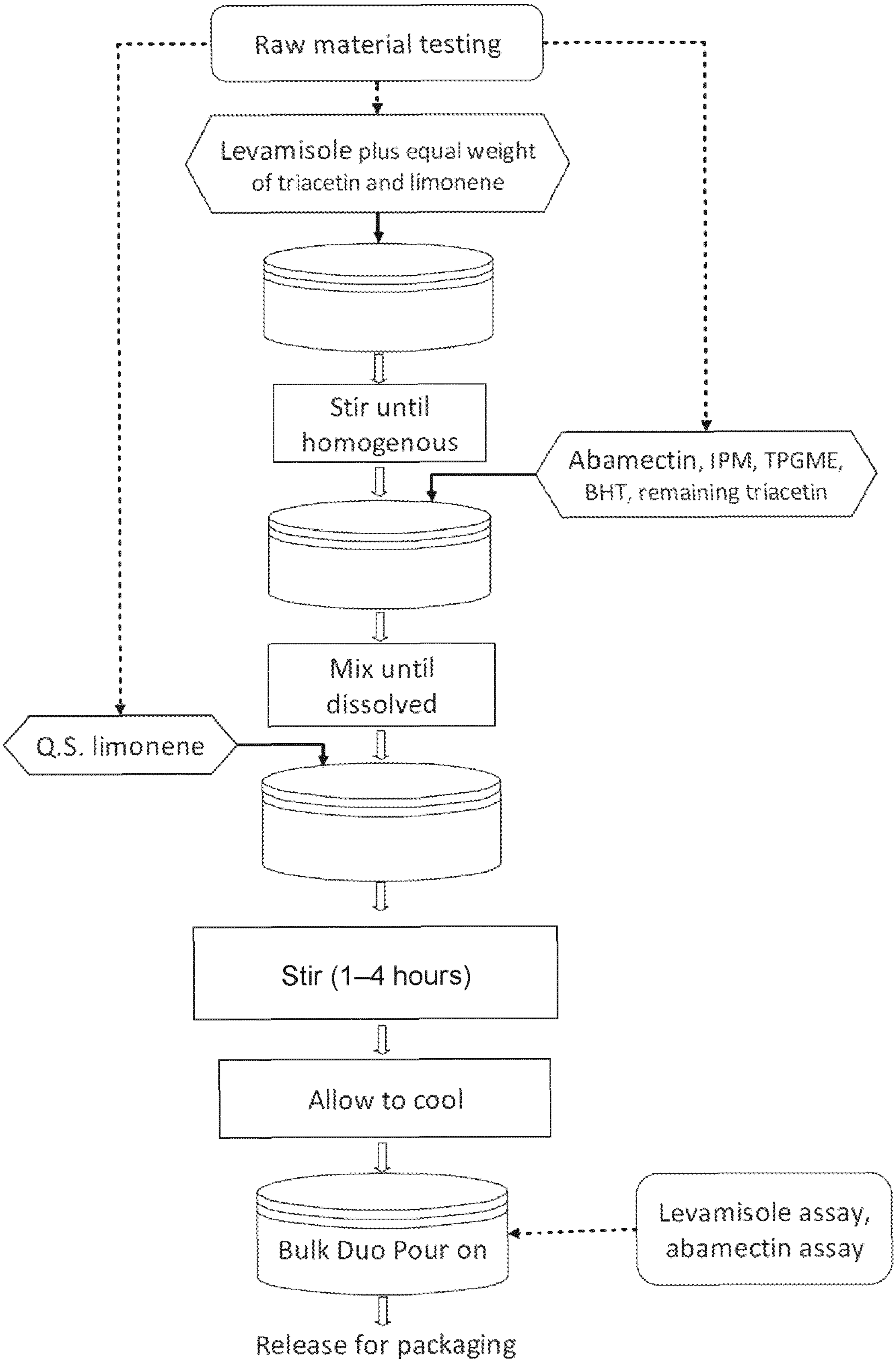
Raw material testing
Levamisole plus equal weight of triacetin and limonene
Stir until homogenous
Abamectin, IPM, TPGME, BHT, remaining triacetin
Mix until dissolved
Q.S. limonene
Stir (1–4 hours)
Allow to cool
Bulk Duo Pour on
Levamisole assay, abamectin assay
Release for packaging

**Figure 1**

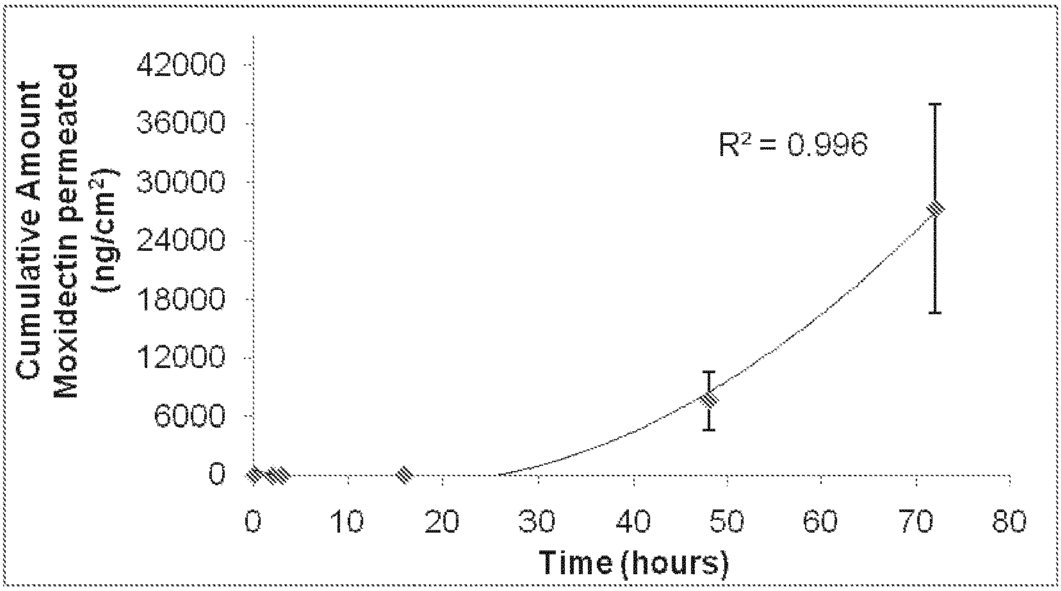


**Figure 2**

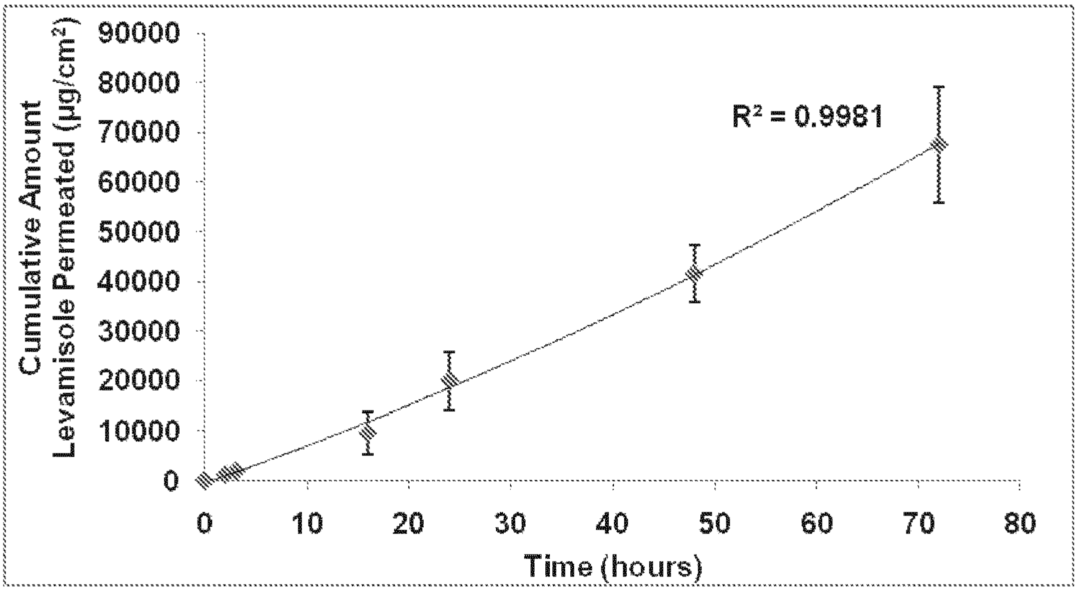


**Figure 3**

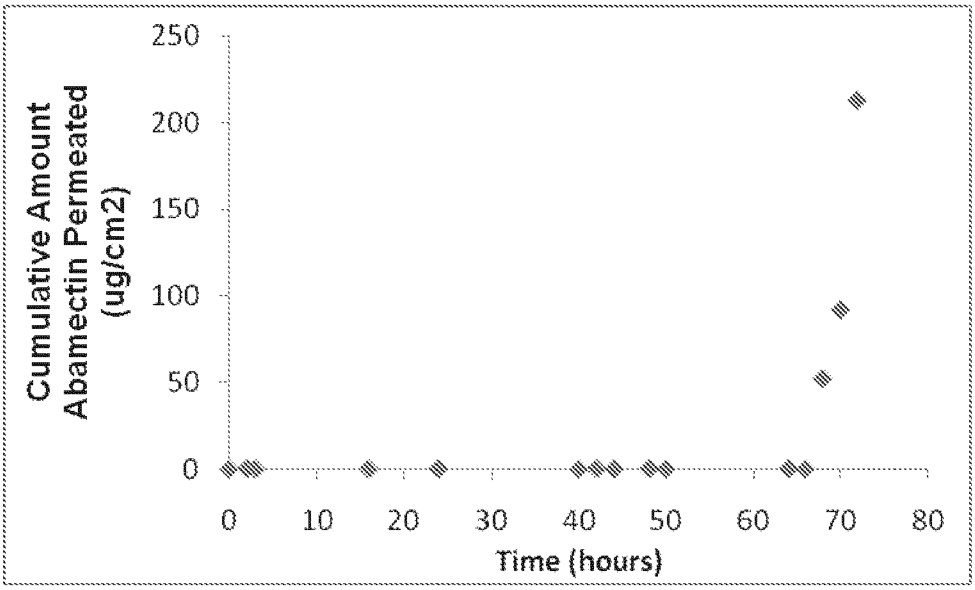


Figure 4

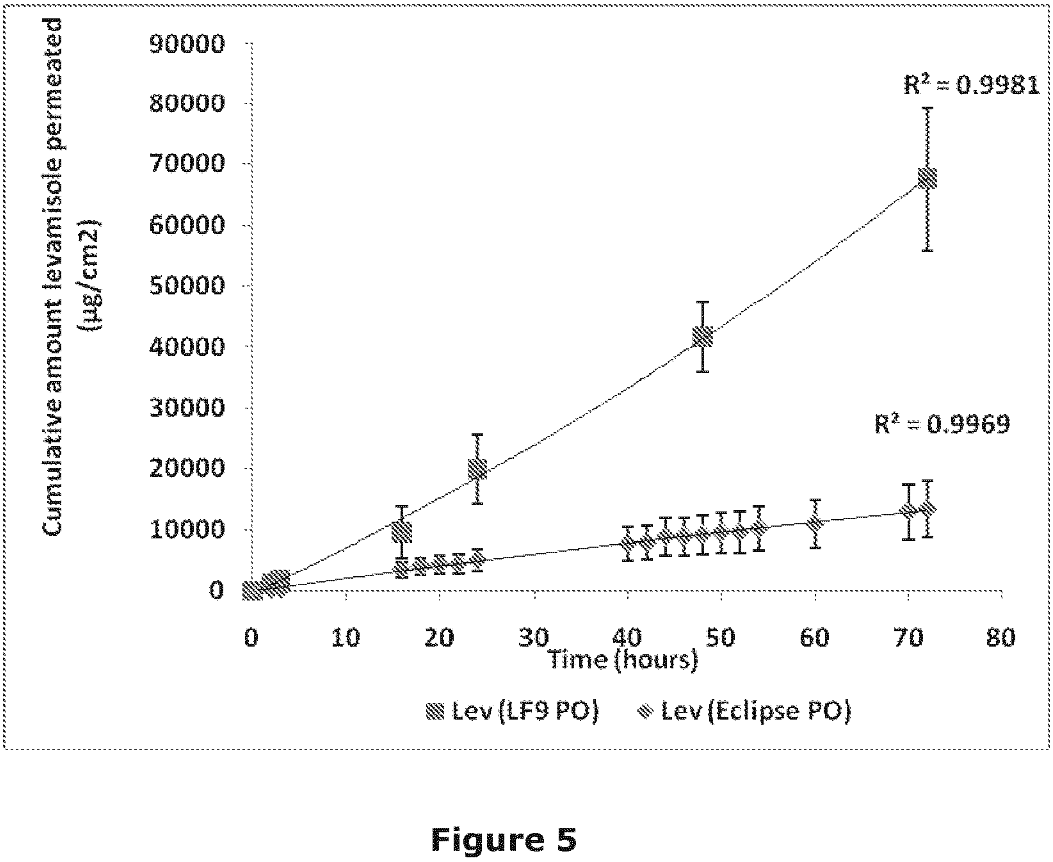


Figure 5

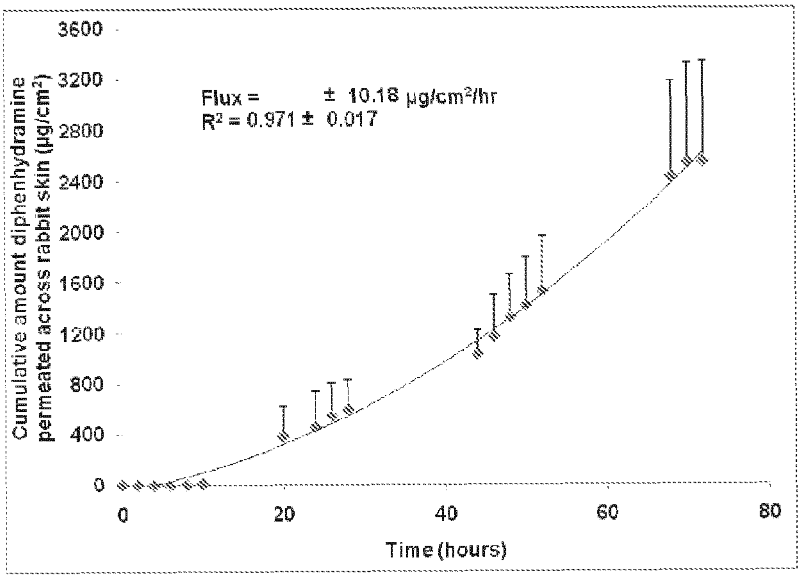


Figure 6

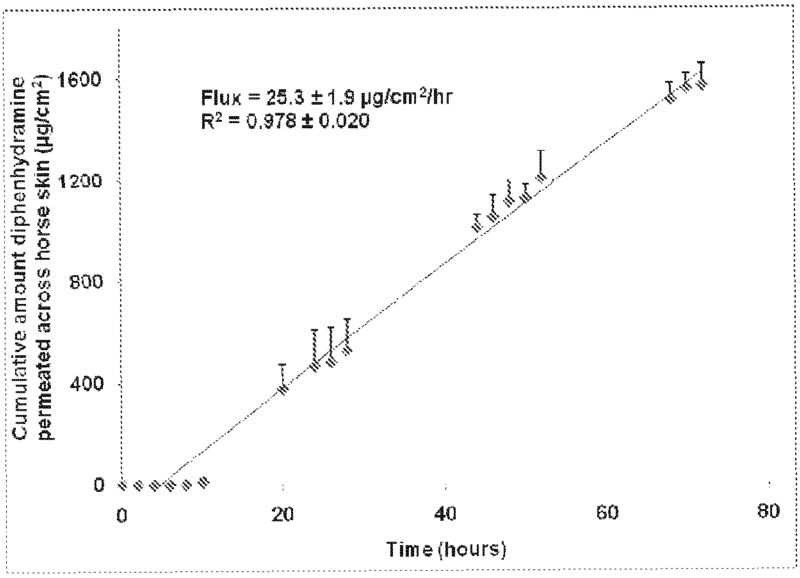


Figure 7

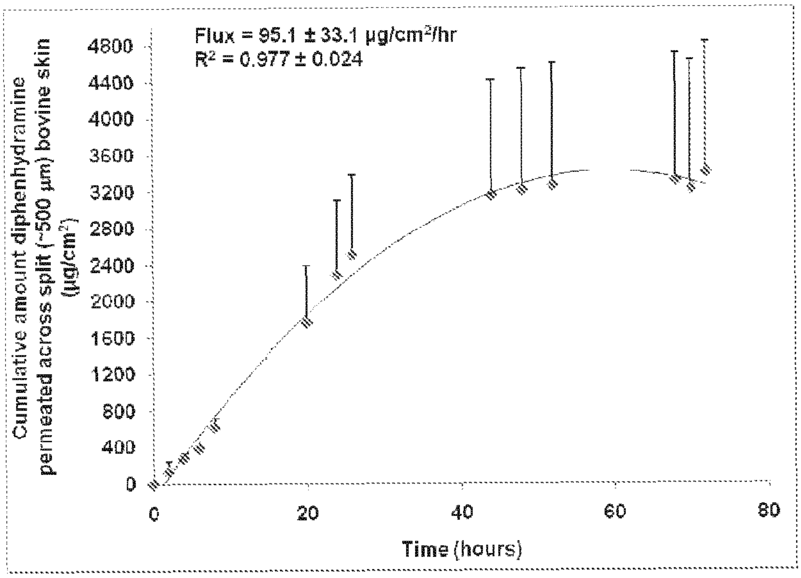


Figure 8

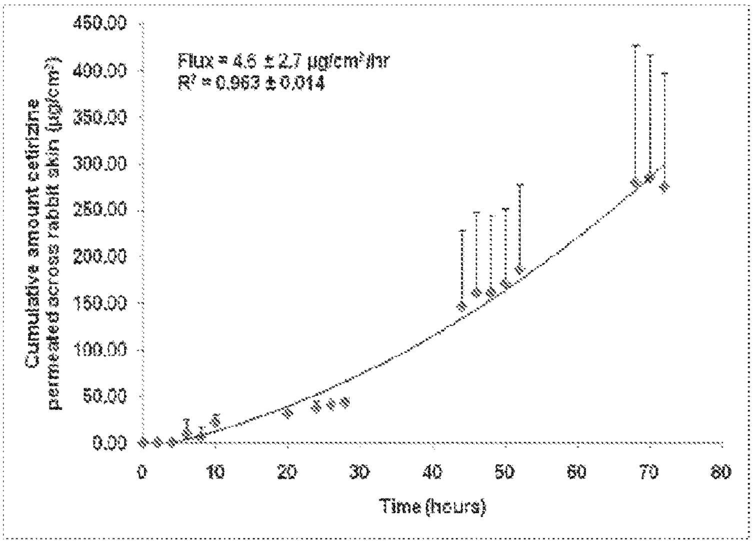


Figure 9

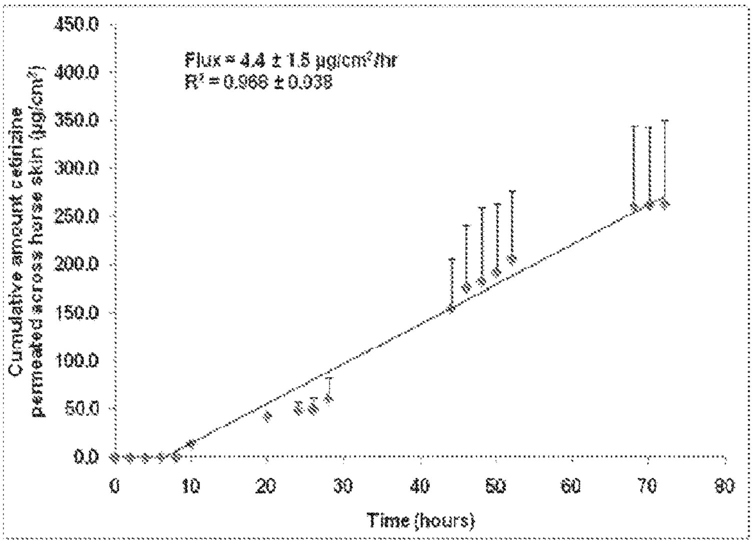


Figure 10

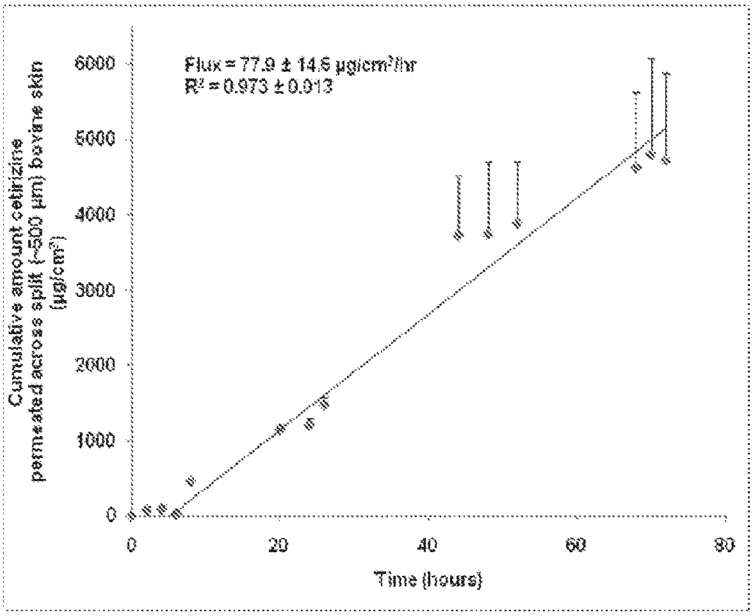


Figure 11

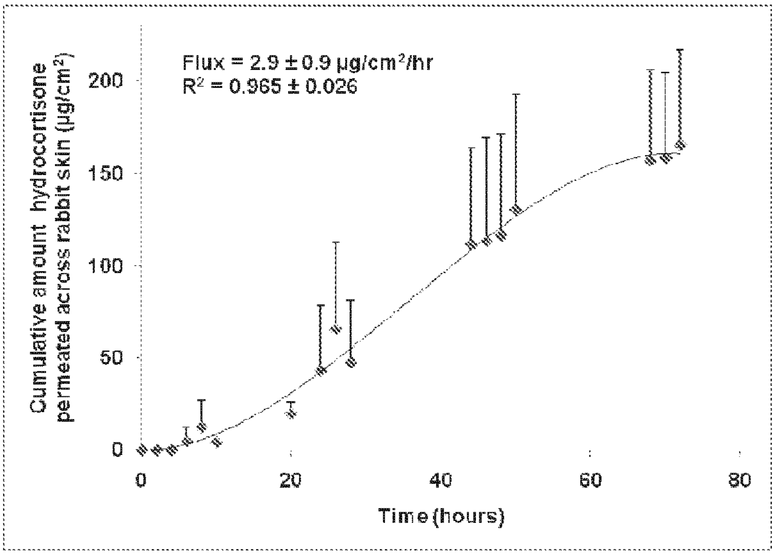


Figure 12

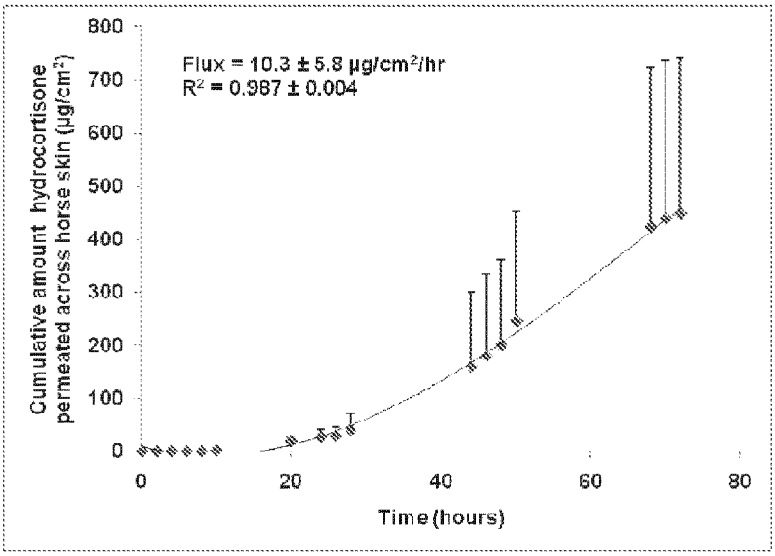


Figure 13

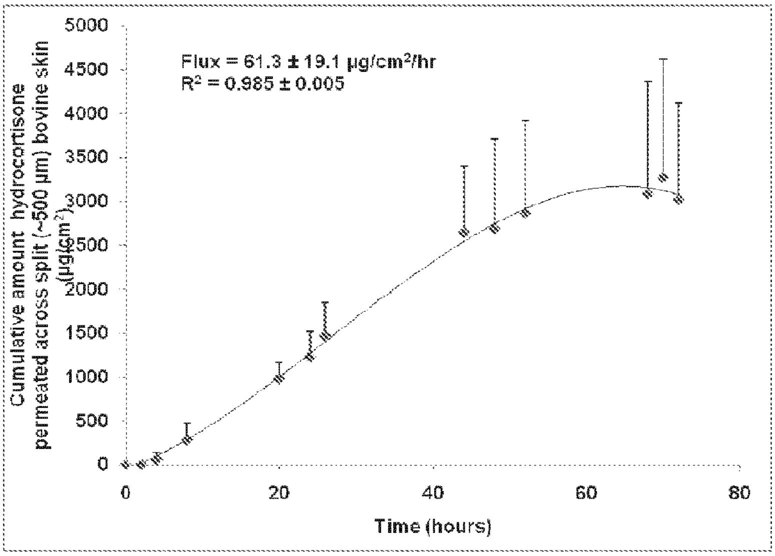


Figure 14

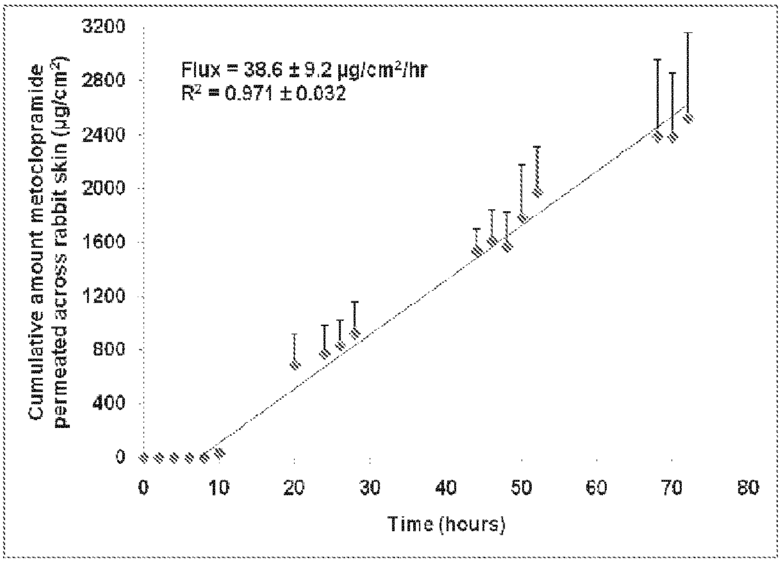


Figure 15

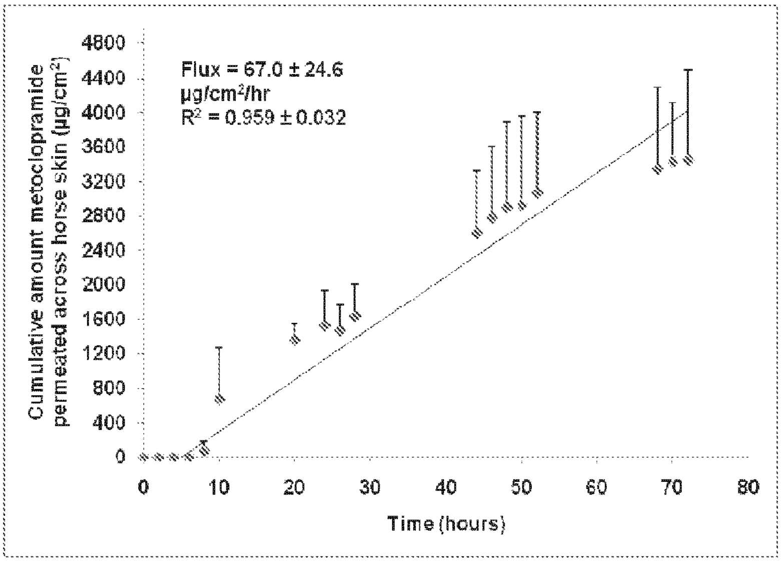


Figure 16

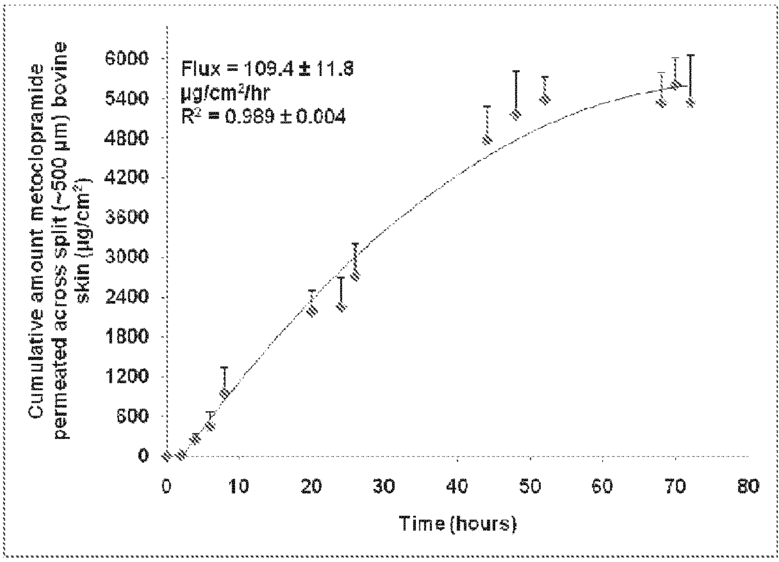


Figure 17

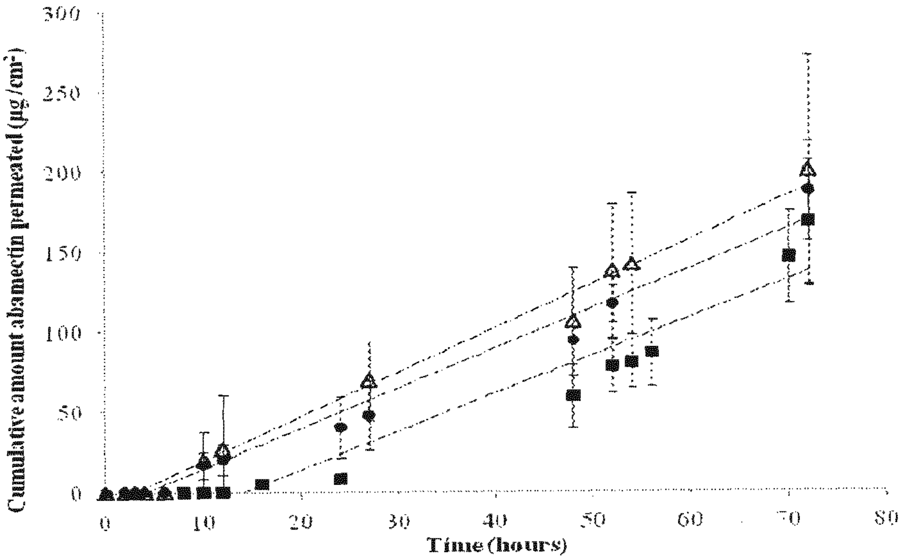


Figure 18

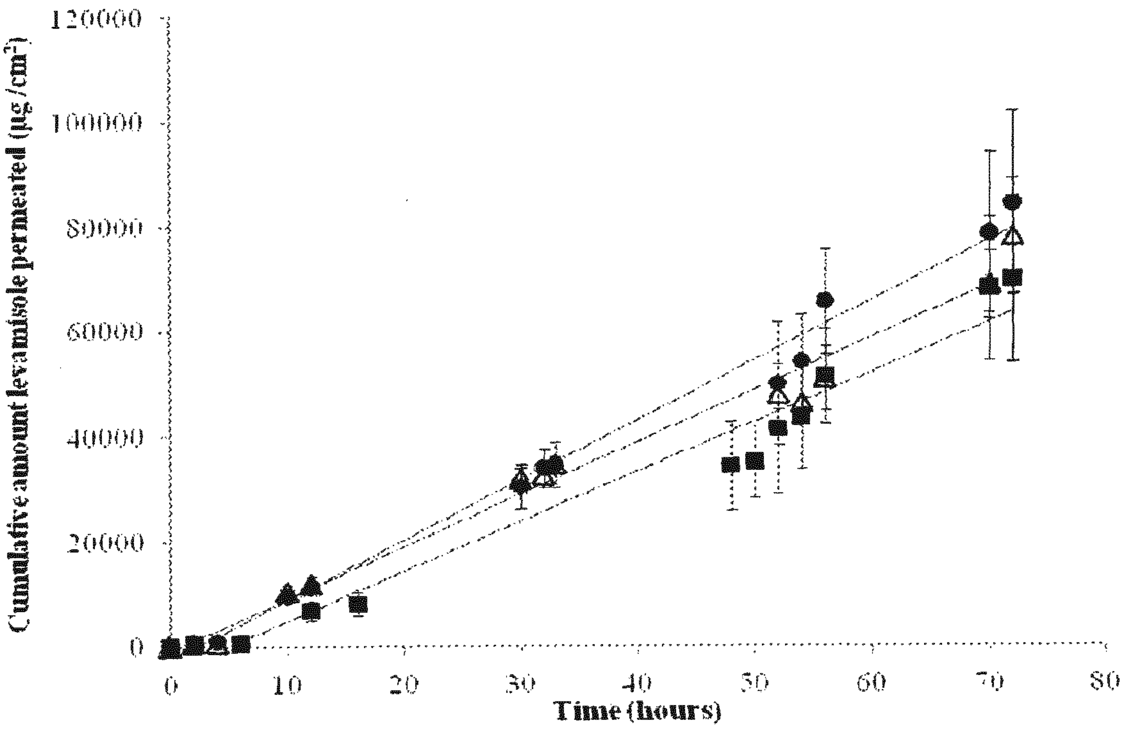


Figure 19

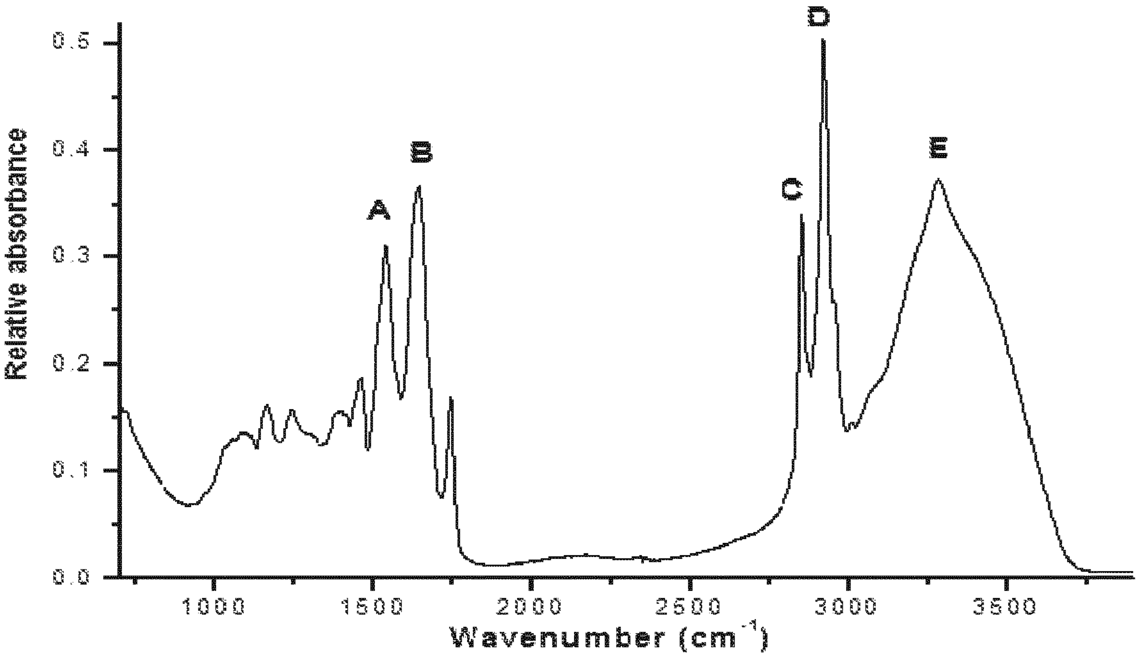
0.5
0.4
0.3
0.2
0.1
0.0
Relative absorbance
A
B
C
D
E
1000
1500
2000
2500
3000
3500
Wavenumber (cm-1)

**Figure 20**

Europäisches Patentamt
European Patent Office
Office européen des brevets

# EUROPEAN SEARCH REPORT

Application Number
EP 21 18 0655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 096 262 A (ANDREWS PETER ET AL) 20 June 1978 (1978-06-20)<br>* column 8; example M *<br>* claims 1-36 * | 1,3-6, 8-13,15 | INV.<br>A61K9/08<br>A61K47/06<br>A61K47/08<br>A61K47/10<br>A61K47/14<br>A61K47/20<br>A61K47/22<br>A61K47/26<br>A61K31/135<br>A61K31/166<br>A61K31/365<br>A61K31/429<br>A61K31/495<br>A61K31/506<br>A61K31/573<br>A61K31/7048 |
| X | WO 01/97777 A1 (WHAN IN PHARM CO LTD [KR]) 27 December 2001 (2001-12-27)<br>* page 4, lines 9-11 *<br>* page 6, lines 4-9 *<br>* page 9; example 3 *<br>* claims 1-14 * | 1,3-15 | |
| X | WO 2010/002880 A1 (UNIV TEMPLE [US]; SCHWARTZ ARTHUR G [US]; WILLIAMS JOHN R [US]) 7 January 2010 (2010-01-07)<br>* page 36; example 4.5 *<br>* page 49 - page 54; example 6 * | 1-3,5,6, 8-11,13, 15 | |
| X | US 2008/153885 A1 (MEADOWS CHEYNEY [US] ET AL) 26 June 2008 (2008-06-26)<br>* page 6; formulation B *<br>* page 7; example 5 * | 1-3,5,6, 8-11,13, 15 | TECHNICAL FIELDS SEARCHED (IPC)<br>A61K |
| X | US 6 933 318 B1 (KASSEBAUM JAMES WEB [US] ET AL) 23 August 2005 (2005-08-23)<br>* column 1, line 58 - column 2, line 4 *<br>* column 9, lines 25-35 *<br>* claims 2-4 * | 1,3,5,6, 8-11,13, 15 | |
| X | US 2005/079164 A1 (FANTUZZI MICHAEL [US] ET AL) 14 April 2005 (2005-04-14)<br>* page 5, paragraph 58 * | 1-3,5,6, 8-12,15 | |

-/--

1 The present search report has been drawn up for all claims

| Place of search | Date of completion of the search | Examiner |
|---|---|---|
| The Hague | 17 December 2021 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 18 0655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 116 401 A2 (YOUNG ROBERT CO LTD [GB]) 22 August 1984 (1984-08-22)<br>* page 1, lines 1-11 *<br>* page 4, lines 16-19 *<br>* page 12; examples 4-6 *<br>----- | 1,3,5,6, 8-12,15 | |
| X | US 2010/087497 A1 (ALBRIGHT ROBERT BRUCE [US] ET AL) 8 April 2010 (2010-04-08)<br>* page 1, paragraphs 8,11 *<br>* page 2, paragraph 29 - page 3, paragraph 34 *<br>* claims 1-42 *<br>----- | 1,3,5,6, 8-10,15 | |
| X | WO 2008/091167 A1 (BOMAC RESEARCH LTD [NZ]; NANJAN KARTHIGEYAN [NZ] ET AL) 31 July 2008 (2008-07-31)<br>* page 6, line 22 - page 7, line 6 *<br>* page 8, lines 13,14 *<br>* page 11, line 19 - page 12, line 6 *<br>* page 16; formulation 1 *<br>* page 16, lines 1-10 *<br>* claims 1-24 *<br>----- | 1,3,5,6, 8-10,15 | TECHNICAL FIELDS SEARCHED (IPC) |
| A | WO 02/062326 A1 (SCHERING PLOUGH LTD [CH]) 15 August 2002 (2002-08-15)<br>* the whole document *<br>----- | 1-15 | |
| A | WO 01/40446 A1 (LILLY CO ELI [US]) 7 June 2001 (2001-06-07)<br>* page 27 - page 29; example 10 *<br>* claims 1-24 *<br>----- | 1-15 | |
| A | WO 2008/072985 A2 (BOMAC RESEARCH LTD [NZ]) 19 June 2008 (2008-06-19)<br>* the whole document *<br>-----<br>-/-- | 1-15 | |

1 The present search report has been drawn up for all claims

| Place of search | Date of completion of the search | Examiner |
|---|---|---|
| The Hague | 17 December 2021 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 18 0655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 02/094221 A1 (JUPITAR PTY LTD [AU]; COOTE WAYNE JOHN [AU] ET AL.) 28 November 2002 (2002-11-28) * page 15 - page 16; example 16 * ----- | 1-15 | |
| A | WO 00/47208 A1 (SAMYANG CORP [KR]; SEO BO YOUN [KR] ET AL.) 17 August 2000 (2000-08-17) * page 15 - page 18; table 1 * ----- | 1-15 | |
| E | WO 2014/098619 A1 (DONAGHYS IND LTD [NZ]) 26 June 2014 (2014-06-26) * the whole document * ----- | 1,3,5,6, 8-11,13, 15 | |
| | | | TECHNICAL FIELDS SEARCHED (IPC) |

1 The present search report has been drawn up for all claims

| Place of search | Date of completion of the search | Examiner |
|---|---|---|
| The Hague | 17 December 2021 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 18 0655

This annex lists the patent family members relating to the patent documents cited in the above-mentioned European search report. The members are as contained in the European Patent Office EDP file on
The European Patent Office is in no way liable for these particulars which are merely given for the purpose of information.

17-12-2021

| Patent document cited in search report | Publication date | Patent family member(s) | Publication date |
|---|---|---|---|
| US 4096262 A | 20-06-1978 | AT 354182 B | 27-12-1979 |
| | | AU 506372 B2 | 20-12-1979 |
| | | BE 853303 A | 06-10-1977 |
| | | CA 1106284 A | 04-08-1981 |
| | | CH 639822 A5 | 15-12-1983 |
| | | CS 203145 B2 | 27-02-1981 |
| | | DD 132845 A5 | 15-11-1978 |
| | | DE 2614841 A1 | 20-10-1977 |
| | | FR 2347052 A1 | 04-11-1977 |
| | | GB 1527584 A | 04-10-1978 |
| | | IE 44980 B1 | 02-06-1982 |
| | | IT 1075689 B | 22-04-1985 |
| | | JP S6251932 B2 | 02-11-1987 |
| | | JP S52122619 A | 15-10-1977 |
| | | NL 7703729 A | 10-10-1977 |
| | | NZ 183776 A | 18-12-1978 |
| | | SE 428173 B | 13-06-1983 |
| | | US 4096262 A | 20-06-1978 |
| | | ZA 772074 B | 26-07-1978 |
| WO 0197777 A1 | 27-12-2001 | AU 7464901 A | 02-01-2002 |
| | | KR 20020000316 A | 05-01-2002 |
| | | US 6500440 B1 | 31-12-2002 |
| | | WO 0197777 A1 | 27-12-2001 |
| WO 2010002880 A1 | 07-01-2010 | AU 2009267083 A1 | 07-01-2010 |
| | | CA 2729343 A1 | 07-01-2010 |
| | | EP 2303279 A1 | 06-04-2011 |
| | | ES 2539952 T3 | 07-07-2015 |
| | | US 2010004217 A1 | 07-01-2010 |
| | | US 2013196959 A1 | 01-08-2013 |
| | | US 2013196963 A1 | 01-08-2013 |
| | | WO 2010002880 A1 | 07-01-2010 |
| US 2008153885 A1 | 26-06-2008 | AR 064425 A1 | 01-04-2009 |
| | | AU 2007339312 A1 | 10-07-2008 |
| | | BR PI0720500 A2 | 04-02-2014 |
| | | CA 2672964 A1 | 10-07-2008 |
| | | CL 2007003711 A1 | 25-07-2008 |
| | | CN 101588789 A | 25-11-2009 |
| | | CN 103690471 A | 02-04-2014 |
| | | CO 6190598 A2 | 19-08-2010 |
| | | EP 2120860 A2 | 25-11-2009 |
| | | ES 2714550 T3 | 29-05-2019 |
| | | JP 5377326 B2 | 25-12-2013 |
| | | JP 2010513500 A | 30-04-2010 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 21 18 0655

This annex lists the patent family members relating to the patent documents cited in the above-mentioned European search report. The members are as contained in the European Patent Office EDP file on
The European Patent Office is in no way liable for these particulars which are merely given for the purpose of information.

17-12-2021

| Patent document cited in search report | Publication date | Patent family member(s) | Publication date |
|---|---|---|---|
| | | KR 20090091328 A | 27-08-2009 |
| | | NZ 598554 A | 30-08-2013 |
| | | PE 20081406 A1 | 17-10-2008 |
| | | RU 2009127500 A | 27-01-2011 |
| | | TW 200833367 A | 16-08-2008 |
| | | US 2008153885 A1 | 26-06-2008 |
| | | WO 2008082507 A2 | 10-07-2008 |
| | | ZA 200904329 B | 26-05-2010 |
| US 6933318 B1 | 23-08-2005 | NONE | |
| US 2005079164 A1 | 14-04-2005 | AU 2005227118 A1 | 06-10-2005 |
| | | EP 1722645 A1 | 22-11-2006 |
| | | JP 2007526303 A | 13-09-2007 |
| | | US 2005079164 A1 | 14-04-2005 |
| | | US 2005287206 A1 | 29-12-2005 |
| | | US 2008152707 A1 | 26-06-2008 |
| | | WO 2005092123 A1 | 06-10-2005 |
| EP 0116401 A2 | 22-08-1984 | AU 2314184 A | 12-07-1984 |
| | | EP 0116401 A2 | 22-08-1984 |
| | | GB 2133690 A | 01-08-1984 |
| | | GR 79470 B | 30-10-1984 |
| US 2010087497 A1 | 08-04-2010 | AR 073730 A1 | 24-11-2010 |
| | | AU 2009245834 A1 | 22-04-2010 |
| | | BR PI0919293 A2 | 01-12-2020 |
| | | CA 2737102 A1 | 15-04-2010 |
| | | CL 2011000768 A1 | 19-08-2011 |
| | | CN 102176899 A | 07-09-2011 |
| | | EP 2331068 A1 | 15-06-2011 |
| | | ES 2617672 T3 | 19-06-2017 |
| | | HK 1158540 A1 | 20-07-2012 |
| | | JP 5547738 B2 | 16-07-2014 |
| | | JP 2012505218 A | 01-03-2012 |
| | | KR 20110050735 A | 16-05-2011 |
| | | NZ 591741 A | 25-01-2013 |
| | | PT 2331068 T | 13-03-2017 |
| | | RU 2011112990 A | 20-11-2012 |
| | | US 2010087497 A1 | 08-04-2010 |
| | | WO 2010042395 A1 | 15-04-2010 |
| | | ZA 201103282 B | 26-11-2014 |
| WO 2008091167 A1 | 31-07-2008 | AU 2008208151 A1 | 31-07-2008 |
| | | CA 2713199 A1 | 31-07-2008 |
| | | EP 2124938 A1 | 02-12-2009 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 21 18 0655

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2021

| Patent document cited in search report | Publication date | Patent family member(s) | Publication date |
|---|---|---|---|
| | | JP 2010516761 A | 20-05-2010 |
| | | NZ 552816 A | 31-05-2009 |
| | | US 2010137389 A1 | 03-06-2010 |
| | | WO 2008091167 A1 | 31-07-2008 |
| WO 02062326 A1 | 15-08-2002 | AR 032545 A1 | 12-11-2003 |
| | | AU 2002253478 B2 | 26-08-2004 |
| | | BR 0207115 A | 25-02-2004 |
| | | CA 2437697 A1 | 15-08-2002 |
| | | CN 1499963 A | 26-05-2004 |
| | | CZ 20032121 A3 | 18-02-2004 |
| | | EC SP034718 A | 24-09-2003 |
| | | EP 1372622 A1 | 02-01-2004 |
| | | HU 0302867 A2 | 29-12-2003 |
| | | IL 157231 A | 28-04-2011 |
| | | JP 4357838 B2 | 04-11-2009 |
| | | JP 2004520390 A | 08-07-2004 |
| | | JP 2008189690 A | 21-08-2008 |
| | | KR 20030075182 A | 22-09-2003 |
| | | MX PA03007126 A | 18-11-2003 |
| | | NZ 527906 A | 29-04-2005 |
| | | PE 20020945 A1 | 19-11-2002 |
| | | PL 365060 A1 | 27-12-2004 |
| | | RU 2302858 C2 | 20-07-2007 |
| | | SK 9942003 A3 | 08-01-2004 |
| | | TW I249987 B | 01-03-2006 |
| | | UA 73848 C2 | 15-09-2005 |
| | | US 2003073667 A1 | 17-04-2003 |
| | | WO 02062326 A1 | 15-08-2002 |
| | | ZA 200306082 B | 26-01-2005 |
| WO 0140446 A1 | 07-06-2001 | AR 027890 A1 | 16-04-2003 |
| | | AT 259590 T | 15-03-2004 |
| | | AU 775911 B2 | 19-08-2004 |
| | | CO 5271707 A1 | 30-04-2003 |
| | | DE 60008422 T2 | 02-12-2004 |
| | | DK 1237408 T3 | 28-06-2004 |
| | | EP 1237408 A2 | 11-09-2002 |
| | | ES 2214339 T3 | 16-09-2004 |
| | | NZ 518030 A | 28-05-2004 |
| | | PT 1237408 E | 30-06-2004 |
| | | TR 200400599 T4 | 21-04-2004 |
| | | US 6955818 B1 | 18-10-2005 |
| | | WO 0140446 A1 | 07-06-2001 |
| | | ZA 200202537 B | 23-09-2003 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 21 18 0655

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2021

| Patent document cited in search report | Publication date | Patent family member(s) | Publication date |
|---|---|---|---|
| WO 2008072985 A2 | 19-06-2008 | AU 2007332222 A1 | 19-06-2008 |
| | | BR PI0720102 A2 | 08-07-2014 |
| | | NZ 552040 A | 30-04-2009 |
| | | WO 2008072985 A2 | 19-06-2008 |
| WO 02094221 A1 | 28-11-2002 | CA 2447170 A1 | 28-11-2002 |
| | | EP 1399130 A1 | 24-03-2004 |
| | | US 2004167034 A1 | 26-08-2004 |
| | | WO 02094221 A1 | 28-11-2002 |
| WO 0047208 A1 | 17-08-2000 | AU 2577500 A | 29-08-2000 |
| | | CA 2360300 A1 | 17-08-2000 |
| | | CN 1364083 A | 14-08-2002 |
| | | DE 60025807 T2 | 17-08-2006 |
| | | EP 1150675 A1 | 07-11-2001 |
| | | ES 2261178 T3 | 16-11-2006 |
| | | JP 4271869 B2 | 03-06-2009 |
| | | JP 2002536412 A | 29-10-2002 |
| | | WO 0047208 A1 | 17-08-2000 |
| WO 2014098619 A1 | 26-06-2014 | AU 2013364520 A1 | 02-07-2015 |
| | | AU 2017201224 A1 | 09-03-2017 |
| | | AU 2018208642 A1 | 09-08-2018 |
| | | AU 2020204198 A1 | 16-07-2020 |
| | | BR 112015014266 A2 | 11-07-2017 |
| | | CA 2895038 A1 | 26-06-2014 |
| | | CL 2015001748 A1 | 13-11-2015 |
| | | CN 104994852 A | 21-10-2015 |
| | | CN 110507606 A | 29-11-2019 |
| | | EP 2934522 A1 | 28-10-2015 |
| | | MX 350224 B | 30-08-2017 |
| | | RU 2015122162 A | 26-01-2017 |
| | | US 2015283119 A1 | 08-10-2015 |
| | | US 2017312256 A1 | 02-11-2017 |
| | | WO 2014098619 A1 | 26-06-2014 |
| | | ZA 201504370 B | 29-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Guidance for Industry Nonsterile Semisolid Dosage Forms Scale-Up and Postapproval Changes: Chemistry, Manufacturing, and Controls. Vitro Release Testing and In Vivo Bioequivalence Documentation. U.S. Department of Health and Human Services, Food and Drug Administration, Center for Drug Evaluation and Research (CDER), May 1997